(19)

Europäisches Patentamt
European Patent Office
Office européen des brevets

(11) **EP 3 494 236 B1**

(12) **EUROPEAN PATENT SPECIFICATION**

(45) Date of publication and mention
of the grant of the patent:
**01.03.2023   Bulletin 2023/09**

(21) Application number: **18736415.3**

(22) Date of filing: **05.01.2018**

(51) International Patent Classification (IPC):
**C12Q 1/6886** *(2018.01)*

(52) Cooperative Patent Classification (CPC):
**C12Q 1/6886;** C12Q 2600/156; C12Q 2600/16

(86) International application number:
**PCT/US2018/012555**

(87) International publication number:
**WO 2018/129293 (12.07.2018 Gazette 2018/28)**

(54) **METHOD FOR CONDUCTING EARLY DETECTION OF COLON CANCER AND/OR OF COLON CANCER PRECURSOR CELLS AND FOR MONITORING COLON CANCER RECURRENCE**

VERFAHREN ZUR DURCHFÜHRUNG VON FRÜHERKENNUNG VON DICKDARMKREBS UND/ODER DICKDARMKREBSVORLÄUFERZELLEN UND ZUR ÜBERWACHUNG DES WIEDERAUFTRETENS VON DICKDARMKREBS

PROCÉDÉ DE DÉTECTION PRÉCOCE DU CANCER DU CÔLON ET/OU DE CELLULES PRÉCURSEURS DU CANCER DU CÔLON, ET DE SURVEILLANCE DE LA RÉCURRENCE DU CANCER DU CÔLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2017   US 201762442898 P**
**04.01.2018   US 201815862581**

(43) Date of publication of application:
**12.06.2019   Bulletin 2019/24**

(73) Proprietor: **Diacarta LLC**
**Richmond, CA 94806 (US)**

(72) Inventors:
• **POWELL, Michael**
**Alamo, CA 94507 (US)**
• **ZHANG, Aiguo**
**San Ramon, CA 94583 (US)**
• **PELETSKAYA, Elena**
**San Pablo, CA 94806 (US)**

(74) Representative: **Schüssler, Andrea**
**Kanzlei Huber & Schüssler**
**Truderinger Strasse 246**
**81825 München (DE)**

(56) References cited:
WO-A2-2011/093606      WO-A2-2015/116868
EA-B1- 023 565      US-A1- 2016 194 691
US-A1- 2016 194 691

• QI WANG ET AL: "Molecular Beacons of Xeno-Nucleic Acid for Detecting Nucleic Acid", THERANOSTICS, vol. 3, no. 6, 1 January 2013 (2013-01-01) , pages 395-408, XP055677985, AU ISSN: 1838-7640, DOI: 10.7150/thno.5935
• ZHENBIN CHEN ET AL: "Molecular Diagnosis of Response to Neoadjuvant Chemoradiation Therapy in Patients with Locally Advanced Rectal Cancer", JOURNAL OF THE AMERICAN COLLEGE OF SURGEONS, COLLEGE, CHICAGO, IL, US, vol. 212, no. 6, 22 February 2011 (2011-02-22), pages 1008-1017.e1, XP028219667, ISSN: 1072-7515, DOI: 10.1016/J.JAMCOLLSURG.2011.02.024 [retrieved on 2011-02-28]

**(Cont. next page)**

- MI JUNG KWON ET AL: "Frequency ofandmutations in advanced colorectal cancers: Comparison of peptide nucleic acid-mediated PCR clamping and direct sequencing in formalin-fixed, paraffin-embedded tissue", PATHOLOGY - RESEARCH AND PRACTICE, ELSEVIER, AMSTERDAM, NL, vol. 207, no. 12, 6 October 2011 (2011-10-06), pages 762-768, XP028397033, ISSN: 0344-0338, DOI: 10.1016/J.PRP.2011.10.002 [retrieved on 2011-10-14]
- SATU OLTEDAL ET AL: "Heterogeneous distribution ofmutations in primary colon carcinomas: implications for EGFR-directed therapy", INTERNATIONAL JOURNAL OF COLORECTAL DISEASE ; CLINICAL AND MOLECULAR GASTROENTEROLOGY AND SURGERY, SPRINGER, BERLIN, DE, vol. 26, no. 10, 15 May 2011 (2011-05-15), pages 1271-1277, XP019955058, ISSN: 1432-1262, DOI: 10.1007/S00384-011-1233-5
- MANDAYAM O. NANDAN ET AL.: 'An Update on the Biology of RAS/RAF Mutations in Colorectal Cancer' CURRENT COLORECTAL CANCER REPORTS vol. 7, no. 2, 01 June 2011, pages 113 - 120, XP019898185
- DAVID BARRAS: 'BRAF Mutation in Colorectal Cancer: An Update' BIOMARKERS IN CANCER vol. 7, no. 1, 2015, pages 9 - 12, XP055518156
- SEYED MOHAMMAD HOSSEIN KASHFI ET AL.: 'Frameshift Mutations (Deletion at Codon 1309 and Codon 849) in the APC Gene in Iranian FAP Patients: a Case Series and Review of the Literature' INT J MOL CELL MED. vol. 3, no. 3, 2014, pages 196 - 202, XP055518160

Remarks:
The complete document including Reference Table(s) and the Sequence Listing(s) can be downloaded from the EPO website

**Description**

**FIELD OF THE INVENTION**

The field of application of the present invention is the medical sector, in the field of Molecular Biology. More specifically, the invention addresses the early diagnosis of colorectal cancer and the kit for performing the diagnosis. This invention may be used for disease diagnosis, including the early detection of colon cancer in patients. Thus, the invention may also include isolating DNA from a stool sample, fresh peripheral blood (PB), and formalin-fixed, paraffin-embedded (FFPE) tissues sample obtained from a patient suspected of having a condition associated with colorectal cancer mutations..

The invention further relates to a kit comprising said XNA clamps and primers (i.e. SEQ ID NOs: 22-53), and, in addition, to the use of said kit in the mutational analysis, particularly in early detection of colon cancer and/or colon cancer precursor cells.

**BACKGROUND OF THE INVENTION**

Polymerase chain reaction (PCR) is a widely used technique for the detection of pathogens. The technique uses a DNA polymerase used to amplify a piece of DNA by in vitro enzymatic replication. The PCR process generates DNA that is used as a template for replication.

This results in a chain reaction that exponentially amplifies the DNA template.

Technologies for genomic detection most commonly use DNA probes to hybridize to target sequences. To achieve required sensitivity, the use of PCR to amplify target sequences has remained standard practice in many labs. While PCR has been the principle method to identify genes associated with disease states, the method has remained confined to use within a laboratory environment. Most current diagnostic applications that can be used outside of the laboratory are based on antibody recognition of protein targets and use ELISA-based technologies to signal the presence of a disease. These methods are fast and fairly robust, but they can lack the specificity associated with nucleic acid detection.

With the advent of molecular diagnostics and the discovery of numerous nucleic acid biomarkers useful in the diagnosis and treatment of conditions and diseases, detection of nucleic acid sequences, and sequence variants, mutations and polymorphisms has become increasingly important. In many instances, it is desirable to detect sequence variants or mutations (which may in some instances, differ by one a single nucleotide) present in low copy numbers against a high background of wild-type sequences. For example, as more and more somatic mutations are shown to be biomarkers for cancer prognosis and prediction of therapeutic efficacy, the need for efficient and effective methods to detect rare mutations in a sample is becoming more and more critical. In the case in which one or more allelic variants is/are present in low copy number compared to wild-type sequences, the presence of excess wild-type target sequence creates challenges to the detection of the less abundant variant target sequence. Nucleic acid amplification/detection reactions almost always are performed using limiting amounts of reagents. A large excess of wild-type target sequences, thus competes for and consumes limiting reagents. As a result amplification and/or detection of rare mutant or variant alleles under these conditions is substantially suppressed, and the methods may not be sensitive enough to detect the rare variants or mutants. Various methods to overcome this problem have been attempted. These methods are not ideal, however, because they either require the use of a unique primer for each allele, or the performance of an intricate melt-curve analysis. Both of these shortcomings limit the ability and feasibility of multiplex detection of multiple variant alleles from a single sample.

Additionally, it is also known that colorectal cancer is a leading cause of death in Western society. However, if diagnosed early, it may be treated effectively by surgical removal of the cancerous tissue. Colorectal cancers originate in the colorectal epithelium and typically are not extensively vascularized (and therefore not invasive) during the early stages of development. Colorectal cancer is thought to result from the clonal expansion of a single mutant cell in the epithelial lining of the colon or rectum. The transition to a highly vascularized, invasive and ultimately metastatic cancer which spreads throughout the body commonly takes ten years or longer. If the cancer is detected prior to invasion, surgical removal of the cancerous tissue is an effective cure. However, colorectal cancer is often detected only upon manifestation of clinical symptoms, such as pain and black tarry stool. Generally, such symptoms are present only when the disease is well established, often after metastasis has occurred, and the prognosis for the patient is poor, even after surgical resection of the cancerous tissue. Early detection of colorectal cancer therefore is important in that detection may significantly reduce its morbidity.

Invasive diagnostic methods such as endoscopic examination allow for direct visual identification, removal, and biopsy of potentially cancerous growths such as polyps. Endoscopy is expensive, uncomfortable, inherently risky, and therefore not a practical tool for screening populations to identify those with colorectal cancer. Non-invasive analysis of stool samples for characteristics indicative of the presence of colorectal cancer or precancer is a preferred alternative for early diagnosis, but no known diagnostic method is available which reliably achieves this goal.

## Gene Mutations and Colorectal Cancer (CRC)

Complex signal pathways are involved in the colorectal cancer pathogenesis such as the WNT and RAS/RAF/MAPK pathways. Genetic and epigenetic changes in the pathway components have been studied extensively in relation to their roles in the initiation and development of CRC. KRAS mutations are found in several cancers including colorectal, lung, thyroid, and pancreatic cancers and cholangiocarcinoma. More than 90% KRAS mutations are located within codons 12 and 13 of exon 2, which may lead to abnormal growth signaling by the p21-ras protein. These alterations in cell growth and division may trigger cancer development as signaling is excessive. KRAS mutations have also been detected in many colorectal cancer patients.

The B-type Raf Kinase (BRAF) protein is a serine/threonine kinase that has important roles in regulating the MAP kinase/ERK signaling pathways, affecting cellular proliferation, differentiation, and programmed cell death. A BRAF mutation is commonly found in many human cancers including melanoma, colorectal cancer, lung cancer, and papillary thyroid carcinoma. The most common mutations in BRAF occur in codon 600, where an amino acid substitution in the activation segment of the kinase domain creates a constitutively active form of the protein. The V600E and V600K mutations are found in high frequencies in human cancer V600E 70-90% and V600K 10-15%. BRAF mutations are generally found in tumors that are wild-type for KRAS.

The adenomatous polyposis coli (APC) gene is a key tumor suppressor gene and APC mutation has been found in most colon cancers. The gene encodes a multi-domain protein that binds to various proteins, including -catenin, axin, CtBP, Asefs, IQGAP1, EB1 and microtubules. Most (-60%) cancer-linked APC mutations occur in a region referred to as the mutation cluster region (MCR) and result in C-terminal truncation of the protein. Mutations in the tumor suppressor gene APC result in the accumulation of catenin which activates the Wnt signaling pathway, leading to tumorigenesis. APC also plays roles in other fundamental cellular processes including cell adhesion and migration, organization of the actin and microtubule networks, spindle formation and chromosome segregation. Mutations in APC cause deregulation of theses cellular process, leading to the initiation and expansion of colon cancer. APC has been used as a biomarker for early colon cancer detection.

The $\beta$-catenin gene (CTNNB 1 or rather BCT) is also an important component of the Wnt pathway. Mutations in the serine or threonine phosphorylation sites in the regulatory domain (exon 3, codon 29-48) of the gene leads to accumulation of the gene product ($\beta$-catenin) which activates the Wnt pathway.

US 2016/0194691 A1 refers to the QClamp™ KRAS Mutation Detection Kit which is based on a XNA mediated quantitative PCR clamping technology for the detection of somatic mutations useful in the diagnosis, prognosis and treatment of various tumors.

The authors of Chen, Z. et al. (6 January 2011), "Molecular diagnosis of response to neoadjuvant chemoradiation therapy in patients with locally advanced rectal cancer", J. Am. Coll. Surg. 212: pp. 1008-1017 (City of Hope, Duarte, CA, US); performed a PNA-clamp PCR to identify mutations in the genes *KRAS, p53* and *APC,* because these mutations are the common genetic aberrations found in colorectal cancer.

Kwon, M.J. et al. (6 October 2011), "Frequency of KRAS, BRAF, and PIK3CA mutations in advanced colorectal cancers: comparison of peptide nucleic acid-mediated PCR clamping and direct sequencing in formalin-fixed, paraffin-embedded tissue", Pathol. Res. Pract. 207: pp. 762-768 (Hallym University College of Medicine, Anynag, KR) refers to the PNA-mediated PCR clamping technology for the detection of *KRAS, BRAF* and *PIK3CA* mutations before administration of anti-epidermal growth factor receptor therapy of metastatic colorectal cancer.

Oltedal, S. et al. (15 May 2011), "Heterogenous distribution of K-ras mutations in primary colon carcinomas: implications for EGFR-directed therapy", Int. J. Colorectal Dis. 26: pp. 1271-1277 (Stavanger University Hospital, Stavanger, NO) discloses that tumor biopsies from 158 patients with non-metastatic colon cancer were analysed for *KRAS* mutations by a sensitive and quantitative PNA-clamp PCR assay.

WO 2011/093606 A2 relates to the detection of mutants in the *BRAF* gene using a PNA probe in order to examine colorectal cancer in the early stages to enable effective treatment trough early diagnosis of cancer.

## BRIEF DESCRIPTION OF THE FIGURES

Figure 1 illustrates the principle of the Mutation Detection Test of the invention.

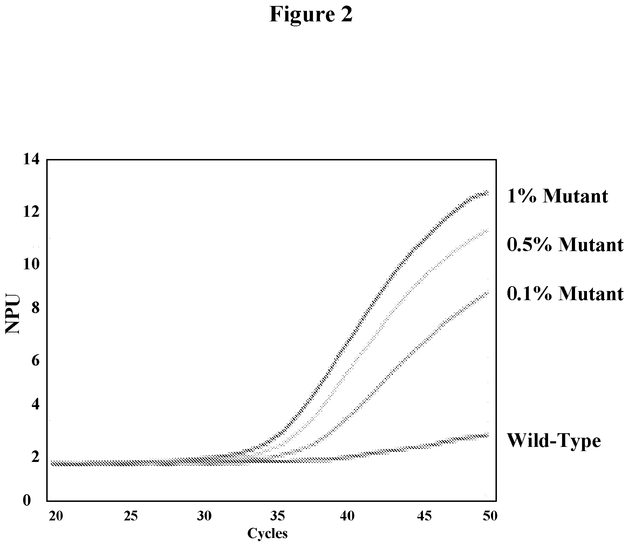

Figure 2 shows qPCR amplification curves generated by the assay of the invention on FFPE tissue.

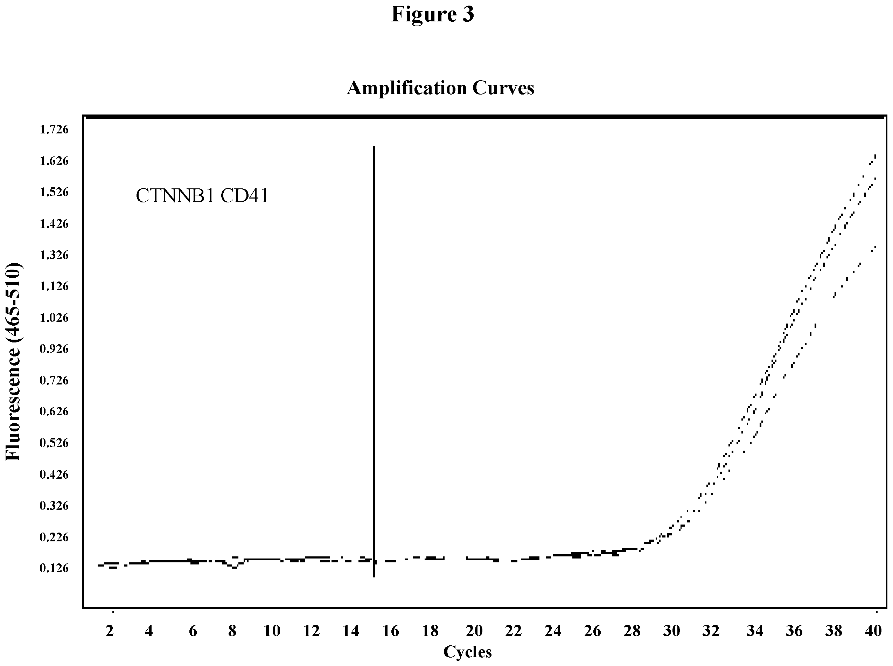

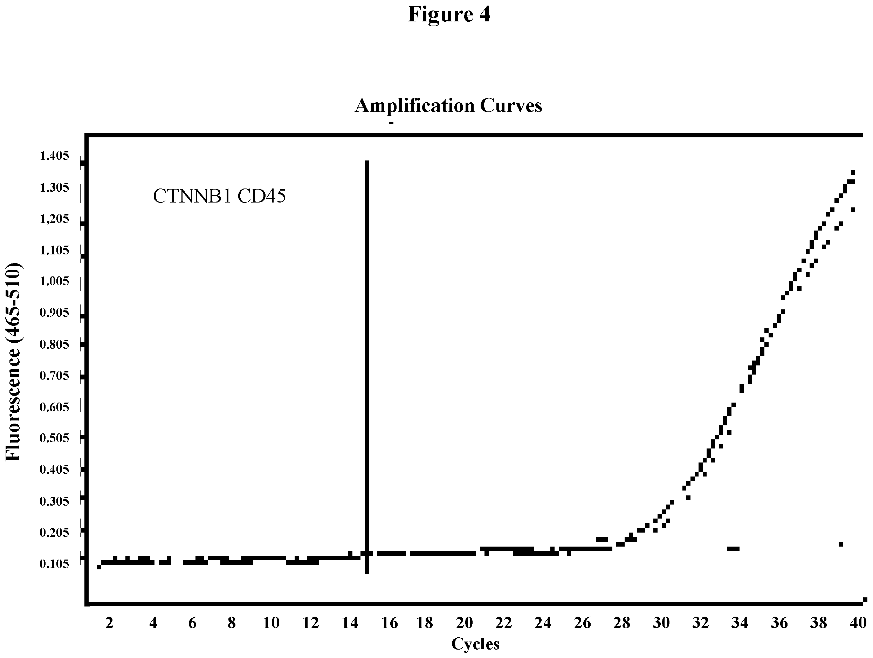

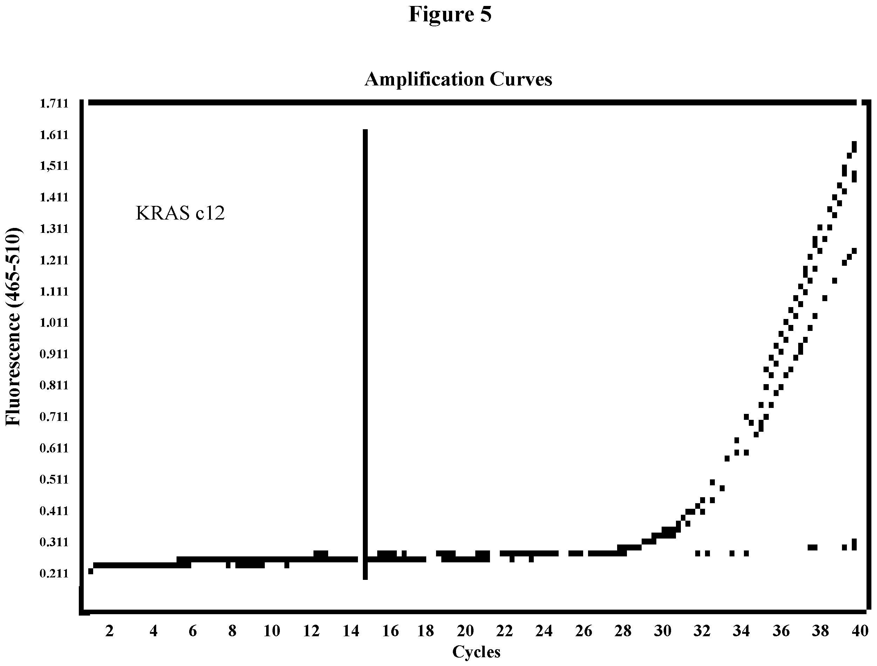

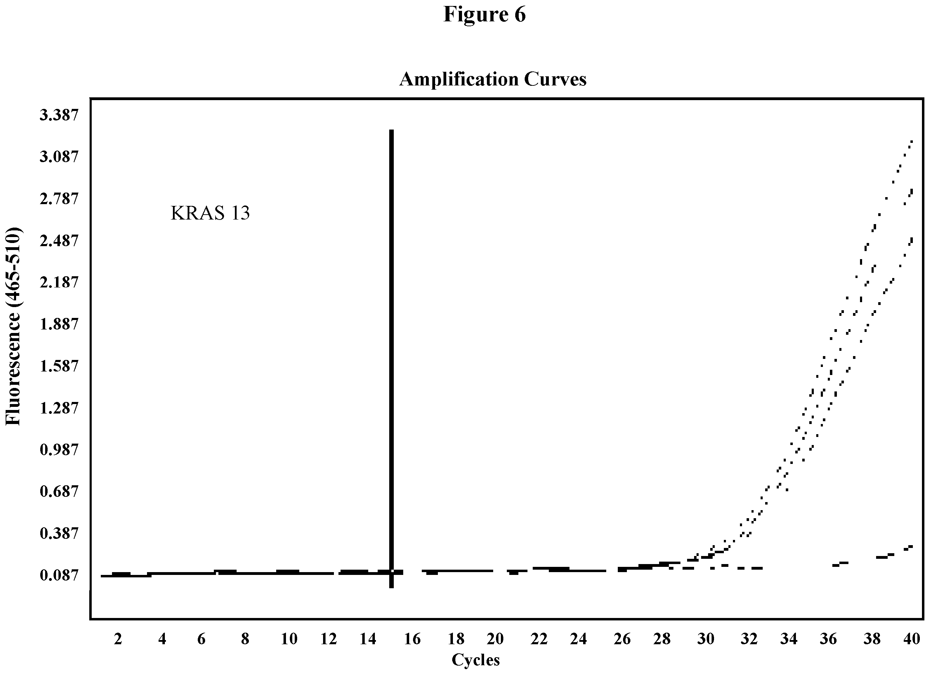

Figures 3-6 illustrate the performance examples of the assays with optimal primer, probe, XNA concentration and $\Delta$Ct between wildtype (Wt) and mutant.

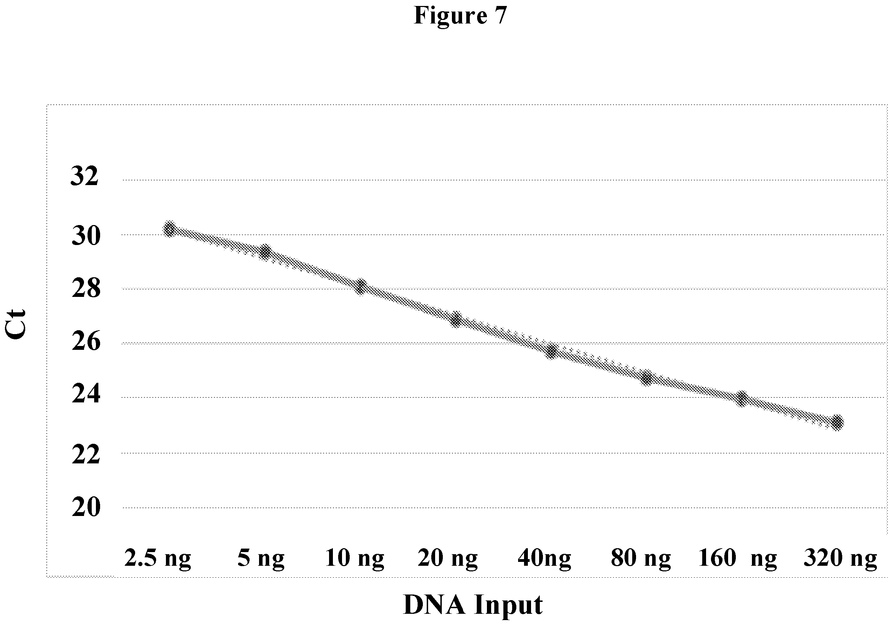

Figure 7 shows quantitative PCR with $\beta$-Actin for different amount of DNA input and demonstrate PCR efficiency in the assay of the invention.

Figure 8 illustrates Watson-Crick Base Pairing of DNA with cognate XNA.

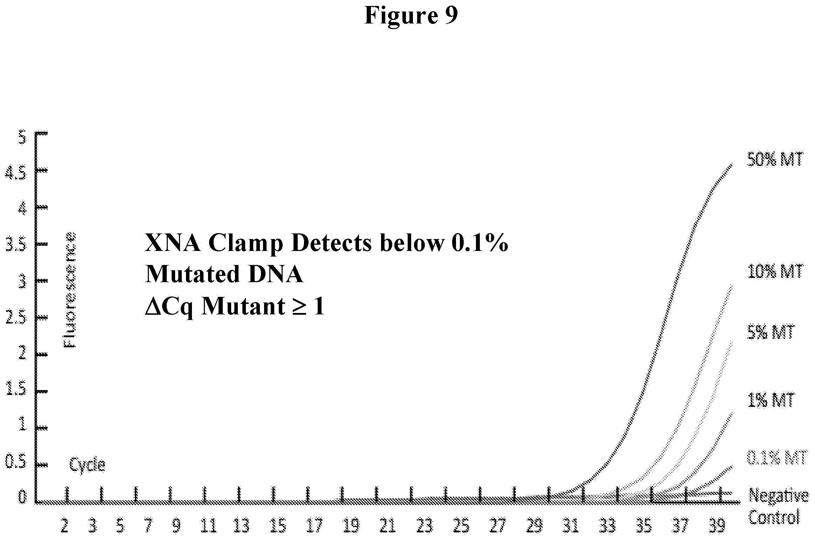

Figure 9 shows how XNA Clamp Detects below 0.1% Mutated DNA.

## SUMMARY OF THE INVENTION

The invention provides a method for detecting the presence or absence of a known mutated gene contained in a biological sample, said method comprising the steps of: (1) allowing a mixture of a clamp primer consisting of XNA which hybridizes with all or part of a target site having a sequence of a wild-type gene or a sequence complementary to the wild-type gene, a primer capable of amplifying a region comprising a target site having a sequence of the mutated gene, and the biological sample to coexist in a reaction solution for gene amplification, and selectively amplifying the region comprising a target site of the mutated gene by a gene amplification method, and (2) selectively detecting a detection region comprising the target site of the mutated gene by a gene detection method, using an amplified product obtained in step (1) or part thereof as a template, to detect the presence or absence of the mutated gene, wherein the mutated genes are selected from the group consisting of APC 1309, APC 1367, APC 1450, BCT 41, BCT 45, KRAS 12, KRAS 13 and BRAF V600, and wherein the XNA clamps and primers of the present invention are selected from the group consisting of

SEQ ID NO:22 - ACGACACAGGAAGCAGATTCT,
SEQ ID NO:23 - TCACAGGATCTTCAGCTGACCT,
SEQ ID NO:24 - TTCCAATCTTTTATTTCTGCTATT,
SEQ ID NO:25 - Lys-0-(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,
SEQ ID NO:28 - 5'/56-FAM/CAAAAGTGG/ZEN/TGCTTAGACACCCAAAAG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAATCCATTCTGGTGC,
SEQ ID NO:35 - AGAAAATCCCTGTTCCCACTCATA,
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:42 - AAGGCCTGCTGAAAATGACTG,
SEQ ID NO:43 - GTTGGATCATATTCGTCCAC,
SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO:48 - TCTGAATTAGCTGTATCGTCAAGGCACTC,
SEQ ID NO:49 - D-LYS-PEG2-TCTTGCCTACGCCACCAGCTCCA-NH$_2$,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG, and
SEQ ID NO:53 - ATCGAGATTTCACTGTAGCTAGAC.

The invention is set out in the appended set of claims. According to the present invention, the method may comprise: isolating DNA from a stool sample, fresh peripheral blood (PB), plasma, and formalin-fixed, paraffin-embedded (FFPE) tissues sample obtained from the patient suspected of having a condition associated with colorectal cancer mutations; performing PCR on the extracted DNA to produce amplified DNA while using a xenonucleic acid clamp for blocking amplification of wild-type DNA; sequencing the amplified DNA in an automated sequencer; analyzing an output of the automated sequencer to identify mutations in the sequence.

The invention also provides a kit for performing the method of the present invention, wherein the XNA clamps and primers are also selected from the group consisting of SEQ ID NOs: 22-53 as listed above.

## DESCRIPTION OF THE INVENTION

The method of the present invention is a real-time PCR based on an in-vitro diagnostic assay for qualitative detection of colorectal cancer associated biomarkers or rather mutations selected from APC 1309, APC 1367, APC 1450, BCT 41, BCT 45, KRAS 12, KRAS 13 and BRAF V600

The mutation detection assay of the invention is based on xenonucleic acid (XNA) mediated PCR clamping technology using the XNA clamps and primers selected from the group consisting of SEQ ID NOs: 22-53 as listed above. Xeno-nucleic acids (XNAs) are synthetic genetic polymers containing non-natural components such as alternative nucleobases, sugars, or a connecting backbone with a different chemical structure. This introduction of a wider selection of functional building blocks could enable XNA sequences to participate in a wider selection of chemical reactions than their DNA or RNA equivalents. XNA is a synthetic DNA analog in which the phosphodiester backbone has been replaced by a repeat formed by units of (2-aminoethyl)-glycine. XNAs hybridize tightly to complementary DNA target sequences only if the sequence is a complete match. Binding of XNA to its target sequence blocks strand elongation by DNA polymerase. When there is a mutation in the target site, and therefore a mismatch, the XNA:DNA duplex is unstable, allowing strand elongation by DNA polymerase. Addition of an XNA, whose sequence with a complete match to wild-type DNA, to a PCR reaction, blocks amplification of wild-type DNA allowing selective amplification of mutant DNA. XNA oligomers are not recognized by DNA polymerases and cannot be utilized as primers in subsequent real-time PCR reactions.

The invention may be used for conducting the early detection of and/or monitoring recurrence of colon cancer and for the detection of colon cancer precursory cells, employing the polymerase chain reaction (PCR) using the primers and xenonucleic acid (XNA) clamp oligomers as described above (i.e. SEQ ID NOs: 22-53) with which the mutation analyses can be carried out in the selected regions of genes APC, K-ras, β-catenin, and B-raf as described above (i.e. APC 1309, APC 1367, APC 1450, BCT 41, BCT 45, KRAS 12, KRAS 13 and BRAF V600).. The invention also relates to a kit containing said primers and xenonucleic acid (XNA) clamp oligomers.

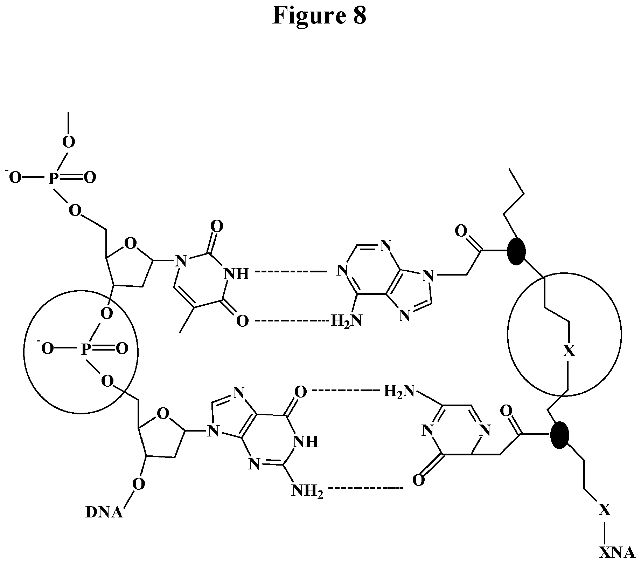

The nucleic acid molecular oligomers of the present invention that hybridize by Watson-Crick base pairing to target DNA sequences yet have a modified chemical backbone. The xenonucleic acid oligomers (Fig. 8) are highly effective at hybridizing to target sequences and can be employed as molecular clamps in quantitative real-time polymerase chain reactions (PCR) or as highly specific molecular probes for detection of nucleic acid target sequences.

The invention allows for a new way to screen for somatic mutations that utilizes the sequence-specific XNA clamps as described above (i.e. SEQ ID NOs: 22-53) that suppress PCR amplification of wild-type template DNA. The clamps allow selective PCR amplification of only mutant templates, which allows the detection of mutant DNA in the presence of a large excess of wild-type templates from a variety of samples including FFPE, liquid biopsy, and traditionally challenging cytology samples, wherein the mutations are selected from the group consisting of APC 1309, APC 1367, APC 1450, BCT 41, BCT 45, KRAS 12, KRAS 13 and BRAF V600.

The molecular clamps of the present invention for qPCR are synthetic oligomers containing natural A,T,C,G or modified nucleosides (15 to 25 nt long) and have hydrophilic and neutral backbone (no phosphate group like PNA) and undergo hybridization by Watson-Crick pairing. The benefits of XNA include resistance to any known nucleases, much higher binding affinity as DNA binding is independent of salt concentration and large melting temperature differential ($\Delta Tm$ = 15-20°C) in single-nucleotide (SNP's) and insertion/deletions (indels) (5-7°C for natural DNA).

The method of the invention utilizes sequence-specific clamps (Xeno-Nucleic Acid XNA probe) that suppresses PCR amplification of wild-type DNA template and selectively amplifies only mutant template, wherein the mutations, XNA clamps and primers of the present invention are selected form the groups as described above (i.e. SEQ ID NOs: 22-53). The assay and kits of the invention represent a rapid, reproducible solution which employs a simple workflow and PCR machines that are commonly used in research and clinical labs.

The xenonucleic acids that can be used in the present invention include functionality selected from the group consisting of azide, oxaaza and aza. Many XNA's are disclosed in Applicant's pending U.S. application No. 15/786,591 filed October 17, 2017.

The biological samples useful for conducting the assay of the invention include, but are not limited to, whole blood, lymphatic fluid, serum, plasma, buccal cells, sweat, tears, saliva, sputum, hair, skin, biopsy, cerebrospinal fluid (CSF), amniotic fluid, seminal fluid, vaginal excretions, serous fluid, synovial fluid, pericardial fluid, peritoneal fluid, pleural fluid, transudates, exudates, cystic fluid, bile, urine, gastric fluids, intestinal fluids, fecal samples, and swabs, aspirates (e.g., bone marrow, fine needle, etc.), washes (e.g., oral, nasopharyngeal, bronchial, bronchialveolar, optic, rectal, intestinal, vaginal, epidermal, etc.), and/or other specimens.

Any tissue or body fluid specimen may be used as a source for nucleic acid for use in the technology, including forensic specimens, archived specimens, preserved specimens, and/or specimens stored for long periods of time, e.g., fresh-frozen, methanol/acetic acid fixed, or formalin-fixed paraffin embedded (FFPE) specimens and samples. Nucleic acid template molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA. A sample may also be isolated DNA from a non- cellular origin, e.g. amplified/isolated DNA that has been stored in a freezer.

Nucleic acid molecules can be obtained, e.g., by extraction from a biological sample, e.g., by a variety of techniques such as those described by Maniatis, et al. (1982) Molecular Cloning: A Laboratory Manual, Cold Spring Harbor, N.Y. (see, e.g., pp. 280-281).

The XNA-PCR chemistry is also the most reliable tool and it is the only technology that provides detection sensitivity of 0.1% or lower, a level that cannot be achieved by droplet digital PCR and Sanger sequencing. The assays can be completed in two hours for a variety of specimens, including solid tumors (e.g. FFPE tissues) and liquid biopsies (e.g. circulating tumor DNA).

**Mutations Interrogated by primers and XNAs:**

*KRAS codon 12 any non-synonymous other than wild-type GGT --->  GXT, XGT etc. Gly--->Asp, Ser, Val, Arg, Ala, Cys*

*KRAS codon 13 - GGC--> GAC Gly>Asp*

*BRAF codon 600 GTG --> GAG, V600E (V600K, D, R or M)*

*CTNNB1*

*codon 33 TCT --> TAT, Ser --> Tyr,*

*codon 41 ACC > GCC Thr > Ala,, ACC > ATC Thr > Ile*

*codon 45 TCT > CCT Ser > Pro, TCT > TTT  Ser > Phe*

*APC codon 1309  delAAAAG*

   *codon 1367  CAG > TAG   Glu > Stop*

   *codon 1450  CGA > TGA   Arg > Stop, 7bpdel*

    *codon 1465  delAG*

    *codon 1556 insA, delA*

   *codon 877*

*TGFBR2  c449_458del p.E150fs*

Primers designed to amplify regions containing each of the target mutations in the target genes are used together with wild-type sequence specific PCR clamp oligomers: peptide nucleic acid (PNA) locked nucleic acids (LNA), bridged nucleic acid (BNA) or more preferably xenonucleic acid clamp oligomers as previously disclosed (Ref DiaCarta XNA patent filings). The PCR reaction is performed and the resulting amplicons generated are detected by real-time fluorescence based PCR using SYBR green intercalating dye or fluorescent 5'-exonuclease hydrolysis probes (taqman). Alternatively the amplicons can be detected employing sequence specific hybridization capture and detection and solid-phase separation techniques.

The gene mutation specific primers and PCR clamp reactions are performed together with primers that are designed to amplify a housekeeping gene such as $\beta$-Actin (ACTB). The housekeeping gene provides a means to monitor the quality and quantity of the input DNA that is obtained from colon cancer tissue biopsy samples, circulating free tumor DNA in patient's plasma or tumor DNA extracted from patient stool samples.

## EXAMPLE I

**Reagents for the stool sample preparation.**

QIAamp DNA Stool Mini Kit. Ca# 51504. Good for 50 ×200mg stool samples.

Qiagen reagents for the large scale (whole stool) preparation:

Buffer ASL. Ca# 1014755. Buffer AL, Ca# 1014600, Buffer AW1 Ca# 1014792, Buffer AW2 Ca# 1014592, InhibitEx tablets, Ca# 19590, RNAse A Ca# 1007885, Proteinase K Ca#19131.
Our storage buffer: 10mM NaCl, 500mM TrisHCl pH9.5, 100mM EDTA. Neutralization buffer: 1M MES pH 5.76 (Teknova). Silica maxi spin columns. Epoch BioSciences ( Ca# 2040-050).
Streptavidin coated Magnetic Dynabeads MyOne (Thermo Fisher Scientific, Ca# 00351575) are the best for DNA capturing. 20XSSC buffer. Beckman Coulter Sorvall centrifuge with JA25.50 rotor and 50 ml centrifugation tubes with screw caps (Ca# 357003). BeckmanCoulter AllegraX-15 bench top centrifuge with SX4750 rotor for maxi columns.

The procedure described below is for the whole stool covered with the storage buffer added by patient. If stool is in the frozen state we recommend to take about 10 × 2 g pieces and add 10 volumes (20 ml) of ASL buffer to each piece. Allow stool to thaw and continue as described.
Add the minimal volume of storage buffer to the fresh stool just to cover stool surface (no more!) Mix suspension with glass rod for few minutes to make it more homogeneous. Close the container and incubate for 16-24h at room temperature.

Determination of stool concentration.

Mix stool and transfer 2-3 spoons of stool suspension into the graduated 50 ml conical tube to determine the volume of an aliquot.
Spin the aliquot at ~20000 g for 5 min. Discard the supernatant and determine the weight of the pellet.

Example

| Aliquot of stool | |
|---|---|
| Volume | 27 ml |
| Weight of Pellet | 15 g |
| Stool concentration | 15 g / 27 ml = 0.55 g/ml |
| Total stool | 0.55 g/ml x 200 ml = 110 g |

Discard the tube with the pellet.
To start DNA purification from **2 g** of stool takes **2 g / 0.55 g/ml =3.6 ml** of the liquid stool. To start with **200 mg** use **360 µl** of stool.

**Isolation of human DNA from 200mg of stool in the storage buffer.**

This procedure is modification of Qiagen's QIAamp protocol. It is recommended for the training purposes. It is quick and may be performed using DNA stool mini kit (Ca# 51504). Mix the liquid stool and transfer 360 µl (200 mg) into 15 ml graduated conical tube. Add 3.6 ml (10 volumes) of ASL buffer. Vortex. Incubate at room temperature for 5min. Add 360 µl 1M MES pH 5.76 buffer. Vortex. Volume = 4.32 ml
Distribute 2 ml × 2 into two 2 ml centrifugation tubes. Spin at 10000-13000 g for 5 min in the bench top centrifuge. Combine supernatants in 15 ml conical tube. Add 1 µl RNAse A (100 mg/ml, Qiagen). Mix and incubate for 5 min. Add 1 InhibitEx tablet (Qiagen). Vortex for about 1 min until tablet is completely dispersed. Transfer the whole mix into two 2 ml tubes. Spin at 13000 g for 5 min. Combine the supernatants in 15 ml conical tube. Add 25 **µl** Proteinase K. Mix. Add the equal volume of AL buffer. Mix. Incubate at 70°C for 20 min in the water bath. Cool the mix to the room temperature. Add the volume of ethanol equal to that of AL buffer.

Mix. Load 0.7 ml mix repeatedly onto one silica column. (It may take more than 10 loadings. See details in instruction to the kit).

Briefly:

a). Perform each loading of sample at 6000 g for 1 min.
b). Washings. 500 µl of AW1 buffer at 6000 g for 1 min.
500 µl of AW2 buffer at 10000 for 1 min, twice.
c). Spin dry column at 13000 g for 2 min.
d). Elution. Load 50-100 µl AE buffer onto silica column. Incubate for 1 min. Spin at 13000 g for 2 min.

### Isolation of human DNA from 2 g of stool in the storage buffer.

Mix the liquid stool and transfer 3.6 ml (2 g) into 50 ml graduated conical tube. Add 36ml

(10 volumes) of ASL buffer. Vortex. Incubate at room temperature for 5 min. Add 3.6 ml of 1M MES pH 5.76 buffer. Vortex. Volume = 43.2 ml. Transfer mix into 50 ml centrifugation tube (Beckman Coulter, Ca# 357003). Spin at 20000 g for 10 min. Collect supernatant in 50ml conical tube. Add 4 µl RNAse A (100mg/ml, Qiagen). Mix and incubate for 5 min. Add 4 InhibitEx tablets (Qiagen). Vortex for about 1 min until tablets are completely dispersed. Transfer the whole mix into 50 ml centrifugation tube. Spin at 20000 g for 10 min. Collect the supernatant in 50 ml conical tube. Add 250 µl Proteinase K. Mix. Add the equal volume of AL buffer. Mix. Incubate at 70°C for 20 min in the water bath. Cool the mix to the room temperature. Add the volume of ethanol equal to that of AL buffer.

Option 1:

1. Use the beads from the bead vial of the Promega Maxwell® RSC Whole Blood DNA Kit

1.1. Resuspend the Bead Mix in the 2nd well of the kit cartridge and
1.2. Transfer the whole content of the bead mixture to the solution from step 3.7
1.3. Incubate for 0.5 hour at RT on an orbital shaker at moderate speed to bind NA to the NA Binding Beads.
1.4. Pull down the beads with the magnet (optional: Spin down and discard supernatant). Save about 500 µl supernatant with beads.
1.5. Transfer bead suspension into the bead compartment of the kit cartridge and proceed with the kit-specific program.
1.6. Use 150 µl elution volume

The DNA is ready for qPCR.

Option 2:

Mix. Load ~20 ml mix repeatedly onto one Maxi silica column inserted into 50ml conical tube. (It may take more than 5 loadings).

a) Perform each loading of sample at 1850 g for 3 min in "Allegra X-15R centrifuge, bucket rotor SX4750, Beckman Coulter)
b) Washings. 5 ml of AW1 buffer at 4500 g for 1 min.
5 ml of AW2 buffer at 4500 g for 15 min.
d) Elution. Load 1 ml of AE buffer onto silica column. Incubate for 5 min. Spin at 4500 g for 2 min.

### Concentration of DNA isolated from 2 g of stool before the sequence dependent capture.

The volume of DNA eluted from maxi column is about 700 µl. Denature DNA by heating at 95°C for 5 min.
Precipitation of DNA.
Transfer DNA into 2ml centrifugation tube. Add 70 µl of 5 M NaCl + 70µl of 5 N NH₄Ac + 700 µl isopropanol. Incubate for 1h at room temperature. Precipitate DNA by centrifugation at 13000 g for 15 min in the bench top centrifuge.

Wash pellet with 70% EtOH. Dry pellet at 55°C for 5 min.

Dissolve DNA in 35 µl of 10 mM Tris pH8.0.

**Enrichment of eluted DNA with the target specific capture 5'-BIOprobe on the magnetic beads.**

Put 35 µl of eluted DNA into 0.2 ml tube and incubate at 95°C for 2 minutes in thermocycler with the preheated lid. Chill the tube on ice for 2 minutes. Transfer denatured DNA into the new 0.2 ml tube. Assemble hybridization mix as shown in the Table below.

| Components | Volume µl | Final concentration |
|---|---|---|
| DNA | 30 | |
| 1 µM Capture probe | 1.5 | 33 nM |
| 20 x SSC | 15 | ~7X (1 MNaCl) |

Perform hybridization in thermocycler at: 95°C 4 min - 58°C 1h.

Prepare magnetic beads during hybridization.

**Washing of magnetic beads**.

Vortex beads in bottle for 20 sec. Transfer 10 µl (10^9 beads) to the bottom of 0.5 ml tube. Place tube on the magnet for 1 min. Remove the liquid covering the concentrated beads. Remove tube from the magnet. Add 100 µl of 1X B&W buffer and suspend beads by gentle aspiration. Put the tube on the magnet. Repeat the washing step with 50 µl of 1X B&W. Cover beads with 50 µl of 1X B&W to prevent beads from drying.

**Capture of hybridization product on the magnetic beads.**

Put the tube with the washed beads covered with 1X B&W on magnet and remove supernatant without disturbing beads. Remove tube from the magnet and immediately suspend beads in 10 µl of B&W buffer. Remove the post hybridization mix from the thermocycler. Add 7 µl of washed beads. Suspend beads by the gentle aspiration. Place tubes into shaker for 2 hours at 1100 rpm. The speed of the shaking should be high enough to don't allow beads to precipitate.

**Washing of beads with captured DNA.**

Collect beads on the magnet. Aspirate the supernatant. Suspend beads in 100 µl B&W. Repeat this wash step with 50 µl of B&W. Repeat the step with suspending beads in 50 µl of 10 mM NaCl + 20 mM TrisHCl pH 7.5 (high stringency wash buffer).

**Elution of DNA from the beads.**

Aspirate the low stringency wash buffer from beads. Suspend beads in 20 µl of 20 ng/µl of poly A or other homopolymer carrier. Place tubes into thermocycler and heat at 70°C for 5 min to elute DNA. Place tube on magnet and collect ~ 20 µl of captured human DNA.

**Sequences of biotinylated capture probes**

**KRAS 01S** SEQ ID NO: 1

5´ -/5Biosg/ GAT ACA GCT AAT TCA GAA TCA TTT TGT GGA CGA ATA TGA TCC AAC AAT AGA GGT AAA TCT TGT TTT AAT ATG CAT ATT ACT GGT GCA GGA CCA TT-3´

**BRAF 01S** SEQ ID NO: 2

5′ -/5Biosg/ AAG TCA ATC ATC CAC AGA GAC CTC AAG AGT AAT AAT ATA TTT
CTT CAT GAA GAC CTC ACA GTA AAA ATA GGT GAT TTT GGT CTA GCT ACA-3′

**ACTB 02AS** SEQ ID NO: 3

5′ -/5Biosg/ AGG AAG GAA GGC TGG AAG AGT GCC TCA GGG CAG CGG AAC
CGC TCA TTG CCA ATG GTG ATG ACC-3′

**APC 01S** SEQ ID NO: 4

5′ -/5Biosg/ TTG TCA TCA GCT GAA GAT GAA ATA GGA TGT AAT CAG ACG ACA
CAG GAA GCA GAT TCT GCT AAT ACC CTG CAA ATA GCA GA-3′

**CTNNB 02AS** SEQ ID NO: 5

5′-/5Biosg/ GTA AAG GCA ATC CTG AGG AAG AGG ATG TGG ATA CCT CCC AAG
TCC TGT ATG AGT GGG AAC AGG GAT TTT CTC AGT-3′

## EXAMPLE II

55 blinded samples of DNA extracted from plasma and FFPE of patients with known clinical and mutational status were provided as 15ul aliquots in microcentrifuge tubes. Some samples were from tumor tissue of colon cancer patients. Samples were labeled from 1 to 55 with the ID# on the sides of tubes.

The goal of the test was for detection of mutations in plasma of colon cancer patients. Samples were accessioned according to accessioning and sample traceability SOPs.

The quantity and qPCR readiness of the DNA was checked by qPCR using the reference amplicon from the internal control of the assay. The samples then were diluted accordingly and tested using the assay. All positive calls for any of the target mutations were confirmed by Sanger sequencing of the amplicons.

### 1. Methodology

### Technology for Mutation Detection

The Colorectal Cancer Mutation Detection Kit of the invention is based on xenonucleic acid (XNA) mediated PCR clamping technology. XNA is a synthetic DNA analog in which the phosphodiester backbone has been replaced by a repeat formed by units of (2-aminoethyl)-glycine. XNAs hybridize tightly to complementary DNA target sequences only if the sequence is a complete match. Binding of XNA to its target sequence blocks strand elongation by DNA polymerase. When there is a mutation in the target site, and therefore a mismatch, the XNA:DNA duplex is unstable, allowing strand elongation by DNA polymerase. Addition of an XNA, whose sequence with a complete match to wild-type DNA, to a PCR reaction, blocks amplification of wild-type DNA allowing selective amplification of mutant DNA. XNA oligomers are not recognized by DNA polymerases and cannot be utilized as primers in subsequent real-time PCR reactions. The qPCR detection is Taqman-based.

### qPCR Assay

The assay of the invention is a real-time PCR based *in vitro* diagnostic assay for qualitative detection of colorectal cancer - associated biomarkers including *APC (codons 1309, 1367, 1450), KRAS (codons 12 and 13), BRAF (codon 600) and CTNNB1 (codons 41 and 45)* . The detection kit identifies the presence or absence of mutations in the targeted regions but does not specify the exact nature of the mutation. The detection kits are designed to detect any mutation at or near the stated codon site without specifying the exact nucleotide change.

*Table 1. List of Mutations and Cosmic Identities Found in the targeted genes of the invention*

| KRAS | | | |
|---|---|---|---|
| **Exon** | **Amino Acid Change** | **Nucleotide change** | **Cosmic No.** |
| | G12>A | c.35G>C | 522 |
| | G12>R | c.34G>C | 518 |
| | G12>D | c.35G>A | 521 |
| | G12>C | c.34G>T | 516 |
| **2** | G12>S | c.34G>A | 517 |
| | G12>V | c.35G>T | 520 |
| | G13>D | c.38G>A | 532 |
| | G13>C | c.37G>T | 527 |
| | G13>R | c.37G>C | 529 |

| APC | | | |
|---|---|---|---|
| **Exon** | **Amino Acid Change** | **Nucleotide change** | **Cosmic No.** |
| | E1309fs* | c.3921_3925delAAAAG | COSM18764 |
| **15** | Q1367* | c.4099C>T | COSM13121 |
| | R1450* | C.4348C>T | COSM13127 |

| CTNNB1 | | | |
|---|---|---|---|
| **Exon** | **Amino Acid Change** | **Nucleotide change** | **Cosmic No.** |
| | p.T41A | c.121A>G | COSM5664 |
| | p.T41I | c. 122C>T | |
| | p.S45P | c.1331>C | COSM5663 |
| | P.S45F | c.134C>T | |

| BRAF | | | |
|---|---|---|---|
| **Exon** | **Amino Acid Change** | **Nucleotide change** | **Cosmic No.** |
| | p.V600E | c.1799T>A | COSM476 |
| **15** | p.V600K | c.1798_1799GT>AA | COSM473 |
| | p.V600R | c.1798_1799G1>AG | COSM474 |
| | p.V600D | c.1799_1800TG>AT | COSM477 |

Table 1 above shows a list of mutations commonly found in the targeted gene that can be detected by the kit. The assay and kit is to be used by trained laboratory professionals within a laboratory environment.

**Table 2 Reagents and Instruments Used**

| Vial No. | Name of Component | Description | Volume, 24-test kit | Volume, 6-test kit |
|---|---|---|---|---|
| 1 | APC cl309 and 1367 Primer/ Probe Mix | APC c1309 and 1367 Primers and probe | 1 X 62 μl. | 1 × 15 μL |
| 2 | APC c1309 and c1367 XNA | APC c1309 XNA | 1 X 28 μL | 1 × 7 μL, |

(continued)

| Vial No. | Name of Component | Description | Volume, 24-test kit | Volume, 6-test kit |
|---|---|---|---|---|
| 3 | BCT c41 primer/ probe Mix | BCT c41 Primers and probe | 1 X 62 µl. | 1 × 15 µL |
| 4 | BCT c41 XNA | BCT c41 XNA | 1 X 28 µL. | 1 × 7 µL |
| 5 | APC c1450 Primer/Probe Mix | APC cl450 Primer and probe | 1 X 62 µL | 1 × 15 µL |
| 6 | APC C1450 XNA | APC C1450 XNA | 1 X 28 µL | 1 X 7 µL |
| 7 | BCT c45 Primer/ probe Mix | BCT c45 Primers and probe | 1 X 62 µL | 1 X 15 µL |
| 8 | BCT c45 XNA | BCT c45 XNA | 1 X 28 µL | 1 X 7 µL |
| 9 | KRAS cl2 Primer/ Probe mix | KRAS cl2 Primer/probe | 1 X 62 µL | 1 X 15 µL |
| 10 | KRAS cl2 XNA | XNA for KRAS c 12 | 1 X 28 µL | 1 X 7 µL |
| 11 | KRAS c13 Primer/Probe mix | KRAS c13 Primer/probe | 1 X 62 µL, | 1 X 15 µL |
| 12 | KRAS c13 XNA | XNA for KRAS c 13 | 1 X 28 µL | 1 X 7 µL |
| 13 | BRAF c600 Primer/Probe Mix | BRAF V600 Primers and probe | 1 X 62 µL | 1 X 15 µL |
| 14 | BRAF c600 XNA | BRAF V600 XNA | 1 X 28 µL | 1 X 7 µL |
| 15 | 2X Assay qPCR Master Mix | PCR Reaction Premix | 1043 µL | 287 µL |
| 16 | Negative Control | Wild-tvpe DNA | 1 X 56 µL | 1 X 28 µL |
| 17 | Positive Control | APC c1309, c1367, c1450, BCT c41, BCT c45, KRAS c12, KRAS c13 BRAF c600 mutant templates Template | 1 X 56 µL | 1 X 28 µL |
| 18 | Non template control | Nuclease-Free Water | 1 X 76 µL | 1 X 56 µL |

### *Materials Required*

### Reagents for DNA Isolation

QIAamp DSP DNA FFPE Tissue Kit (QIAGEN, Cat. No. 60404) or equivalent

QIAamp Circulating Nucleic Acid Kit (QIAGEN, Cat. No. 55114) or equivalent

### Consumables

0.2 ml DNase-free PCR tubes or plates, nuclease-free, low-binding micro centrifuge tubes and nuclease-free pipet tips with aerosol barriers.

**Equipment**

Permanent marker, real time PCR instrument, dedicated pipettes* (adjustable) for sample preparation, dedicated pipettes* (adjustable) for PCR master mix preparation, dedicated pipettes* (adjustable) for dispensing of template DNA, micro centrifuge, bench top centrifuge* with rotor for 1.5 ml tubes, vortex, PCR rack, reagent reservoir, distilled water. * Prior to use ensure that instruments have been maintained and calibrated according to the manufacturer's recommendations.

***Instruments***

The assays have been developed and validated on the instruments shown in the table below. Instrument platforms not listed in the table should be validated by the individual labs. Guidance for validation can be obtained from DiaCarta upon request.

*Table 3. List of Instruments Validated with This Kit.*

| Company | Model |
|---|---|
| Roche | Light cycler 96 |
| Roche | Light cycler 480 |
| Bio-rad | CFX384 |
| ABI | QuantStudio 5 |

The kits of the invention should be stored at -20 °C immediately upon receipt, in a constant-temperature freezer and protected from light. When stored under the specified storage conditions, the kit is stable until the stated expiration date. It is recommended to store the PCR reagents (Box land 2) in a pre-amplification area and the controls (Box 3) in a postamplification (DNA template-handling) area. The kit can undergo up to 6 freeze-thaw cycles without affecting performance.

**DNA Isolation**

Human genomic DNA must be extracted from fixed paraffin-embedded tissue, frozen tissue or plasma prior to use. Several methods exist for DNA isolation. For consistency, we recommend using a commercial kit, such as Qiagen DNA extraction kit (QIAamp DNA FFPE Tissue Kit, cat No. 56404, for paraffin embedded specimens; DNeasy Blood & Tissue kit, cat. No. 69504 or 69506, for tissue and blood specimens, QIAamp Circulating Nucleic Acid Kit, cat. No. 55114 for plasma). Follow the genomic DNA isolation procedure according to manufacturer's protocol. Sufficient amounts of DNA can be isolated from FFPE blocks or fresh frozen sections as well as plasma (approx. 2-10 $\mu$g).

This assay requires a total of 22.5 - 35 ng of DNA per sample (2.5-5 ng/reaction). After DNA isolation, measure the concentration using fluorometric analysis (i.e. Qubit) and dilute to 1.25-2.5 ng/$\mu$l. If using spectrophotometric analysis, make sure the A260/A230 value is greater than 2.0 and A260/A280 value between 1.8 and 2.0.

**Preparation of Reagents**

A 24-test kit contains enough control material for 3 runs. Thaw all primer and probe mixes, XNAs, Positive Control, WT Negative Control, Nuclease-Free Water and 2X PCR mastermix provided. Thaw all reaction mixes at room temperature for a minimum of 30 minutes. Vortex all components except the PCR Master Mix and Primer and probe Mix for 5 seconds and perform a quick spin. The PCR Master Mix and Primer/probe mix should be mixed gently by inverting the tube a few times. Prior to use, ensure that any precipitate in the PCR Master Mix is re-suspended by pipetting up and down multiple times. Do not leave kit components at room temperature for more than 2 hours. The PCR reactions are set up in a total volume of 10 $\mu$l/reaction.

Table 4 shows the component volumes for each 10 ul reaction.

| Components | Volume/Reaction |
|---|---|
| 2X PCR Master mix | 5 $\mu$l |
| Primer and probe Mix | 2 $\mu$l |

(continued)

| Components | Volume/Reaction |
|---|---|
| XNA | 1 $\mu$l |
| DNA sample or Controls | 2 $\mu$l |
| Total volume | 10 $\mu$l |

For accuracy, 2x PCR Mastermix, primers and XNA should be pre-mixed into assay mixes as described in Table 5 below.

### *Preparation of Assay Mixes*

Assay mixes should be prepared just prior to use. Label a micro centrifuge tube (not provided) for each reaction mix, as shown in Table 5. For each control and mutation detection reaction, prepare sufficient working assay mixes for the DNA samples, one Positive Control, one Nuclease-Free Water for No-Template Control (NTC), and one WT Negative Control, according to the volumes in Table 5. Include reagents for 1 extra sample to allow sufficient overage for the PCR set up. The assay mixes contain all of the components needed for PCR except the sample.

Table 5. Preparation of Assay Mixes.

| | Volume of 2X PCR Master Mix | Volume of Primer and probe Mix | Volume of XNA |
|---|---|---|---|
| APC 1309 and 1367 Mix | 5 $\mu$l x (n+1) | 2 $\mu$l x (n+1) | 1 $\mu$l x (n+1) |
| APC 1450 Mix | 5 $\mu$l x (n+1) | 2 $\mu$l x (n+1) | 1 $\mu$l x (n+1) |
| CTNNB 1 41 Mix | 5 $\mu$l x (n+1) | 2 $\mu$l x (n+1) | 1 $\mu$l x (n+1) |
| CTNNB1 45Mix | 5 $\mu$l x (n+1) | 2 $\mu$l x (n+1) | 1 $\mu$l x (n+1) |
| KRAS 12 Mix | 5 $\mu$l x (n+1) | 2 $\mu$l x (n+1) | 1 $\mu$l **x** (n+1) |
| KRAS 13 Mix | 5 $\mu$l x (n+1) | 2 $\mu$l x (n+1) | 1 $\mu$l x (n+1) |
| BRAF c600 Mix | 5 $\mu$l x (n+1) | 2 $\mu$l x (n+1) | 1 $\mu$l **x** (n+1) |
| Note: n = number of reactions (DNA samples plus 3 controls). Prepare enough for 1 extra sample (n +1) to allow for sufficient overage for the PCR set. You may want to consider increasing volume of mix to (n+2) when processing larger number of samples. | | | |

Negative Control, Positive Control and Non template control must be run with each reaction mix, every time the assay is run.

### **Negative Control:**

- Uses commercially available wild-type human genomic DNA as the template at 2.5ng/pl concentration.
- No target mutations, efficient binding by XNA clamps suppressing the target amplification.

### **Positive control:**

- A mix of synthetic reference mutant templates for each target of the assay at 5% allelic frequency in 2.5 ng/$\mu$l WT human genomic DNA (hgDNA).
- XNA clamps will not bind, allowing amplification of the mutant template.
- Positive controls must show the appropriate values in both HEX and FAM channels for the run to be valid.

**Non- template control (NTC):**

- Nuclease free water is used in the place of template
- No amplification should be observed in both HEX and FAM channel, assuring the absence of contamination during assay set-up.

The Internal Control assay uses ACT-B housekeeping gene as a reference gene to assess the quality of amplifiable DNA and demonstrating if the reagents are working correctly. When assessed using the HEX channel, this control should make amplicons efficiently for all samples and controls except NTC, providing another way to monitor performance of the primers, probes, polymerase, and sample DNA quality/quantity.

Please always use white plates, strips, or tubes. In pre-amplification area, add 8 μl of the appropriate assay mix to the plate or tubes. In designated template area, add 2 μl of template to each well.

Table 6. Suggested Plate Layout.

|   | 1 | 2 | 3 | 4 | 5 | 6 |
|---|---|---|---|---|---|---|
| A | NTC APC1309, 1367 Mix | PC APC 1309, 1367 Mix | CC APC 1309, 1367 Mix | S1 APC 1309, 1367 Mix | S2 APC 1309, 1367 Mix | S3 APC 1309, 1367 Mix |
| B | NTC APC 1450 Mix | PC APC 1450 Mix | CC APC 1450 Mix | S1 APC 1450 Mix | S1 APC 1450 Mix | S3 APC 1450 Mix |
| C | NTC CTNNB1 41 Mix | PC CTNNB1 41Mix | CC CTNNB 1 41 Mix | S1 CTNNB 1 41 Mix | S2 CTNNB1 41 Mix | S3 CTNNB1 41Mix |
| D | NTC CTNNB 1 45 Mix | PC CTNNB1 45 Mix | CC CTNNB1 45 Mix | S1 CTNNB 1 45 Mix | S2 CTNNB 1 45 Mix | S3 CTNNB1 45 Mix |
| E | NTC KRAS 12 Mix | PC KRAS 12 Mix | CC KRAS 12 Mix | S1 KRAS 12 Mix | S2 KRAS 12 Mix | S3 KRAS 12 Mix |
| F | NTC KRAS 13 Mix | PC KRAS 13Mix | CC KRAS 13Mix | S1 KRAS 13 Mix | S2 KRAS 13 Mix | S3 KRAS 13 Mix |
| G | NTC BRAF c600 Mix | PC BRAF c600 Mix | CC BRAF c600 Mix | S1 BRAF c600 Mix | S2 BRAF c600 Mix | S3 BRAF c600 Mix |
| PC: Positive Control, NTC: No-Template Control (water), CC: Negative Control (Wild-type DNA), S1-3: Samples 1-3 ||||||

Table 6 is a suggested plate set-up for a single experiment analyzing 3 unknown samples. Please disregard any assay mixes listed below that are not part of your kit. When all reagents have been added to the plate, tightly seal the plate to prevent evaporation. Spin at 1000rpm for 1 minute to collect all the reagents. Place in the real-time PCR instrument immediately.

**Instrument Set-Up**

Roche Light cycler 96 and Light cycler 480, Bio-Rad CFX 384 and ABI QuantStudio 5

1) Selection of Detectors:

    i. Use 'FAM/HEX' as the Detector on Roche light cycler

    ii. Select 'All Channel' as detection format on Bio-Rad CFX384

    iii. For ABI QuantStudio 5, assign individual mutation target as 'FAM', and select all Targets and assign to VIC

2) Setup the cycling parameters as shown in Table 7a or Table 7b

3) Start the run

Table 7a. Roche Light Cycler and Bio-Rad CFX 384 Platforms Cycling Parameters

| Step | Temperature (°C) | Time (Seconds) | Ramp Rate (°C/s) for Roche instruments * | Cycles | Data Collection |
|---|---|---|---|---|---|
| PreIncubation | 95 | 300 | 4.4 | 1 | OFF |
| Denaturation | 95 | 20 | 4.4 | X50 | OFF |
| XNA Annealing | 70 | 40 | 2.2 | | OFF |
| Primer | 64 | 30 | 1 | | OFF |
| Extension | 72 | 30 | 1 | | FAM and |
| *On Bio-Rad CFX 384, use the default ramp rate | | | | | |

Table 7b. ABI QuantStudio 5 Cycling Parameters

| Step | Temperature (°C) | Time (Seconds) | Ramp Rate (°C/s) | Cycles | Data Collection |
|---|---|---|---|---|---|
| PreIncubation | 95 | 300 | 1.6 | 1 | OFF |
| Denaturation | 95 | 20 | 1.6 | X50 | OFF |
| XNA Annealing | 70 | 40 | 1.6 | | OFF |
| Primer Annealing | 66 | 30 | 1 | | OFF |
| Extension | 72 | 30 | 1 | | FAM and VIC |

**Assessment of Real-Time PCR Results**

The real-time PCR instrument generates a cycle threshold (Cq, also called as Ct) value for each sample. Cq is the cycle number at which a signal is detected above the set threshold for fluorescence. The lower the cycle number at which signal rises above background, the stronger the PCR reaction it represents and the higher initial template concentration (**please see MIQE Guidelines under References for more information).

*• Data Analysis for Light Cycler 480*

For the Light Cycler 480, open the LightCycler480 SW 1.5.1.61 and select Abs Quant/2nd Derivative Max algorithm to analyze the run file data.

*• Data Analysis for Bio-Rad CFX384*

For the BioRad CFX384, open the qPCR run file using BioRad CFX manager. In the Log scale view, adjust the threshold to 100 ±20 for HEX and 300 ±60 for FAM. Export the Cq data to excel. Exact threshold setting may be different for individual instruments.

• *Data Analysis for ABI QuantStudio 5*

For the ABI Quant Studio 5 instrument, adjust the threshold according to **Table 8**. Exact threshold setting may be different for individual instruments.

Export the Cq data to excel. For each control or sample, calculate the difference in Cq value between the mutation assay and the Internal Control Assay as follows: Cq difference

$$(\Delta Cq) = \text{Mutation Assay Cq - Internal Control Assay Cq}$$

Table 8. ABI QuantStudio 5 Recommended Threshold

| Target | Recommended threshold |
|---|---|
| ACT-B (internal control) | $5900 \pm 600$ |
| APC c1309/1367 | $100000 \pm 10000$ |
| APC c1450 | $20000 \pm 2000$ |
| CTNNB1 c41 | $20000 \pm 2000$ |
| CTNNB1 c45 | $8000 \pm 800$ |
| KRAS c12 | $20000 \pm 2000$ |
| KARS c13 | $20000 \pm 2000$ |
| BRAF c600 | $20000 \pm 2000$ |

**Evaluation of Controls**

Verify that no amplification is observed in the non-template controls (NTC) for each of the reaction mixes. Cq should be Undetermined. For each control or sample, calculate the difference in Cq value between the mutation assay and the External Control Assay as follows:

$$\text{Cq difference } (\Delta Cq) = \text{Mutation Assay Cq - Internal Control Assay Cq}$$

**Negative and Positive Controls:** For the assay to be valid, the Negative Control and Positive Control must meet the criteria in Table 9a and Table 9b.

Table 9a. Acceptable values for positive controls and negative controls on Roche Light Cycler 480 and Bio-Rad CFX384

| Assay | Positive | Negative |
|---|---|---|
| Internal Control | 25 < Cq < 31 | 25< Cq <31 |
| APC 1309/1367 | $\Delta Cq \leq 4.7$ | $\Delta Cq > 20.7$ |
| APC 1450 | $\Delta Cq \leq 2.6$ | $\Delta Cq > 9.8$ |
| CTNNB1 41 | $\Delta Cq \leq 0$ | $\Delta Cq > 7.1$ |
| CTNNB 1 45 | $\Delta Cq \leq 3.4$ | $\Delta Cq > 7.3$ |
| KRAS12 | $\Delta Cq \leq 6$ | $\Delta Cq > 10.6$ |
| KRAS 13 | $\Delta Cq \leq 3.3$ | $\Delta Cq > 7.3$ |
| BRAF V600 | $\Delta Cq \leq 2.4$ | $\Delta Cq > 7.5$ |

Table 9b. Acceptable values for positive controls and negative controls on ABI QuantStudio 5

| Assay | Positive | Negative |
|---|---|---|
| Internal Control | $25 \leq Cq \leq 30$ | $25< Cq <30$ |
| APC 1309/1367 | $\Delta Cq \leq 4.6$ | $ACq>20.3$ |
| APC 1450 | $\Delta Cq \leq 3.4$ | $\Delta Cq>9.4$ |
| CTNNB1 41 | $\Delta Cq \leq 0.5$ | $\Delta Cq>7.2$ |
| CTNNB1 45 | $\Delta Cq \leq 3.1$ | $\Delta C>7.5$ |
| KRAS12 | $\Delta Cq \leq 4.2$ | $\Delta Cq>13.2$ |
| KRAS13 | $\Delta Cq \leq 5.7$ | $\Delta Cq>10.0$ |
| BRAF V600 | $\Delta Cq \leq 3.7$ | $\Delta Cq >7.6$ |

**Evaluating Validity of Sample Data Based on Internal Control Results**

The Cq value of the Internal Control Mix serve as an indication of the purity and concentration of DNA in each well. Thus, the validity of the test can be decided by the Cq value of the Internal Control mix. Cq values of any sample with Internal Control Mix should be in the range of $25<Cq<31$ (Roche Light cycler 480 and Bio-Rad CFX 384) or $25<Cq<30$ (ABI QuantStudio 5). If the Cq values fall outside this range, the test results should be considered invalid. The experiment should be repeated following the recommendations in Table 10.

Table 10. Acceptable internal control Cq ranges for samples

| Validity | Cq Value of Internal Control Mix | | Descriptions and Recommendations |
|---|---|---|---|
| | Roche LC480 Bio-Rad CFX 384 | ABI QuantStudio 5 | |
| | $25 < Cq$ | $25 < Cq < 30$ | The amplification and amount of DNA sample were optimal. |
| **Invalid** | $Cq <25$ | $Cq <25$ | Possibility of a false positive is high. Repeat the PCR reaction |
| **Invalid** | $Cq \geq 31$ | $Cq \geq 30$ | Not enough DNA or DNA not pure. The amplification is not optimal. Check DNA amount and purity. Repeat the experiment with more DNA or a new DNA prep may be |

**Scoring Mutational Status**

If a Cq value is Undetermined, assign a Cq of 50 and proceed to analysis. The tables below should be used to determine mutational status based on $\Delta Cq$ values.

Note: If the Cq value of FAM is 50, the mutational status will be scored as "Negative" regardless of the $\Delta Cq$ values.

Table 11. Scoring mutational status for Roche Light Cycler 480

| Sample type | Mutation | APC c1309/136 | APC c1450 | CTNNB1 c41 | CTNNB1 c45 | KRAS c12 | KRAS c13 | BRAF c600 |
|---|---|---|---|---|---|---|---|---|
| FFPE | **Positive**: | < 19.3 | < 9.0 | < 7.1 | < 6.7 | < 9.5 | < 7.3 | < 7.5 |
| | **Negative** | > 19.3 | > 9.0 | >7.1 | > 6.7 | > 9.5 | > 7.3 | >7.5 |
| Plasma cfDNA | **Positive**: | < 19.2 | < 10.2 | <7.7 | < 10.5 | <12.6 | < 8.4 | < 7.3 |
| | **Negative** | $\geq 19.2$ | $\geq 10.2$ | $\geq 7.7$ | $\geq 10.5$ | $\geq 12.6$ | $\geq 8.4$ | $\geq 7.3$ |

Table 12. Scoring mutational status for Bio-Rad CFX384

| Sample type | Mutation | APC c1309/1367 | APC c1450 | CTNNB 1 c41 | CTNNB1 c45 | KRAS c12 | KRAS c13 | BRAF c600 |
|---|---|---|---|---|---|---|---|---|
| FFPE | Positive: | <13.6 | < 8.1 | < 7.0 | < 8.1 | < 8.9 | <7.0 | < 8.5 |
| | Negative | ≥13.6 | ≥ 8.1 | ≥ 7.0 | ≥ 8.1 | ≥ 8.9 | ≥7.0 | ≥ 8.5 |
| Plasma cfDNA | Positive: | <18.9 | <9.3 | < 7.9 | < 10.2 | < 11.5 | <7.9 | < 9.7 |
| | Negative | >18.9 | > 9.3 | > 7.9 | >10.2 | >11.5 | >7.9 | > 9.7 |

Table 13. Scoring mutational status for ABI QuantStudio 5

| Sample type | Mutation | APC c1309/1367 | APC c1450 | CTNNB 1 c41 | CTNNB1 c45 | KRAS c12 | KRAS c13 | BRAF c600 |
|---|---|---|---|---|---|---|---|---|
| FFPE | Positive: | <20.3 | < 7.6 | < 6.9 | < 6.8 | < 9.7 | < 10 | < 7.6 |
| | Negative | ≥20.3 | ≥ 7.6 | ≥ 6.9 | ≥ 6.8 | ≥ 9.7 | ≥ 10 | ≥ 7.6 |
| Plasma cfDNA | Positive: | <18.1 | < 9.3 | < 6.9 | < 8.5 | < 19.2 | < 11.8 | < 10.7 |
| | Negative | ≥18.1 | ≥ 9.3 | ≥ 6.9 | ≥ 8.5 | ≥ 19.2 | ≥ 11.8 | ≥ 10.7 |

**Differentiating KRAS c12/KRAS c13 Mutational Status**

The KRAS c12 reaction mix detects both KRAS c12 and KRAS c13 mutations, whereas the KRAS c13 reaction mix detects only KRAS c13 mutations. Therefore, in order to differentiate between KRAS c12 and KRAS c 13 mutations a combination of results from the two mixes should be used as described in Table 14 below.

Table 14. Interpretation of G12/G13 mutational status

| Reaction Mix | Result Based on Tables 11-13 | Mutational Status |
|---|---|---|
| KRAS c12 Reaction Mix KRAS c13 Reaction Mix | Positive Negative | G12 Mutation |
| KRAS c12 Reaction Mix KRAS cl3 Reaction Mix | Positive Positive | G13 Mutation |
| KRAS c12 Reaction Mix KRAS c13 Reaction Mix | Negative Positive | G13 Mutation |

**Assay Performance Characteristics**

The performance characteristics of the assay were established on the Roche LightCycler 96, Roche LightCycler 480, Bio-Rad CFX 384 and ABI QuantStudio 5 real-time PCR instruments. The studies were performed using genetically defined reference standards ( genomic DNA and FFPE) from cell lines with defined mutations obtained from Horizon Discovery (Cambridge, England) and cfDNA reference standards from SeraCare (Massachusetts, US). These samples have been characterized genetically as containing heterozygous or homozygous mutations in the coding sequence of the respective target regions. These single nucleotide polymorphisms in the target regions have been confirmed by genomic DNA sequencing and/or ddPCR. Additional samples consisted of cancer patient tissue, plasma samples and normal healthy donor DNA from tissue and plasma.

**Reproducibility**

Reproducibility of the assay was determined with defined analytical levels of genomic DNA with known mutational status and allelic frequencies.

- To establish lot-to-lot variation, a reproducibility study was performed using three different lots of kit. Each lot was tested on one wild-type control and two reference samples containing each mutation at 5% and 1% allelic frequency in nine replicates on Roche LC480 and Bio-Rad CFX 384 instruments.
- Inter-assay %CV was established for same lot of reagents tested on the same instrument by the same user.
- Intra-assay %CV was established through performance of kit on reference samples run in replicates of nine.
- Operator variability was evaluated with one lot of reagents by two operators.

Reproducibility is demonstrated based on %CV of Cq values and rate of % correct mutation calls for all assays on two lots and operators for Roche and Bio-Rad instruments.

Table 15. Summary of reproducibility results

| Variation | %CV |
|---|---|
| Intra-assay | $\leq 3\%$ |
| Inter-assay | $\leq 4\%$ |
| Lot-to-Lot | $\leq 4\%$ |
| Operator | $\leq 3\%$ |

Table 16. Intra-assay reproducibility results on Roche LC480.

| Target | 1% mutant | | | 0.5% mutant | | |
|---|---|---|---|---|---|---|
| | Average Cq | SD | %CV | Average Cq | SD | %CV |
| APC 1309 | 32.11 | 0.71 | 2.20% | 32.30 | 0.51 | 1.58% |
| APC 1367 | 32.72 | 0.42 | 1.27% | 33.87 | 0.32 | 0.96% |
| APC 1450 | 33.29 | 0.31 | 0.92% | 34.44 | 0.36 | 1.06% |
| CTNNB1 41 | 29.35 | 0.27 | 0.91% | 30.45 | 0.26 | 0.84% |
| CTNNB1 45 | 31.74 | 0.35 | 1.10% | 32.46 | 0.41 | 1.26% |
| KRAS 12 | 34.60 | 0.60 | 1.73% | 35.99 | 0.55 | 1.54% |
| KRAS 13 | 33.71 | 0.73 | 2.18% | 34.73 | 0.39 | 1.12% |
| BRAF 600 | 32.37 | 0.31 | 0.95% | 33.37 | 0.24 | 0.71% |
| Internal Control | 28.43 | 0.21 | 0.74% | 28.42 | 0.19 | 0.66% |

The intra-assay data demonstrated good reproducibility with low %CV (Table 16).

**Analytic Sensitivity and Limit of Detection (LOD)**

To determine the limit of detection (LOD) and analytical sensitivity of the kit, the studies were performed using serial dilutions of mutant DNA (reference FFPE and cfDNA) in wild-type background. The wild-type DNA used for dilution was obtained from mutant-free FFPE and normal human plasma respectively. Mutant allelic frequencies tested were 1%, 0.5% and 0.1% at 2.5, 5 and 10ng/reaction DNA input levels. The mutant copy numbers present in genomic DNA with 1%, 0.5% and 0.1% allelic frequency at different DNA input levels are shown in Table 17a.

Table 17a. Mutant DNA copy numbers at different allelic frequencies

| Allelic frequency | Mutant DNA copy numbers at different DNA inputs | | |
|---|---|---|---|
| | 10ng DNA | 5ng DNA | 2.5ng DNA |
| 1% | 28 copies | 14 copies | 7 copies |
| 0.50% | 14 copies | 7 copies | 3.5 copies |
| 0.10% | 2.8 copies | 1.4 copies | 0.7 copies |

Table 17b. LOD summary determined using genomic DNA reference standards

| Target mutation | DNA Input, ng/well | | | |
|---|---|---|---|---|
| | | 10 | 5 | 2.5 |
| APC 1309 | | % Correct Call | % Correct Call | % Correct Call |
| | 1% mutation | 100% | 100% | 100% |
| | 0.5% mutation | 100% | 100% | 90% |
| | 0.10% mutation | 20% | 0% | 0% |
| APC 1367 | 1% mutation | 100% | 100% | 90% |
| | 0.5% mutation | 100% | 100% | 20% |
| | 0.10% mutation | 50% | 10% | 5% |
| APC 1450 | 1% mutation | 100% | 100% | 100% |
| | 0.5% mutation | 100% | 100% | 95% |
| | 0.10% mutation | 90% | 60% | 35% |
| CTNNB1 41 | 1% mutation | 100% | 100% | 100% |
| | 0.5% mutation | 100% | 100% | 100% |
| | 0.10% mutation | 100% | 100% | 90% |
| CTNNB1 45 | 1% mutation | 100% | 100% | 100% |
| | 0.5% mutation | 100% | 100% | 95% |
| | 0.10% mutation | 95% | 40% | 40% |
| KRAS 12 | 1% mutation | 100% | 100% | 100% |
| | 0.5% mutation | 100% | 100% | 67% |
| | 0.10% mutation | 45% | 32% | 35% |
| KRAS 13 | 1% mutation | 100% | 100% | 60% |
| | 0.5% mutation | 95% | 80% | 50% |
| | 0.10% mutation | 56% | 56% | 30% |
| BRAF V600 | 1% mutation | 100% | 100% | 90% |
| | 0.5% mutation | 100% | 75% | 70% |
| | 0.10% mutation | 65% | 15% | 20% |

Table 17c. LOD summary determined using cfDNA reference standards

| Target mutation | DNA Input, ng/well | | |
|---|---|---|---|
| | | 5 | 2.5 |
| | | % Correct Call | % Correct Call |
| APC 1309 | 1% mutation | 100% | 83% |
| | 0.5% mutation | 100% | 83% |
| | 0.10% mutation | 67% | 42% |
| APC 1367 | 1% mutation | 100% | 82% |
| | 0.5% mutation | 92% | 75% |
| | 0.10% mutation | 50% | 50% |

(continued)

| Target mutation | | DNA Input, ng/well | |
|---|---|---|---|
| | | 5 | 2.5 |
| | | % Correct Call | % Correct Call |
| APC 1450 | 1% mutation | 100% | 100% |
| | 0.5% mutation | 100% | 100% |
| | 0.10% mutation | 83% | 58% |
| CTNNB1 41 | 1% mutation | 100% | 100% |
| | 0.5% mutation | 100% | 60% |
| | 0.10% mutation | 20% | 0% |
| CTNNB1 45 | 1 % mutation | 100% | 90% |
| | 0.5% mutation | 83% | 60% |
| | 0.10% mutation | 67% | 40% |
| KRAS 12 | 1% mutation | 100% | 79% |
| | 0.5% mutation | 79% | 29% |
| | 0.10% mutation | 46% | 21% |
| KRAS 13 | 1% mutation | 100% | 70% |
| | 0.5% mutation | 83% | 67% |
| | 0.10% mutation | 25% | 33% |
| BRAF V600 | 1% mutation | 100% | 100% |
| | 0.5% mutation | 85% | 79% |
| | 0.10% mutation | 58% | 36% |

**Conclusion:**

- All targets can be detected at 1% allelic frequency at 5 ng DNA input per PCR reaction;
- For FFPE DNA samples, 0.5% mutation frequency in APC 1309, APC 1307, APC 1450, CTNNB1 (c41,c45) and KRAS(c12) can be detected at 5 ng DNA input;
- For plasma cfDNA samples, 0.5% mutation frequency in APC 1309, APC 1450 and CTNNB 1 41 can be detected at 5 ng DNA input;
- The recommended DNA input is a minimum of 5ng/well.

Recommended input of FFPE should not be higher than 20 ng/well due to possible PCR inhibition. Optimal FFPE sample input is between 25 and 31 Cq of the Internal Control.

**Analytic Specificity**

Analytical specificity of the kit was determined as the correct calling of the samples with no mutation at different concentrations of WT template. There were no false positive calls for up to 320 ng of gDNA per well and up to 20 ng FFPE DNA.

Cross-reactivity of the assays within the kit was tested with one or more mutations present in a mixed positive control at 50% allelic frequency.

Table 18. Analytic specificity: cross-reactivity

| Assay | Expected mutations in tested 50% templates | | | | | | | |
|---|---|---|---|---|---|---|---|---|
| | APC 1309 | APC 1367 | APC 1450; KRAS 12 | CTNNB 141 | CTNNB1 45; KRAS 13 | KRAS 12 | KRA S13 | BRAF 600; CTNNB1 45; KRAS 13 |
| APC 1309 | + | - | - | - | - | - | - | - |
| APC 1367 | - | + | - | - | - | - | - | - |
| APC 1450 | - | - | + | - | - | - | - | - |
| CTNNB1 41 | - | - | - | + | - | - | - | - |
| CTNNB1 45 | - | - | - | - | + | - | - | + |
| KRAS 12 | - | - | + | - | * | + | * | * |
| KRAS 13 | - | - | - | - | + | - | + | + |
| BRAF V600 | - | - | - | - | - | - | - | + |

The data demonstrates that the present invention Kit can correctly identify several mutations within one template. There is cross reactivity between KRAS12 and KRAS13, due to the proximity of the mutations, which can be differentiated (Refer to Table 13).

**Clinical performance of the assay**

Clinical sensitivity and specificity was tested on the samples extracted from FFPE and plasma of patients with different stages of CRC from normal to advanced adenomas (AA), to colorectal cancer stages 1 through 4.
A sample was considered positive if at least one of the target mutations tested positive based on the cutoffs presented in Tables 10-12.

Table 19. Clinical sample testing

| Types of Clinical Samples | | Clinical pa rameter | |
|---|---|---|---|
| | | specificity | sensitivity |
| Clinical stage | CRC, including plasma and FFPE | N/A | 100% |
| | CRC, tissue | N/A | 90% |
| | Advanced Adenomas | N/A | 60% |
| | Non-malignant | 95% | N/A |
| | Non-malignant FFPE only | 90% | N/A |
| Sample Type | FFPE | 95% | 91% |
| | Plasma | 100% | 100% |
| | Excluding adenomas | 95% | 100% |
| | CRC FFPE | N/A | 100% |

**1 Products used in this study**

- qPCR Colorectal cancer detection assay and Kit of the invention

EP 3 494 236 B1

- QClamp® BRAF Genotyping Mutation Test (60 Samples), Cat.# DC-10-0066

**2 Instrumentation used in this study**

- Roche LC480 qPCR instrument

**3 SOPs used in this study**

- CL-OPS.0005 Sample accessioning and management

- CL-OPS 0006 Clinical Lab Patient Test Management

- CL-TF.0007 DiaCarta Clinical Lab Specimen Accessioning and Tracking Log

- CL-OPS.0013- NA Measurements using the Qubit HS dsDNA Assay for QClamp assays

**4 Reported results**

**Summary of the qPCR test results is presented in Table 20 below**:

| Sample ID | Cq of Internal Control | delta Ct/call | | | | | | | overall invention call | Sanger results |
|---|---|---|---|---|---|---|---|---|---|---|
| | | APC1309/1367 | APC1450 | BCT41 | BCT45 | KRAS12 | KRAS13 | BRAF600 | | |
| S1 | 29.71 | 6.71 | | | | | | | Positive APC | APC1367 |
| S2 | 29.58 | | | 8.56 | | 2.53 | | | Positive, BCT, KRAS | KRAS 12 GGT>GAT |
| S3 | 29.37 | | | | | | | 8.9 | WP, BRAF | BRAF QNS |
| S4 | 30.04 | | | 7.22 | | 2.82 | | | Positive, BCT, KRAS | KRAS12 GGT>GAT |
| S5 | 29.0 | 6.76 | | | | | | | Positive APC | APC1367, KRAS 13 GGC>AGC? |
| S6 | 28.29 | 7.56 | | | | | | 8.51 | Positive APC, wp BRAF | APC 1367, BRAF neg |
| S7 | 29.09 | 2.5 | | | | | | | Positive, APC | APC1367 |
| S8 | 29.08 | | | 8.3 | 9.52 | | | | Positive, APC, BCT | BTC c46 CTG>TTG |
| S9 | 27.81 | | | | | | | | Negative | |
| S10 | 2838 | 7.83 | | | | | | | Positive, APC | No Sanger data |
| S11 | 29.89 | | | 7.56 | | 7.19 | 2.16 | 2.2 | Positive, KRAS, BRAF, BCT | KRAS13 GGC>GAC, BTC c45 TCT>TTT, BRAF K601K: AAA>AAG |
| S12 | 28.9 | | | | | | | | Negative | |
| S13 | 29.8 | | 3.17 | | | 1.48 | 8.57 | 8.57 | Positive, APC, KRAS, wp BRAF | APC 1367, 1450 GCG>GTG, KRAS 12 GGT>GAT, BRAF K601K:AAA>AAG |
| S14 | 27.62 | 6.61 | | | | | 9.35 | | Positive, APC, KRAS | KRAS 13 GGC>AGC, APC 1367 |
| S15 | 29.1 | | | 7.67 | | 2.56 | 4.71 | | Positive, BCT, KRAS | KRAS12 GGT>GTT |

(continued)

| Sample ID | Cq of Internal Control | delta Ct/call | | | | | | | overall invention call | Sanger results |
|---|---|---|---|---|---|---|---|---|---|---|
| | | APC1309/1367 | APC1450 | BCT41 | BCT45 | KRAS12 | KRAS13 | BRAF600 | | |
| S16 | 29.47 | 6.41 | | | 8.2 | | | | Positive, APC, wp BCT | APC1367 |
| S17 | 29.9 | | | | 9.88 | | | 8.99 | wp BCT, BRAF | BRAF WT, BCT ONS |
| S18 | 28.64 | | | | | | | | Negative | |
| S19 | 30.05 | | | | | | 10.23 | | wp KRAS | poor sequence |
| S20 | 29.14 | 5.95 | | | | | 9.61 | | Positive APC, WP KRAS | APC1367, KRAS poor sequence |
| S21 | 30.04 | | | | | | | | Negative | |
| S22 | 32.41 | 6.05/7.0 | | | | | | 8.43 | Positive, APC, WP BFAF | APC1367, BRAF QNS |
| S23 | 29.15 | | 1.34 | | | 2.41 | | 8.92 | Positive, APC, KRAS | KRAS12 GGT>GTT, BRAF V600V GTG>GTA |
| S24 | 30.33 | | | | | | | | Negative | |
| S25 | 29.43 | | | | | | | 8.01 | wp BRAF | BRAF K601K:AAA>AAG |
| S26 | 30.03 | | | | | | | | Negative | |
| S27 | 29.74 | | | | | | | | Negative | |
| S28 | 29.82 | | | | | 6.01 | 1.98 | | Positive, KRAS, BRAF | KRAS13 GGC>GAC |
| S29 | 29.86 | 7.76 | | | | | | | Positive, APC | APC1367 |
| S30 | 29.04 | | | | | | | 8.98 | Wp BRAF | BRAF S602S TCT>TC |
| S31 | 29.51 | | 7.32 | | | | | 8.9 | Positive, APC, WP BRAF | APC 1450 GCG>GTG, BRAF K601K:AAA>AAG |
| S32 | 29.69 | | | | | | | | Negative | |
| S33 | 28.56 | | | | | | | | Negative | |
| S34 | 30.13 | | | | | | | | Negative | |

| Sample ID | Cq of Internal Control | delta Ct/call | | | | | | | overall invention call | Sanger results |
|---|---|---|---|---|---|---|---|---|---|---|
| | | APC1309/ 1367 | APC1450 | BCT41 | BCT45 | KRAS12 | KRAS13 | BRAF600 | | |
| S35 | 29.78 | | | | | | | | Negative | |
| S36 | 31.41 | 15.79 | 9.74 | | | | | 8.89 | Wp APC, BRAF | no Sanger data |
| S37 | 27.14 | | | | | | | | Negative | |
| S38 | 29.225 | | | | | | | | Negative | |
| S39 | 30.54 | 7.4/ 7.24 | | | | | | | Positive, APC | APC 1367 |
| S40 | 2996 | | | 7.48 | | | | | wp, BCT | poor sequence, WT APC1450? |
| S41 | 30.55 | | | | | 3.21 | | | Positive, KRAS | poor sequence |
| S42 | 29.49 | | | | | | | 1.31 | Positive, BRAF | BRAF V600E GTG>GAG |
| S43 | 29.35 | | | | | | | 8.21 | wp BRAF | no Sanger data |
| S44 | 30.97 | 6.3/6.95 | | | | | | | Positive, APC | APC1367 |
| S45 | 296 | | | | | | | | Negative | |
| S46 | 29.93 | | | | 7.54 | 6.92 | 3.12 | | Positive, BCT, KRAS | KRAS13 GGC>GAC, BCT c44 CCT>CTT |
| S47 | 29.18 | | | 8.6 | | 1.76 | | | Positive, KRAS, wp APC | KRAS12 GGT>GAT |
| S48 | 27.91 | | | | | | | | Negative | |
| S49 | 28.93 | | | 7.68 | | | 10.87 | | WpBCT, KRAS | no Sanger data |
| S50 | 29.58 | | | | | 3.99 | 4.03 | | Positive, KRAS | KRAS 13 GGC>TGC |
| S51 | 29.24 | | | 8.085 | | | | | WpBCT | WT BCT |
| S52 | 32.05 | 4.3/7.325 | | | | 11/10.24 | | | Positive, APC, wp KRAS | APC 1367,BRAF: K601E: AAA>CAA |
| S53 | 29.09 | | | 8.4 | | | | | Positive, BCT | BCT c42 ACA>ATA |
| S54 | 29.73 | | | | 10.01 | | | | Positive, BCT | no Sanger data |
| SSS | 30.55 | | | | | | | | Negative | |

(continued)

| Sample ID | Cq of Internal Control | delta Ct/call | | | | | | | overall invention call | Sanger results |
|---|---|---|---|---|---|---|---|---|---|---|
| | | APC1309/1367 | APC1450 | BCT41 | BCT45 | KRAS12 | KRAS13 | BRAF600 | | |
| Total positive | | 13 | 3 | 7 | 3 | 11 | 6 | 2 | **29** | 26 samples confirmed positive, no seq data for 2 samples |
| Total weak positive | | 1 | 1 | 3 | 2 | 1 | 4 | 13 | **10** | 2 |
| Total Neg. | | 41 | 51 | 45 | 50 | 43 | 45 | 40 | 16 | 13 ( 1 BRAF, 1 BCT and 1 APC weak positive tested Negative) |

In the column listing the overall assay calls (second from the right) samples highlighted in green were positive by Light green- weak positive. Negative calls - orange. Since the samples were blinded and most of them were expected to be extracted from plasma, we have used positive, negative and weak positive calls due to un-validated cutoffs for the plasma sample type. The column also shows all the genes that were found positive/weak positive for target mutations. Overall there were 29 positive calls, 16 negative calls and 10 weak positive calls.

Samples s22, s24, s34, s36, s39, s44, s52 and s55 had insufficient DNA as evidenced by the Cq of the IC over 30. These samples were processed by present invention-assay, but results of these tests should be interpreted with caution, especially the negative calls on Samples s24, s34 and s55.

qPCR results of all positive and weak positive samples were further confirmed by Sanger sequencing of the qPCR amplicons. Some BRAF samples were also tested by alternative BRAF qPCR DiaCarta kit.

Results are presented in the last column to the right. Out of the 49 samples tested for all the relevant targets, Sanger sequencing produced no satisfactory data for 6 samples. 2 BRAF weak positive samples were found to be negative (either WT by Sanger sequencing or Negative by the alternative qPCR DiaCarta assay for BRAF c600). Several BRAF mutations outside of the V600E were shown to be present in the other weak positive cases that did not change the overall calls for the samples carrying these mutations. 100% of the positive present invention calls were confirmed by Sanger sequencing with available data. 3 calls could not be confirmed due to poor Sanger data.

3 out of 10 weak positive calls were negative by Sanger, 5 did not produce sequencing or qPCR data and 2 BRAF WP calls tested positive for mutations other than V600E.

## 1 Conclusions:

The test results clearly demonstrate that the assay can be used to detect mutations in the CRC DNA samples extracted from patient plasma. As little as 30 ng of DNA is sufficient to provide test results as evidenced by concordance rate of 100% for positive calls with available Sanger data. Most of the samples with low quantity/quality of DNA are difficult to test, but these can be identified by using the internal control data.

|  | N | Specificity | Sensitivity |
|---|---|---|---|
| Cancer (Stages I-IV) | 35 | N/A | 100% |
| Non-Malignant | 22 | 95% | N/A |
| Overall (Exclude Pre-Cancer) | 57 | 95% | 100% |
| Overall (Include Pre-Cancer) | 67 | 95% | 91% |

The table above shows the assay performance from FFPE samples. Pre-cancer detection sensitivity is 60% (6 out of 10 samples).

The Table below compares the technical details and performance characteristics of assay of the invention with prior art assays.

|  | **Assay of the invention** | **Prior art assays** |
|---|---|---|
| Sample | Human Tissue | Human Stool |
| Molecular Targets | 20 DNA Mutation Markers (APC Exon 15, CTNNB 1 Exon 3, KRAS Exon 2, BRAF Exon 15) 1 DNA Normalization Marker (Beta Actin) | 2 DNA Methylation Markers (NDRG4, BMP3), DNA Mutation Markers (KRAS Exon 2), 1 DNA Normalization Marker (Beta Actin), 1 Fecal Hemoglobin Marker (FIT) |
| Turnaround Time | 1 Dav | 2 Weeks |
| Sensitivity (Stage 0) | 62.3% | 42.4% |
| Sensitivity (Stages I-IV) | 95.5% | 92.3% |
| Specificity | 100% | 100% |

(continued)

|  | **Assay of the invention** | **Prior art assays** |
|---|---|---|
| Sample | Human Tissue | Human Stool |
| Equipment | Real-Time PCR Machine | Real-Time PCR Machine, ELISA Reader, Liquid Handling Workstation |

## EXAMPLE III

This example describes the feasibility studies of the Assay for qualitative detection of mutations in targeted genes of APC (codons 1309, 1367, 1450), KRAS (codons 12 and 13), BRAF (codon 600) and CTNNB1 or rather BCT (codons 41 and 45) genes associated with colorectal cancer initiating events.

The Assay is a real-time qPCR-based in vitro diagnostic test intended for use in the detection of mutations in the APC (codons 1309, 1367, 1450), KRAS (codons 12 and 13), BRAF (codon 600) and CTNNB1 or rather BCT (codons 41 and 45) genes in DNA extracted from FFPE sections and human stool samples.

Since clinical samples from cancer patients frequently contain trace amounts of mutant allele in a large excess of wild-type DNA, DiaCarta's proprietary QClamp® XNA-PCR technology is employed in the present invention Taqman assays to suppress amplification of WT alleles to improve the sensitivity of mutation detection.

**Target Gene and Mutation selection**

A panel of target genes were selected based on their mutation frequency in early-stage colorectal cancer patients (UP patent 0,172,823 A1 licensed from Potsdam University), preliminary clinical trials by Dr. Sholttka (publications). These early colorectal cancer related biomarkers include APC (codons 1309, 1367, 1450), KRAS (codons 12 and 13), BRAF (codon 600), CTNNB1 or rather BCT (codons 41 and 45) genes and TGFβ (to be included). A housekeeping gene, beta- actin (ACTβ), was selected as internal control based on preliminary data from B. Sholttka and because competitor assay (ColoGuard from Exact Sciences) also uses that same gene for internal control. ACTβ assay is used to monitor sample DNA extraction efficiency and presence of PCR inhibitors as well as to provide a way of quantitation of amplifiable template in each reaction well to prevent false positive/negative results.

3.2. Primer, Probe and XNA Design

3.2.1. Primers and probes were designed using PrimerQuest Tool following the qPCR primer and probe design rules. The primers were designed with a Tm of 62-64°C while the probes were designed with Tm of 66-68°C.

3.2.2. The amplicon sizes were designed under 150 bp if possible.

3.2.3. Primers and probes were checked in-silico for specificity (BLAST), primer dimers/secondary structure (autoDimer) and amplicon secondary structure (M-fold).

3.2.4. The XNAs were designed to be between the forward and reverse primers or overlap a few bases with the forward primer.

3.2.5. The probes were designed to be parallel (on the same strand as) to XNAs and either overlap with the XNA (mutant-specific probes) or be distal to the XNA (locus specific probes).

3.3. Design Selection strategy

3.3.1. Primer, probe and XNA combinations and concentration optimization tests were performed to find the optimal conditions for differentiating mutant and WT alleles for each targeted somatic mutation.

3.3.2. Several qPCR master mixes are tested to find the best one that gives the lowest Ct and highest delta Ct for best performance in differentiating mutant and WT.

3.3.3. For efficient clamping by the XNA, a XNA annealing step at 70°C before the binding of primers and probes is included in the qPCR cycling program. Optimal annealing temperature for primers and probes will be tested by gradient analysis.

4. MATERIALS AND METHODS

4.1. Composition of the PCR reaction Mix

Table 21. PCR reaction mix

| Reagent | Volume in 10 $\mu$l reaction, | Final concentration |
|---|---|---|
| 2xMaster Mix | 5 | 1x |
| 10 um primer F-R mut | 0.4-0.1 | 400-100 nM |
| 10 um primer F-R ACTB | 0.1 | 100 nM |
| 10 um probe ACTB | 0.1 | 100 nM |
| 10 um probe (mut) | 0.1-0.05 | 100-50 nM |
| Template | 2 | 20 |
| XNA (40, 20, 10, 5, 2.5 uM) | 0.5 | 2, 1, 0.5, 0.25, 0.125 um |
| Nuclease free water | 2-2.15 | 20 |
| TOTAL | 10 | 100 |

1.1. Reference templates:

CTNNB1 CD 41: IDT gBlock, custom

CTNNB1 CD 45: ATCC CCL-247_D1

BRAF C600: BRAF C600 Reference standard (Horizon Cat#: HD238)

KRAS c13: KRAS G13D Reference Standard (Horizon Cat#: HD290)

KRAS c12: KRAS G12D Reference Standard (Horizon Cat#: HD272)

APC 1309: ATCC CRL-2158_D1

APC 1367: ATCC CRL-2101_D1

APC 1450: ATCC CCL-235_D1

1.2. Instruments

Roche LC96 DC2034, Cat. No. 05 815 916 001

Roche LC480II DC2035, Cat. No. 05015243001

BioRad CFX384 DC2044, Cat. No. 4329001

1.1. Reagents/Kits

Takara Bio Premix Ex Taq (Probe qPCR) 2X master mix, Clontech/TAKARA, Cat. # RR390A

SensiFAST Probe No-ROX mix (2x), Bioline, Cat. # BIO-86002
STAT-NAT-DNA -Mix (lyophilized), SENTINEL, Cat. # 1N001)
KAPA Probe Fast qPCR Master Mix (2x) Universal (Cat. #: KK4703)
SensiFAST Probe No-ROX mix (2x), Bioline, Cat.# BIO-86002

1.2. Software

PrimerQuestT ool/IDT
Autodimer
M-fold
CLC sequence viewer 7

1.3. Browser based tools

UCSC Genome Browser
NCBI
dbSNP
dbVar
COSMIC

1.1. Referenced DiaCarta Documents

DDC.0007_ present invention qPCR Project Plan
DDC.0006_Product Requirements for present invention multiplex qPCR Test CO.0001 Product development and Commercialization

2. DESIGN ASSESSMENT RESULTS

2.1. Master Mix selection

Based on previous tests on master mixes for Taqman based qPCR assays , KAPA Probe Fast qPCR Master Mix (2x) Universal was selected as the primary master mix for Taqman probe based qPCR reactions for mutation detection assay development. The following additional master mixes were compared with the KAPA master mix:

1) Takara Bio Premix Ex Taq (Probe qPCR) 2X master mix, Clontech/TAKARA, Cat.# RR390A
2) SensiFAST Probe No-ROX mix (2x), Bioline, Cat.# BIO-86002
3) STAT-NAT-DNA -Mix (lyophilized), SENTINEL, Cat. # 1N001)

**Table** 22. Comparison of master mixes for detection of CTNNB1 codon 45 mutation by QClamp Taqman real-time PCR

| Master Mix | No XNA Average Ct (mean ± SD) | | XNA (5um) Average Ct (mean ± SD) | | |
|---|---|---|---|---|---|
| | 5% BCT CD45 | WT | 5% BCT CD45 | WT | ΔCt |
| KAPA | 24.37 ± 0.1 | 24.45 ± 0.05 | 28.83 ± 0.16 | 40 | 11.17 |
| STAT-NAT | 24.93 ± 0.23 | 25.09 ± 0.28 | 30.81 ± 0.23 | 40 | 9.19 |
| CloneTech | 25.51 ±0.05 | 25.88 ± 0.10 | 29.99 ± 0.05 | 40 | 10.01 |
| Bioline | 24.88 | 24.81 | 28.7 ± 0.14 | 38.06 ± 3.36 | 9.36 |

3.1.1. Lyophilized master mix can be conveniently stored and shipped at room temperature, so lyophilized Bioline master mix was also evaluated and compared with KAPA probe Fast qPCR
Master Mix (2x) Universal (Table 23)

**Table 23.** Present invention mutation detection assay using Bioline SensiFAST Probe mix (lyophilized)

| BIOLINE | 1 | 2 | 3 | AVE | SD | CV | 1 | 2 | 3 | AVE | SD | CV | Delta Ct |
|---|---|---|---|---|---|---|---|---|---|---|---|---|---|
| TARGET | | | | | | | | | | | | | |
| APC 1309 | 50 | 50 | 50 | 50 | 0 | 0.0% | 30.2 | 30.36 | 30.07 | 30.21 | 0.15 | 0.5% | 19.79 |
| APC 1367 | 50 | 50 | 50 | 50 | 0 | 0.0% | 32.6 | 32.5 | | 32.55 | 0.07 | 0.2% | 17.45 |
| APC 1450 | 50 | 38.56 | 38.5 | 42.36 | 6.619 | 156% | 33.53 | 33.52 | 33.28 | 33.44 | 0.14 | 0.4% | 8.913 |
| CTNNB1 CD41 | 38.24 | 35.47 | 38.3 | 37.34 | 1.62 | 4.3% | 32.01 | 31.88 | 31.76 | 31.88 | 0.13 | 0.4% | 5.457 |
| CTNNB1 CD45 | 50 | 38.25 | 36.9 | 41.7 | 7.222 | 17.3% | 31.18 | 31.43 | 31.12 | 31.24 | 0.16 | 0.5% | 10.46 |
| KRAS CD12 | 41.56 | 41.87 | 45 | 42.81 | 1.903 | 4.4% | 32.97 | 34.89 | 34.31 | 34.06 | 0.98 | 2.9% | 8.753 |
| KRAS CD13 | 50 | 42.6 | 50 | 47.53 | 4.272 | 9.0% | 32.55 | 34.98 | 34.15 | 33.89 | 1.24 | 3.6% | 13.64 |
| BRAF | 36.98 | 40.83 | 43 | 40.28 | 3.067 | 7.6% | 33.52 | 34.19 | 33.78 | 33.83 | 0.34 | 1.0% | 6.453 |
| KAPA FAST PROBE | 1 | 2 | 3 | AVE | SD | CV | 1 | 2 | 3 | AVE | SD | CV | Delta_Ct |
| TARGET | | | | | | | | | | | | | |
| APC 1309 | 50 | 50 | 50 | 50 | 0 | 0.0% | 31.93 | 31.61 | 32.02 | 31.85 | 0.22 | 0.7% | 18.15 |
| APC 1367 | 50 | 50 | 50 | 50 | 0 | 0.0% | 50 | 50 | 31.92 | 43.97 | 10.4 | 23.7% | 6.027 |
| APC 1450 | 41.52 | 31.38 | 29.5 | 34.15 | 6.451 | 18.9% | 34.11 | 30.4 | 50 | 38.17 | 10.4 | 27.3% | -4.023 |
| CTNNB1 CD41 | 50 | 50 | 35.6 | 45.19 | 8.337 | 18.4% | 32.48 | 31.07 | 30.93 | 31.49 | 0.86 | 2.7% | 13.69 |
| CINNB1 CD45 | 50 | 50 | 50 | 50 | 0 | 0.0% | 30.08 | 32.59 | 33.01 | 31.89 | 1.58 | 50% | 18.11 |
| KRAS CD12 | 50 | 50 | 50 | 50 | 0 | 0.0% | 50 | 50 | 50 | 50 | 0 | 0.0% | 0 |
| KRAS CD13 | 50 | 50 | 50 | 50 | 0 | 0.0% | 50 | 36.76 | 50 | 45.59 | 7.64 | 16.8% | 4.413 |
| BRAF | 36.98 | 40.83 | 43 | | | | 33.52 | 34.19 | 33.78 | 33.83 | 0.34 | 1.0% | -33.83 |

Bioline master mix and KAPA Probe Fast qPCR master Mix (2x) were further compared using samples with different mutation frequency and on different qPCR machines (Roche LC 480 vs BioRad CFX 384). The control is in HEX channel while all the targeted mutations are in Fam. Delta Ct was calculated for each sample as follows: Ct difference ($\triangle$Ct) = Mutation Assay Ct

- Control Assay Ct. The data were summarized in Table 24.

3.1.1.

Table 24. Comparison of KAPA probe fast qPCR master master mix and Bioline master mix on present invention targets with different mutation frequency and on different qPCR machines (Roche LC 480 vs BioRad CFX 384).

| | ROCH | | | I | | | BIORAD | | | | | |
| | KAPA | | | BIOLINE | | | KAPA | | | BIOLINE | | |
| | Delta_Cq | | | Delta_Cq | | | Delta_Cq | | | Delta_Cq | | |
| TARGET | WT | 0.50% | 0.10% | WT | 0.50% | 0.10% | WT | 0.50% | 0.10% | WT | 0.50% | 0.10% |
|---|---|---|---|---|---|---|---|---|---|---|---|---|
| APC 1309 | 21.7 | 3.47 | 21.49 | 22.753 | 23.11 | 23.83 | 16.94 | 17.07 | 18.92 | 23.3 | 17.84 | 23.46 |
| APC 1367 | 8.955 | 6.47 | 8.8 | 22.967 | 22.19 | 22.58 | 22.3 | 7.729 | 13 | 23.67 | 24.16 | 8.129 |
| APC 1450 | 12.81 | 7.09 | 9.238 | 9.12 | 7.663 | 8.625 | 12.99 | 4.49 | 6.2 | 12.62 | 8.822 | 9.679 |
| CTNNB1 | 11.51 | 2.4667 | 5.767 | 7.1833 | 3.903 | 5.677 | 8.273 | 2.58 | 5.125 | 7.759 | 4.488 | 6.096 |
| CTNNB1 | 11.2 | 5.3667 | 8.86 | 5.1767 | 4.377 | 4.067 | 14.29 | 5.988 | 8.237 | 7.687 | 7.341 | 7.471 |
| KRAS CD12 | 10.65 | 7.2867 | 10.86 | 5.69 | 4.97 | 5.663 | 11.88 | 7.973 | 10.03 | 4.242 | 4.459 | 4.466 |
| KRAS CD13 | 11.51 | 8.97 | 8.497 | 2.0733 | 1.25 | 2.075 | 13.24 | 7.155 | 9.599 | -0.21 | 0.189 | 2.345 |
| BRAF | 9.33 | 7.7267 | 8.843 | 5.4783 | 5.928 | 6.237 | 9.58 | 6.107 | 9.098 | 4.428 | 5.404 | 4.839 |

5.1. Master Mix selection Conclusion

Bioline master mix and KAPA Probe Fast qPCR Master Mix (2x) Universal are comparable when the mutation frequency is 5% or higher while when mutation frequency is lower (0.5% or 0.1% or lower), KAPA Probe Fast qPCR Master Mix (2x) Universal performed better in regarding to differentiating mutant and WT alleles. Therefore, KAPA Probe Fast qPCR Master Mix (2x) Universal will be used in the present invention assay.

5.2. Optimization of the assay reagent composition and thermocycling conditions.

5.2.1. Primers for BRAF c600 and KRAS c12, c13 were designed and optimized previously in existing QClamp SYBR commercial products (DC-10-1066, DC-10- 0036, DC-10-0039, DC-10-1039, DC-10-0197, DC-10-0169).

5.2.2. The APC, CTNNB1, beta-ACT primers were designed to have annealing temperatures same as BRAF and KRAS primer pairs (64C for Roche and BioRad instruments).

5.2.3. Annealing temperature gradients (60-70C) were performed using the Roche LC96 with KAPA Probe Fast qPCR Master Mix (2x) Universal to find the optimal annealing temperature of each target primers and probes. The results of the gradient analysis were summarized in Table 25 and Table 26.

Table 25. Gradient analysis of annealing temperature for assay primers and probes (No XNA)

| | Average Ct (mean $\pm$ SD) | | |
|---|---|---|---|
| Temperature (° C) | 5% CTNNB1 CD41 | 5% KRAS13 | 5% BRAFV600E |
| 60 | 25.2 $\pm$ 0.28 | 25.85 $\pm$ 0.03 | 24.64 $\pm$ 0.04 |
| 60.5 | 25.21 $\pm$ 0 | 25.92 $\pm$ 0.14 | 24.69 $\pm$ 0.11 |
| 61.5 | 25.18 $\pm$ 0.09 | 26.03 $\pm$ 0.08 | 24.98 $\pm$ 0.23 |
| 62.6 | 25.42 $\pm$ 0.07 | 26.08 $\pm$ 0.11 | 24.88 $\pm$ 0.14 |
| 64 | 25.93 $\pm$ 0.25 | 26.2 $\pm$ 0.02 | 25.23 $\pm$ 0.04 |
| 65.3 | 26.81 $\pm$ 0.03 | 26.3 $\pm$ 0.04 | 26 $\pm$ 0.03 |
| 66.7 | 30.86 $\pm$ 0.55 | 28.06 $\pm$ 0.17 | 28.36 $\pm$ 0.30 |
| 67.9 | NA | NA | 36.01 $\pm$ 5.64 |
| 68.9 | NA | NA | NA |
| 69.6 | NA | NA | NA |
| 70.0 | NA | NA | NA |

Table 26. Gradient analysis of annealing temperatures for assay primers and probes (With XNA)

| | Average Ct (mean $\pm$ SD) | | | Average Ct (mean $\pm$ SD) | | | Average Ct (mean $\pm$ SD) | | |
|---|---|---|---|---|---|---|---|---|---|
| Temperature (°C) | 5% | WT | $\Delta$ Ct | 5% | WT | $\Delta$ Ct | 5% | WT | $\Delta$ Ct |
| 60 | 31.39 | 40 | 8.61 | 31.69 | 34 | 2.31 | 33.16 | 35.07 | 1.91 |
| 60.5 | 31.26 | 40 | 8.74 | 31.71 | 34.53 | 2.82 | 33.66 | 36.18 | 2.52 |
| 61.5 | 31.1 | 40 | 8.9 | 31.75 | 35.21 | 3.46 | 33.41 | 36.37 | 2.96 |
| 62.6 | 31.7 | 40 | 8.3 | 32.48 | 40 | 7.52 | 32.69 | 40 | 7.31 |
| 64 | 32.5 | 40 | 7.5 | 32.58 | 40 | 7.42 | 34.27 | 36.52 | 5.73 |
| 65.3 | 34.54 | 40 | 4.4 | na | na | na | 34.21 | 40 | 5.79 |
| 66.7 | na | na | na | na | na | na | 36.36 | 40 | 3.64 |
| 67.9 | NA | NA | NA | NA | NA | NA | NA | NA | NA |
| 68.9 | NA | NA | NA | NA | NA | NA | NA | NA | NA |
| 69.6 | NA | NA | NA | NA | NA | NA | NA | NA | NA |

(continued)

| Temperature (°C) | Average Ct (mean ± SD) | | | Average Ct (mean ± SD) | | | Average Ct (mean ± SD) | | |
|---|---|---|---|---|---|---|---|---|---|
| | 5% | WT | Δ Ct | 5% | WT | Δ Ct | 5% | WT | Δ Ct |
| 70 | NA | NA | NA | NA | NA | NA | NA | NA | NA |

5.2.1. PCR annealing temperature conclusion: 63-64°C annealing temperatures were demonstrated to be optimal for differentiation of mutant and WT alleles for all the invention assay targets.

5.2.2. Optimization of PCR cycling conditions

XNAs are employed in the invention Taqman mutation detection assays to suppress wt amplification in order to improve mutation detection sensitivity. For efficient clamping by the XNA, a XNA annealing step at 70°C before the binding of primers and probes is included in the qPCR cycling program. Based on gradient analysis of the primer and probe annealing temperature, the thermocycling conditions for the invention Taqman mutation detection assays is optimized as follows:

5.2.3. 95°C for 5 min followed by 50 cycles of 95°C 20 seconds, 70°C 40 seconds, 64°C 30 seconds and 72°C 30 seconds (data acquisition).

5.3. Optimization of Primer and Probe Concentrations

5.3.1. Primer and probe matrix dilution experiments were conducted to find the optimal concentrations for differentiating mutant and WT alleles of targeted genes.

Table 27. Primer concentrations and Ratio of Primer to probe tested:

| Primer | Probe | primer: probe Ratio | Final Primer Concentration in Reaction | Final probe Concentration in Reaction |
|---|---|---|---|---|
| 8 um | 4 um | 2:1 | 0.8 uM | 0.4 uM |
| 4 um | 2 um | 2:1 | 0.4 uM | 0.2 uM |
| 2um | 1 um | 2:1 | 0.2 uM | 0.1 uM |
| 1 um | 0.5 um | 2:1 | 0.1 uM | 0.05 uM |

5.3.1. The use of XNA combined with limited primer/probe concentration resulted in less or no WT background Amplification for selected locus specific probes. The results of optimization of primer, probe and XNA concentrations are summarized in Tables 28 - 36.

Table 28. Primer, probe and XNA titration for CTNNB1 c41 assay on BioRad CFX 384

| | Primer/probe 800 nM/400 Nm | | | Primer/probe 400 nM/200 Nm | | | Primer/probe 200 nM/100 Nm | | | Primer/probe 100 nM/50 Nm | | |
|---|---|---|---|---|---|---|---|---|---|---|---|---|
| | AverageCt(mean±SD | | | Average Ct (mean ± S | | | Average Ct (mean ± SD) | | | Average Ct (mean ± SD) | | |
| XNA final | 5%BCTCD4 | WT | $\Delta$ Ct | 5%BCTCD | WT | $\Delta$Ct | 5%BCTCD | 'WT | $\Delta$Ct | 5%BCT CD41 | WT | $\Delta$ Ct |
| 1 | 29.43 ± .164 | 37.92 ± 1.19 | 8.49 | 29.22±0.1 | 37.8 ± 1.47 | 8.58 | | | | | | |
| 0.5 | 29.35±0.20 | 37.39±1.32 | 8.04 | 29.14 ± 0. | 36.60 ± 1.08 | 7.46 | 29.33±0.1 | 37.73 ± 2.08 | 8.4 | 31.47 ± 0.54 | 40 ± 0 | 8.53 |
| 0.25 | 29.28±0.09 | 35.20±0.32 | 5.92 | 29.07 ± 0. | 35.77 ±0.3 | 6.63 | | | | | | |
| 0.125 | 29.28 ± 0.17 | 35.36±0.26 | 6.08 | | | | | | | | | |
| 0 | 25.54±0.06 | 25.28±0.06 | 0.04 | 29.40 ± 0. | 29.39 ±0.05 | 0.01 | | | | | | |

**Table 29.** XNA titration for CTNNB1 c41 assay with primer/probe at 200 nM/100 nm on LC 96

| XNA final conc. (um) | Average Ct (mean ± SD) | | |
|---|---|---|---|
| | 5% BCT CD41 | WT | ∆ Ct |
| 2 | 31.02 ± 0.04 | 40 ± 0 | 8.98 |
| 1 | 30.33 ± 0.04 | 40 ± 0 | 9.7 |
| 0.5 | 30.13 ± 0.39 | 40 ± 0 | 9.87 |
| 0 | 27.22 ± 0.11 | 27.24 ± 0.19 | 0.02 |

**Table 30.** Primer, probe and XNA titration for CTNNB 1 c45 assay on LC 96

| XNA final conc. | Primer/probe 800 nM/400 Nm Average Ct (mean ± SD) | | | Primer/probe 200 nM/100 Nm Average Ct (mean ± SD) | | | Primer/probe 100 nM/50 Nm Average Ct (mean ± SD) | | |
|---|---|---|---|---|---|---|---|---|---|
| | 5% BCT CD41 | WT | ∆ Ct | 5% BCT CD45 | WT | ∆ Ct | 5%BCT CD41 | WT | ∆ Ct |
| 2 | 27.41 ± 0.15 | 34.96 ± 0.78 | 7.55 | 29.14 ± 0.02 | 38.68 ± 2.29 | 9.54 | 29.7 ± 0.30 | 40 ± 0 | 10.3 |
| 1 | 27.29 ± 0.16 | 34.27 ± 0.21 | 6.98 | 29.87 ± 0.25 | 38.91 ± 1.88 | 9.04 | 29.30 ± 0.14 | 40 ± 0 | 10.7 |
| 0.5 | 27.20 ± 0.31 | 33.23 ± 0.52 | 6.03 | 31.36 ± 0.44 | 40 ± 0 | 8.64 | 29.20 ± 0.29 | 40 ± 0 | 10.8 |
| 0 | 24.46 ± 0.13 | 24.52 ± 0.02 | 0.06 | 26.64 ± 0.39 | 26.40 ± 0.17 | 0.24 | 25.48 ± 0.10 | 25.19 ± 0.38 | 0.29 |

**Table 31.** XNA titration for BRAF c600 assay with primer/probe at 100 nM/50 nM on LC 96

| XNA final conc. (um) | Average Ct (mean ± SD) | | |
|---|---|---|---|
| | 5% BRAF V600 | WT | ∆ Ct |
| 2 | 29.68 ± 0.36 | 40 ± 0 | 10.32 |
| 1 | 30.25 ± 0.7 | 38.78 ± 2.1 | 8.53 |
| 0.5 | 29.78 ± 0.49 | 38.77 ± 2.12 | 8.99 |
| 0 | 24.61 ± 0.11 | 24.73 ± 0.18 | 0.12 |

**Table 32.** Primer, probe and XNA titration for BRAF codon 600 assay on LC 480

| XNA final cone. (um) | 400 Nm primer/200 nM probe (4x) Average Ct (mean ± SD) | | | 100 nM primer/50 nM probe (1x) Average Ct (mean ± SD) | | |
|---|---|---|---|---|---|---|
| | 5% BRAF V600E | WT | ∆ Ct | 5% BRAF V600E | WT | ∆ Ct |
| 2 | 29.07 ± 0.3 | 36.07 ± 0.77 | 7 | 31.14 ± 0.32 | 39.27 ± 1.26 | 8.13 |
| 1 | 28.92 ± 0.42 | 36.6 ± 0.27 | 7.68 | 30.79 ± 0.17 | 38.86 ± 1.96 | 8.07 |
| 0.5 | 28.92 ± 0.18 | 36.04 ± 0.48 | 7.12 | 30.06 ± 0.58 | 39.16 ± 1.44 | 9.1 |

**Table 33.** XNA titration for KRAS codon12 assay with primer/probe 400 nM/200 nM on LC96

| XNA final conc. (um) | Average Ct (mean ± SD) | | |
|---|---|---|---|
| | 5% KRAS c12 | WT | Δ Ct |
| 1 | 31.19 ± 0.06 | 40 ± 0 | 8.81 |
| 0.25 | 31.19 ± 0.12 | 40 ± 0 | 8.81 |
| 0.125 | 31.39 ± 0.34 | 40 ± 0 | 8.61 |
| 0.0625 | 31.25 ± 0.29 | 38.99 ± 1.74 | 7.74 |
| 0 | 26.7 ± 0.01 | 26.79 ± 0.34 | 0.09 |

**Table 34.** Primer and probe titration for KRAS c12 assay on LC480

| Primer/probe conc. | Average Ct (mean ± SD) | | | | |
|---|---|---|---|---|---|
| | 5% KRAS12 | 1% KRAS12 | WT | 5%Δ Ct | 1%Δ Ct |
| 400 Nm primer/200 nM probe | 29.7 ± 0.17 | 32.23 ± 0.48 | 36.92 ± 0.75 | 7.22 | 4.69 |
| 300 Nm primer/250 nM probe | 30.17 ± 0.25 | 32.05 ± 0.91 | 37.2 ± 2.57 | 7.03 | 5.15 |
| 200 Nm primer/100 nM probe | 30.89 ± 0.40 | 33.75 ± 0.68 | 40 ± 0 | 9.11 | 6.25 |

3.1.2.

Table 35. XNA titration for KRAS c13 assay with primer/probe 400 nM/200 nM on LC96

| XNA final conc. ($\mu$M) | Average Ct (mean ± SD) | | |
|---|---|---|---|
| | 5% KRAS c13 | WT | ΔCt |
| 2 | 31.95 ± 0.45 | 40 ± 0 | 8.05 |
| 1 | 31.13 ± 0.13 | 40 ± 0 | 8.87 |
| 0.5 | 31.58 ± 0.11 | 40 ± 0 | 8.42 |
| 0 | 27.36 ± 0.15 | 27.06 ± 0.06 | 0.3 |

Table 36. KRAS c13 XNA titration with primer/probe 200-100 nM/100-50 Nm on LC480

| XNA final conc. (um) | 200 nM primer/ 100 nM probe | | | 100 nM Primer/50 nM probe | | |
|---|---|---|---|---|---|---|
| | Average Ct (mean ± SD) | | | Average Ct (mean ± SD) | | |
| | 5% KRAS13 | WT | Δ Ct | 5% KRAS13 | WT | Δ Ct |
| 2 | 29.99 ± 0.48 | 36.67 ± 0.37 | 6.68 | | | |
| 1 | 29.33 ± 0.44 | 38.88 ± 1.93 | 9.55 | | | |
| 0.5 | 29.59 ± 0.27 | 39.29 ± 1.22 | 9.7 | 32.18 ± 0.8 | 40 ± 0 | 7.82 |
| 0.25 | 29.63 ± 0.23 | 37.74 ± 2.06 | 8.11 | | | |

5.3. Optimized Primer and Probe Concentrations

5.3.1. Optimal concentrations are 0.1 uM primer and 0.05 uM probe for differentiating mutant and WT of CTNNB1 c45, BRAF c600 on Roche LightCycler 96, Roche LightCycler480 and BioRadCFX384.

5.3.2. For CTNNB1 c41, 0.2 um primer and 0.1 um probe are optimal for differentiating mutant and WT alleles on Roche Light cycler 96, Roche Light Cycler 480.

5.3.1. For KRAS12 and 13, 0.4/0.2 um primer and probe are optimal for differentiating mutant and WT on LC 96, 0.2 um primer and 0.1 um probe are optimal for differentiating mutant and WT on LC 480 and BioRad CFX384.

5.3.2. In general, using limited primers and locus specific probes concentration (100 Nm to 200 Nm/50 to 100 nM) result in less or no WT background amplification with XNA. Primer/probe conc. above 0.4/0.2 um usually result in WT background amplification with XNA. The use of limited primer/probe conc. combined with XNA will result in less or no WT background amplification for selected locus specific probes.

5.4. Optimization of XNA concentration with primer-probes

5.4.1. Primers and probes were screened for differentiating mutant and WT alleles in presence of XNA. For optimization, XNA titration and limited primer and probe concentration (100 Nm to 200 nM/50 to 100 nM) were used.

5.4.2. The following primers were screened by SYBR assay to find the primer pairs that result in best ΔCt between mutant and WT alleles (Tables 37-39).

Table 37. Primers screened for APC 1309, APC 1367 and APC 1450

| Target | Forward Primer | Sequence | Forward Primer | Sequence |
|---|---|---|---|---|
| APC 1309 | APC001F SEQ ID NO:6 | GAATCAGCTCCATCCAAGT | APC001R SEQ ID NO:7 | CTGTGACACTGCTGGAACTTCGC |
| APC 1367 | APC002F SEQ ID NO:8 | AGCACCCTAGAACCAAATCCAGCAG | APC002R SEQ ID NO:9 | TGGCATGGTTTGTCCAGGGC |
| APC 1450 | APC003F SEQ ID NO: 10 | ACAAACCATGCCACCAAGCAGA | APC003R SEQ ID NO:11 | GAGCACTCAGGCTGGATGAACAAG |
| APC 1309 | APC S1F2 SEQ ID NO:12 | GGATGTAATCAGACGACACAGGA | APCS1R2 SEQ ID NO:13 | CACAGGATCTTCATCTGACCTAGTT |
| APC 1367 | APC_S2_F2 SEQ ID NO:14 | TCTCCCTCCAAAAGTGGTG | APCS2 R2 SEQ ID NO:15 | AAACTATCAAGTGAACTGACAGAAG |
| APC 1450 | APC_S3_1_F2 SEQ ID NO:16 | CCAGATAGCCCTGGACAAACC | APC_S3_1 R2 SEQ ID NO: 17 | CTTTTCAGCAGTAGGTGCTTTATTTTA |

**Table 38**. Optimization of XNA and primer concentration for APC 1309, 1367 and 1450.

|   |         | Primer            | Primer Conc. | XNA          | XNA conc. | WT1 | WT2 | WT3 | MT1   | MT2   | MT3   | MT3   |
|---|---------|-------------------|--------------|--------------|-----------|-----|-----|-----|-------|-------|-------|-------|
| 1 | APC     | APCS1_F2 + APCS1_R2 | 0.05       | CS01         | 2         | 40  | 40  | 40  | 33.2  | 33.03 | 34.21 | 34.21 |
| 2 | APC1367 | APCS2_F2+APCS2_R2 | 0.1          | APCXNA002S   | 0.5       | 40  | 40  | 40  | 29.78 | 29.78 | 29.92 | 29.92 |
| 3 | APC1450 | APC 53.1_F2+      | 0.1          | APC3.1XNA001 | 0.0125    | 40  | 40  | 40  | 34.36 | 33.26 | 33.79 | 33.79 |

**Table 39.** Primers screened for BCT 41 and BCT 45.

| Primer Name | Primer Sequence |
|---|---|
| **PBBCT-F** - SEQ ID NO:18 | ACTCTGGAATCCATTCTGGTGC |
| **PBBCT-R** - SEQ ID NO:19 | AGAAAATCCCTGTTCCCACTCATA |
| **LPBCT-F2** - SEQ ID NO:20 | ATCCATTCTGGTGCCACTAC |
| **LPBCT-R2** - SEQ ID NO:21 | ACTTGGGAGGTATCCACATC |

A Primer/XNA Matrix was run to find the optimal Primer/XNA concentrations which gave the best differential between WT and 5%MT of BCT c41. The primer/XNA matrix analysis was summarized in Table 40.

Table 40. Matrix analysis of XNA and primer concentration for BCT 41.

| F | R | XNA | | | | | | |
|---|---|---|---|---|---|---|---|---|
| PBBCT-F | PBBCT-R | BCT41XNA00 | 1 | 2 | 3 | AVE | SD | %CV |
| PRIMER, uM | XNA, uM | WT | 40 | 40 | 40 | 40 | 0 | 0 |
| 0.75 | 5 | 5%BCTCD41 | 28.46 | 28.57 | 28.11 | 28.38 | 0.24 | 0.01 |
| | | 1%BCTCD41 | 28.9 | 30.05 | 32.09 | 30.35 | 1.62 | 0.05 |
| | | 0.1%BCTCD41 | 32.29 | 34.95 | 36.13 | 34.46 | 1.97 | 0.06 |

5.3.1. For the other targeted mutations including BRAF V600 and KRAS c12 and c13, the primer pairs that were used in DiaCarta Qclamp SYBR Kits of BRAF and KRAS c12 and KRAS c13 assays were also used in the development of the Taqman probe based BRAF and KRAS c12 and KRAS c13 mutation detection assays.

5.3.2. Primer, probe and XNA combinations and concentrations that resulted in highest delta Ct (measured as the difference between Cts of the mutation detection assay for the WT and 5%Mut samples) were selected for each targeted mutation detection assay.

5.3.3. The following primers and probes and XNA showed the best performance in regarding to differentiating mutant and WT alleles. For more details in the screening of primers by SYBR assay, please see the attached file with this document.

**Table 41.** Names of Primers, probes and XNAs selected for final configuration of assays:

| Assay | Forward Primer | Reverse Primer | Probe | XN |
|---|---|---|---|---|
| CTNNB1 c41 | PBCTNNB1-F | PBCTNNB1-R | CTNNB1 | CS05S |
| CTNNB1 c45 | PBCTNNB1-F | PBCTNNB1-R | CTNNB1 | CS06S |
| KRAS c12 | KRASBioFP002 | KRASG12VBPR00 | KRASCS02 | DPCK001C2 |
| KRAS cl3 | C13F001 | KRASG12VBPR00 | KRASCS02 | DPCK002B |
| BRAF C600 | BRAFAZFPNEW0 | BRAFAZRP001 | BRAF600P01 | BR001B |
| APC c1367 and c1309 | EAPC 1367F001 APC 1309TAQ-F | EAPC 1367R001 APC 1309TAQ-R | APC1309Pr APC1367 Zen | APCXNA002S |
| APC 1450 | APC3_1F002 | APC3_1R002 | APC 1450_01 | CS03.1 |
| External | ACTBF | ACTBR | ACTBPr | |

**Table 42.** Final Composition of assay of the invention

| Target | Component Name | Component Sequence | Final Concentration (nM) |
|---|---|---|---|
| APC 1309DEL | APC 1309TAQ-F - SEQ ID NO:22 | ACGACACAGGAAGCAGATTCT | 300 |
| APC 1309DEL | APC 1309TAQ-R-SEQ ID NO:23 | TCACAGGATCTTCAGCTGACCT | 300 |
| APC 1309DEL | APC 1309Pr - SEQ ID NO: 24 | TTCCAATCTTTTATTTCTGCTATT | 250 |
| APC 1309DEL | APCXNA001A-SEQ ID NO: 25 | Lys-O-(CTGACCTAGTTCCAATCTTTTCTT)PNA | 250 |
| APC 1367C>T | EAPC 1367F001-SEQ ID NO:26 | TTCAGGAGCGAAATCTCCC | 400 |
| APC 1367C>T | EAPC 1367R001-SEQ ID NO:27 | TGAACATAGTGTTCAGGTG | 400 |
| APC 1367C>T | APC 1367 Zen probe-2 SEQ ID NO:28 | | 200 |
| APC 1367C>T | APCXNA002S SEQ ID NO: 29 | Lys-O-(AGTGGTGCTCAGACA)PNA | 250 |
| APC c1450 | APC3_1F002 SEQ ID NO: 30 | CCAGATAGCCCTGGACAAACC | 400 |
| APC c1450 | APC3_1R002 SEQ ID NO: 31 | CTTTTCAGCAGTAGGTGCTTTATTTTTA | 400 |
| APC c1450 | APC1450 01 SEQ ID NO:32 | AGGTACTTCTCACTTGGTTT | 200 |
| APC cl450 | CS03.1 SEQ ID NO:33 | TAGGTACTTCTCGCTTGGTTT | 250 |
| CTNNB 1 c41 | PB-CTNNB1-F SEQ ID NO: 34 | ACTCTGGAATCCATTCTGGTGC | 200 |
| CTNNB 1 c41 | PB-CTNNB 1-R SEQ ID NO: 35 | AGAAAATCCCTGTTCCCACTCATA | 200 |
| CTNNB1 c41 | CTNNB1M02S SEQ ID NO: 36 | AGGAAGAGGATGTGGATACCTCCCAAG | 100 |
| CTNNB 1 c41 | CS05SXNA SEQ ID NO:37 | Lys-O-(TGCCACTACCACAGCTC)PNA | 500 |
| CTNNB1 c45 | PB-CTNNB2-F SEQ ID NO: 38 | ACTCTGGAATCCATTCTGGTGC | 100 |
| CTNNB1 c45 | PB-CTNNB2-R SEQ ID NO: 39 | AGAAAATCCCTGTTCCCACTCATA | 100 |
| CTNNB1 c45 | CTNNB2M02S SEQ ID NO: 40 | AGGAAGAGGATGTGGATACCTCCCAAG | 50 |
| CTNNB1 c45 | CS06SXNA SEQ ID NO:41 | Ac-CTCCTTCTCTGAGTG-NH2 | 500 |
| KRAS c12 | KRASBioFP002 SEQ ID NO:42 | AAGGCCTGCTGAAAATGACTG | 200 |
| KRAS c12 | KRASG12VBPR001 SEQ ID NO:43 | GTTGGATCATATTCGTCCAC | 200 |

(continued)

| Target | Component Name | Component Sequence | Final Concentration (nM) |
|---|---|---|---|
| KRASc12 | KRASCS02 SEQ ID NO:44 | TCTGAATTAGCTGTATCGTCAAGGCACTC | 100 |
| KRAS c12 | K001C2XNA SEQ ID NO:45 | CTACGCCACCAGCTCCAACTACCA-O-D-Lys | 250 |
| KRAS c13 | C13F001 SEQ ID NO:46 | ACTTGTGGTAGTTGGAGCTGGT | 200 |
| KRAS c13 | KRASG12VBPR002 SEQ ID NO:47 | GTTGGATCATATTCGTCCAC | 200 |
| KRAS c13 | KRASCS03 SEQ ID NO:48 | TCTGAATTAGCTGTATCGTCAAGGCACTC | 100 |
| KRAS cl3 | K002BXNA SEQ ID NO:49 | D-LYS-PEG2-TCTTGCCTACGCCACCAGCTCCA-NH2 | 500 |
| BRAF c600 | BRAFAZFPNEW02 SEQ ID NO:50 | ACAGTAAAAATAGGTGATTTTGGTCTAGCTA | 100 |
| BRAF c600 | BRAFAZRP001 SEQ ID NO:51 | CATCCACAAAATGGATCCAGACAA | 100 |
| BRAF c600 | BRAF600P01 SEQ ID NO: 52 | CAAACTGATGGGACCCACTCCATCG | 50 |
| BRAF C600 E | BR001B SEQ ID NO:53 | ATCGAGATTTCACTGTAGCTAGAC | 500 |
| ACTP | ACTBF SEQ ID NO:54 | CCTGGACTTCGAGCAAGAGA | 100 |
| ACTβ | ACTBR SEQ ID NO:55 | CCGTCAGGCAGCTCGTA | 100 |
| ACTβ | ActBPr SEQ ID NO:56 | CTTCCAGCTCCTCCCTGGAGAA | 100 |

5.3. Examples of amplification curves for final configuration of the invention assays

5.3.1. The following figures illustrate the performance examples present invention assays with optimal primer, probe, XNA concentration and ΔCt between Wt and mutant.

5.3. Preliminary Analytical Validation for present invention

Based on experiments on each of the invention target primers, probe and XNA combination and titration, optimal conditions were obtained for each targeted mutation detection assay as listed in Table 22 and illustrated in figures above. The finalized assays of the invention were assessed for test specificity, sensitivity and reproducibility.

5.3.1. Accuracy

A set of cell lines with known mutation status were tested to evaluate the assay of the invention accuracy. The invention assays were run on the Roche LC 96 instrument. Only expected mutations were detected in all tested cell lines.

Table 43. CTNNB1 c41, CTNNB1 c45, KRAS c12, KRAS c13 and BRAF c600 tests on cell lines with known mutation status.

| Cell line | Known Mutation status | Invention Test result (Ct ± SD) | Correct? Yes/No |
|---|---|---|---|
| SW1417 | Cd 1450 (C>T), BRAFV600 E | BRAFV600E (34.74 ± 1.11) | **Yes** |
| HDC 135 | Cd. 41 (ACC > ATC), BRAFV600 E | CD41 (34.39 ± 0.42), BRAF C600 (35.96) | **Yes** |
| C2BBE1 | Cd. 1367 (C>T) | Cd. 1367 | **Yes** |
| LS1034 | E1309fs * | E1309fs | **Yes** |

(continued)

| Cell line | Known Mutation status | Invention Test result (Ct ± SD) | Correct? Yes/No |
|---|---|---|---|
| SW48 | p.S33Y | no mutations detected | **Yes*** |
| LS174T | CD45 | CD 45 (33.10 ± 0.01) | **Yes** |
| LIM1215 | p.T41A | CD41 (35.20 ± 1.08) | **Yes** |
| CW2 | APC1465 DELAG | no mutations detected | |
| HCT 116 | Cd. 45 delTCT. KRAS Glv to Asp (13) | CD45 (31.89 ± 0.04). G13D | **Yes** |
| NC 14549 | APC CD1556 INSA | BRAF C600 (33.4 ± 0.5) | **Yes** |
| COLO678 | T1556fs*3. KRAS Gly to Asp (12) | KRAS 12 (34.72 ± 0.23) | **Yes** |
| HDC73 | WT | no mutations detected | **Yes** |

5.3.1. Analytical sensitivity

Analytical Sensitivity was determined by testing of DNA samples with a serial dilution of DNA into wild type DNA. Mutation detection assays were performed on DNA samples with 5%, 1%, 0.5%, 0.1%, mutation DNA in wt background respectively. The lowest percentage of mutated DNA in wild type background that can be detected is determined. At least 0.5% of mutation DNA in wild type background can be detected by the invention Taqman assays (See Table 18) and Table 19.

**Table 44.** Invention Mutation detection assays of mutation DNA diluted to wt DNA (5%, 1%, 0.5% and 0.10%) with PCR cycles of 40 and run file data analysis using the Abs Quant/Fit Points algorithm. The control is in HEX channel while all the targeted mutations are in Fam. Delta Ct was calculated for each sample as follows: Ct difference (ΔCt) = Mutation Assay Ct - Control Assay Ct.

**Table** 45. Sensitivity of the invention Mutation detection assays.

| Assay | Ct Hex | SD | Ct Fam | SD | ΔCt = Ct Fam - Ct | SD |
|---|---|---|---|---|---|---|
| BCT c41 | | | | | | |
| 5% | 26.28333 | 0.516946 | 29.82333 | 0.520128 | 3.54 | 0.271846 |
| 1% | 26.73 | 0.096437 | 32.4 | 0.450777 | 5.67 | 0.43 |
| 0.50% | 26.28 | 0.450333 | 33.30333 | 0.288848 | 7.023333333 | 0.161967 |
| 1% | 26.99 | 0.554346 | 34.93667 | 0.959184 | 7.946666667 | 0.972077 |
| CC | 26.02 | 0.517397 | 39.10667 | 1.547299 | 13.08666667 | 2.046811 |
| BCT c45 | | | | | | |
| 5% | 25.59333 | 0.787676 | 29.92667 | 0.763566 | 4.333333333 | 0.722519 |
| 0.50% | 25.72333 | 0.089629 | 32.98 | 0.29 | 7.256666667 | 0.368284 |
| CC | 25.90667 | 0.381095 | 40 | 0 | 14.09333333 | 0.381095 |
| KRAS c12 | | | | | | |
| 5% | 26.02 | 0.269629 | 31.39333 | 0.851254 | 5.373333333 | 0.608632 |
| 1% | 26.46333 | 0.556088 | 34.40333 | 0.757914 | 7.73 | 0.293087 |
| 0.50% | 26.04667 | 0.305505 | 35.83333 | 0.803762 | 9.786666667 | 0.715565 |
| CC | 26.27 | 0.26 | 40 | 0 | 13.73 | 0.26 |
| KRAS c13 | | | | | | |
| 5% | 26.14333 | 0.198578 | 32.18667 | 0.802579 | 6.043333333 | 0.791981 |

(continued)

| KRAS c13 | | | | | | |
|---|---|---|---|---|---|---|
| 1% | 26.39333 | 0.089629 | 35.45 | 0.840417 | 9.056666667 | 0.801519 |
| 0.50% | 26.15667 | 0.428991 | 36.48 | 1.05 | 10.32333333 | 1.057607 |
| CC | 26.04333 | 0.412593 | 40 | 0 | 13.95666667 | 0.412593 |
| BRAF V600E | | | | | | |
| 5% | 25.44 | 0.991817 | 30.68333 | 0.115902 | 5.243333333 | 0.876147 |
| 1% | 25.67667 | 0.246644 | 32.56333 | 0.661085 | 6.886666667 | 0.552298 |
| 0.50% | 25.4 | 0.043589 | 32.87 | 0.144222 | 7.47 | 0.101489 |
| 0.10% | 25.935 | 0.459619 | 34.755 | 0.13435 | 8.82 | 0.325269 |
| CC | 25.62667 | 0.219621 | 37.45 | 2.225421 | 11.82333333 | 2.356742 |

Mutant DNA diluted into WT DNA to 5%, 1% and 0.10%. 50 cycles PCR and run file data analysis using the Abs Quant/2nd derivative max algorithm. The control is in HEX channel while all the targeted mutations are in Fam. Delta Ct was calculated for each sample as follows: Ct difference ($\Delta$Ct) = Mutation Assay Ct - Control Assay Ct.

| | Ct Hex | | Ct Fam | | $\Delta$Ct = Ct Fam - Ct Hex | $\Delta$Ct = Ct Fam - Ct Hex |
|---|---|---|---|---|---|---|
| **APC 1309** | Run1 | Run2 | Run1 | Run2 | Run1 | Run2 |
| **5%** | 29.01 | 28.85 | 30.81 | 43.43 | 1.8 | 14.58 |
| **1%** | 28.63 | 28.55 | 32.81 | 46.39 | 4.18 | 17.84 |
| **0.10%** | 28.5 | 28.49 | 33.15 | 50 | 4.65 | 21.51 |
| **CC** | 29.01 | 28.69 | 50 | 50 | 20.99 | 21.31 |
| **APC 1367** | | | | | | |
| **5%** | 28.94 | 28.77 | 31.47 | 31.65 | 2.53 | **2.88** |
| | 28.69 | 28.69 | | | | |
| **1%** | 28.71 | 28.63 | 33.78 | 34.2 | 5.09 | **5.57** |
| **0.10%** | 28.71 | 28.53 | 35.83 | 37.05 | 7.12 | **8.52** |
| **CC** | 28.94 | 28.76 | 50 | 50 | 21.06 | **21.24** |
| **APC 1450** | | | | | | |
| **5%** | 29.04 | 29.21 | 34.05 | 35.65 | 5.01 | **6.44** |
| **1%** | 28.98 | 29.25 | 36.49 | 38.16 | 7.51 | **8.91** |
| **0.10%** | 28.87 | 29.12 | 39.36 | 41.49 | 10.49 | **12.37** |
| **CC** | 29.04 | 29.01 | 47.37 | 45.64 | 18.33 | **16.63** |
| **CTNNB1 CD41** | | | | | | |
| **5%** | 28.85 | 29.63 | 31.96 | 33.04 | 3.11 | **3.41** |
| **1%** | 28.89 | 29.22 | 34.66 | 39.28 | 5.77 | **10.06** |
| **0.10%** | 28.5 | 29.15 | 39.99 | 37.25 | 11.49 | **8.1** |
| **CC** | 28.86 | 29.02 | 40.06 | 39.98 | 11.2 | **10.96** |
| **CTNNB1 CD45** | | | | | | |
| **5%** | 29 | 29.83 | 31.34 | 31.59 | 2.34 | 1.76 |

(continued)

| CTNNB1 CD45 | | | | | | |
|---|---|---|---|---|---|---|
| **1%** | 28.98 | 29.07 | 34.1 | 34.57 | 5.12 | 5.5 |
| **0.10%** | 28.75 | 28.76 | 36.53 | 38.25 | 7.78 | 9.49 |
| **CC** | 29 | 28.95 | 41.13 | 41.47 | 12.13 | 12.52 |
| **KRAS CD12** | | | | | | |
| **5%** | 28.87 | 28.95 | 34.51 | 34.17 | 5.64 | 5.22 |
| **1%** | 28.77 | 28.66 | 37.17 | 37.87 | 8.4 | 9.21 |
| **0.10%** | 28.81 | 28.93 | 42.12 | 45 | 13.31 | **16.07** |
| **CC** | 28.87 | 28.92 | 43.54 | 43.28 | 14.67 | **14.36** |
| **KRAS CD13** | | | | | | |
| **5%** | 28.84 | 28.89 | 36.17 | 36.2 | 7.33 | **7.31** |
| **1%** | 28.88 | 28.95 | 39.95 | 40.23 | 11.07 | **11.28** |
| **0.10%** | 28.93 | 29.11 | 42.78 | 43.9 | 13.85 | **14.79** |
| **CC** | 28.84 | 29.19 | 43.99 | 46.67 | 15.15 | **17.48** |
| **BRAF** | | | | | | |
| **5%** | 28.97 | 29.18 | 32.61 | 32.75 | 3.64 | **3.57** |
| **1%** | 28.88 | 29.04 | 35.52 | 34.75 | 6.64 | **5.71** |
| **0.10%** | 28.71 | 29.05 | 38.05 | 37.13 | 9.34 | **8.08** |
| **CC** | 28.97 | 29.19 | 39.65 | 39.95 | 10.68 | **10.76** |

### 5.3.1. Assay Precision

Reproducibility (Precision) of the invention assays was demonstrated by comparing test results from mutation detection assays on 5% AF sample from multiple runs throughout the feasibility study period (See Table 46 and 47). %CV values were calculated within runs and between runs to test inter-assay and intra-assay precision (Table 47 and 48).
Data presented in tables 47 and 48 indicated that all the invention assays have good intra- and inter- assay precision with %CV <10.

**Table 46.** Assay Precision and instrument comparison: Inter-assay reproducibility: Invention Taqman assays run on different dates on LC96

| Assay Targeted mutation | Run 1 Average Ct (mean ± SD) | | | Run2 Average Ct (mean ± SD) | | | |
|---|---|---|---|---|---|---|---|
| | 5% PC | WT | Δ Ct | Targeted mutation | 5% PC | WT | Δ Ct |
| CTNNB 1 c41 (0.5%) | 33.9 ± 0.73 (CV 2.15%) | 40 ± 0 | 6.1 | CTNNB1 c41 | 30.61 ± 0.16 (CV 0.52%) | 40 ± 0 | 9.39 |
| CTNNB1 c45 | 31.25 ± 0.33(CV 1.05%) | 40 ± 0 | 8.75 | CTNNB1 c45 | 31.01 ± 0.38 (CV 1.22%) | 40 ±0 | 8.99 |
| KRAS c12 | 30.99 ± 0.40 (CV 1.29%) | 40 ± 0 | 9.01 | KRAS c12 | 30.66 ± 0.35 (CV 1.14%) | 40 ±0 | 9.34 |
| KRAS cl3 | 30.12 ± 0.18 (CV 0.6%) | 40 ± 0 | 9.88 | KRAS c13 | 30.32 ± 0.11 (CV 0.36%) | 40 ± 0 | 9.68 |
| BRAF C600 (0.5%) | 33.23 ± 0.75 (CV 2.25%) | 40 ± 0 | 6.77 | BRAF C600 | 30.09 ± 0.36 (CV 1.20%) | 40 ± 0 | 9.91 |

**Table 47.** Assay Precision and instrument comparison: Inter-assay reproducibility: Invention Taqman assays run on different dates on LC480

| Assay | Run 1 | | | | Run 2 | | | |
|---|---|---|---|---|---|---|---|---|
| | Average Ct (mean ± SD) | | | | Average Ct (mean ± SD) | | | |
| Targeted mutation | 5% PC | WT | Δ Ct | | Targeted mutation | 5% PC | WT | Δ Ct |
| APC 1309 | 31.37 ± 1.75 | 40 ± 0 | 8.63 | | APC 1309 | 31.91 ± 0.07 | 40 ± 0 | 8.09 |
| APC 1367 | 32.45 ± 0.43 | 40 ± 0 | 7.55 | | APC 1367 | 31.03 ± 0.45 | 40 ± 0 | 8.97 |
| APC 1450 | 32.73 ± 0.37 | 40 ± 0 | 7.27 | | APC 1450 | 32.41 ± 0.88 | 40 ± 0 | 7.59 |
| CTNNB1 c41 | 28.22 ± 0.43 | 37.96 ± 1.76 | 9.74 | | CTNNB1 c41 | 29.2 ± 0.28 | 40 ± 0 | 10.8 |
| CTNNB1 c45 | 30.08 ± 0.57 | 40 ± 0 | 9.92 | | CTNNB1 c45 | 29.39 ± 0.22 | 37.1 ± 0.7 | 7.71 |
| KRAS c12 | 29.1 ± 1.47 | 40 ± 0 | 10.9 | | KRAS c12 | 30.35 ± 0.57 | 39.27 ± 1.2 | 8.92 |
| KRAS c13 | 32.1 ± 0.26 | 40 ± 0 | 7.9 | | KRAS cl3 | 32.43 ± 0.26 | 40 ± 0 | 7.57 |
| BRAF C600 | 32.73 ± 0.26 | 40 ± 0 | 7.27 | | BRAF C600 | 31.38 ± 0.38 | 40 ± 0 | 8.62 |

**Table 48.** Assay precision (Intra-assay reproducibility)

| Assay | Average Ct (mean ± SD) | | | |
|---|---|---|---|---|
| Targeted mutation | 5% PC | | WT | ΔCt |
| APC 1309 | 31.37 ± 1.75 | (CV 5.6%) | 40 ± 0 | 8.63 |
| APC 1367 | 32.45 ± 0.43 | (CV 1.3%) | 40 ± 0 | 7.55 |
| APC 1450 | 32.73 ± 0.37 | (CV 1.1%) | 40 ± 0 | 7.27 |
| BCT c41 | 28.22 ± 0.43 | (CV 1.5%) | 37.96 ± 1.76 | 9.74 |
| BCT c45 | 30.08 ± 0.57 | (CV 1.9%) | 40 ± 0 | 9.92 |
| KRAS c12 | 29.1 ± 1.47 | (CV 5.1%) | 40 ± 0 | 10.9 |
| KRAS c13 | 32.1 ± 0.26 | (CV 0.8%) | 40 ± 0 | 7.9 |
| BRAF V600 | 32.73 ± 0.26 | (CV 0.8%) | 40 ± 0 | 7.27 |

**Table 49.** Table Assay precision (Inter-assay reproducibility)

| Assay | 5% | | | | | |
|---|---|---|---|---|---|---|
| | AVE | SD | CV % | AVE | SD | CV % |
| APC 1309 | 31.64 | 0.381838 | 1.206819 | 40 | 0 | 0 |
| APC 1367 | 31.74 | 1.004092 | 3.16349 | 40 | 0 | 0 |
| APC 1450 | 32.57 | 0.226274 | 0.694732 | 40 | 0 | 0 |
| CTNNB 1 c41 | 28.71 | 0.692965 | 2.41367 | 38.98 | 1.44 | 1.694202 |

(continued)

| Assay | 5% | | | | | |
|---|---|---|---|---|---|---|
| | AVE | SD | CV % | AVE | SD | CV % |
| CTNNB1 c45 | 29.735 | .487904 | 1.64084 | 38.55 | 2.05061 | 5.319351 |
| KRAS12 | 29.725 | 0.883883 | 2.973536 | 39.635 | 0.516188 | 1.302354 |
| KRAS13 | 32.265 | 0.233345 | 3.723215 | 40 | 0 | 0 |
| BRAF C600 | 32.055 | 0.954594 | 2.977988 | 40 | 0 | 0 |

5.3.1. Analytical specificity

Analytical specificity was tested by performing the assay on reference samples with known mutation negative status. All the test results were as expected (see Table 43).

5.7.1. Limit of blank was tested by performing the assay on the NTC samples.

All tested NTC samples were called negative.

1. Conclusions

1.1. The final design is presented in the Tables 41 and 42.

1.2. Final assay PCR cycling parameters are presented in section 5.2.3:
95°C for 5 min followed by 50 cycles of 95°C 20 seconds, 70°C 40 seconds, 64°C 30 seconds and 72°C 30 seconds (data acquision).

1.3. The present invention design demonstrated that the performance parameters of the tested design met or exceeded specifications set in the product requirement document (DDC.0006) and the assay is ready for the development stage.

1.3.1. Product requirement 1 for the sample types tested will be tested in the Matrix interference test of the Verification study. Requirements 11-15 will be also tested in Verification and Stability studies of the Development stage.

1.3.2. Product requirement 2 is met: Under 60 min for reaction setup and under 2.5 h for the reaction PCR run on the three qPCR instruments tested.

1.3.3. Product requirement 3 will be addressed in a separate stool DNA preparation study

1.3.4. Product requirement 4 is met by having 7 reaction mixes where each gene is tested in a separate reaction mix, KRAS 12 and KRAS 13 are in two separate reactions; CTNNB 41 and 45
are also in 2 separate reactions. APC is tested in 2 tubes.

1.3.5. Product requirement 5 is met by testing the assay on all three listed qPCR instruments - Roche LC96 and LC480 and BioRad CFX

1.3.6. Product requirement 6 is met by including the internal control assay in each reaction tube that provides evidence of the sufficient quantity of amplifyable DNA in each reaction well.

1.3.7. Product requirement 7 is met, kit contains NTC, WT control and mixed positive control.

1.3.8. At least 0.5% of mutant DNA in wild type background can be detected by the present invention Taqman assays (high sensitivity) with total DNA input of 2.5 ng/well. Exceeds Product requirement 8 set for detection of 1% mutant DNA.

1.3.9. The data presented in this report demonstrate the feasibility of the present invention design to detect intended mutations with no cross-reactivity observed. Product requirement 9 1.3.10. The design also showed good intra and inter- assay reproducibility (CV < 10%).

Product requirement 10 is met.

1.4. The final design is presented in the Tables 41 and 42.

1.5. Final assay PCR cycling parameters are presented in section 5.2.3: 95°C for 5 min followed by 50 cycles of 95°C 20 seconds, 70°C 40 seconds, 64°C 30 seconds and 72°C 30 seconds (data acquisition).

## EXAMPLE IV

This example further describes the verification and validation studies of the assay of the invention for qualitative detection of mutations in targeted genes of *APC (codons 1309, 1367, 1450), KRAS (codons 12 and 13), BRAF (codon 600) and CTNNB1 (codons 41 and 45)* genes associated with colorectal cancer initiating events. The assay and kit has been validated for precision, limit of detection (LOD), stability, specificity/cross-reactivity and matrix interference.

The verification and validation studies were performed on two development lots of the assays and kits. Mixed positive controls were used as test samples except that positive controls for APC 1309 and APC 1367 were prepared individually for the LOD studies. The mutation detection protocol is as described in the present invention for doing the test samples. The validation tests were run on LC 480 (for instrument comparison, the tests were also run on BioRad 384).

The assay of the invention is a real-time qPCR-based *in vitro* diagnostic test intended for use in the detection of mutations in the *APC (codons 1309, 1367, and 1450), KRAS (codons 12 and 13), BRAF (codon 600) and CTNNB1 (codons 41 and 45)* genes in DNA extracted from FFPE sections and Human stool samples.

## OUTLINE OF THE VALIDATION PLAN

Test analytic sensitivity of the assay (LOD) and allelic frequency

Test Limit of Blank of the assay

Test matrix interference (e.g. FFPE extraction, add ethanol) for the potential inhibitory effect of several substances that would most probably be encountered in the real patient samples Test cross-reactivity (detection of each of the present invention target DNA).

## Test reproducibility of the assay:

Intra-assay: replicate samples representative of all mutations near LOD

Inter-assay: 3x3 samples in 3 runs per instrument

Lot-to-lot variation tested by repeating 1.5.1 and 1.5.2 on second lot, on same run Instrument comparison on Roche LC480, BioRad CFX384

Operator variability (2 operators test same lot on the same day on same instrument)

Test analytic specificity on both lots

Analytic Specificity test on high concentration of WT reference samples

Invention stability studies

## Accelerated stability studies

Freeze-thaw stability studies

Real-time stability studies

Deviations from the planned V&V of the invention assay analytical performance

Sensifast lyophilized Bioline mastermix was reverted to KAPA Universal 2x liquid formulation for the two reasons: The timelines of the manufacturing on the Bioline side were too long and The assay sensitivity at 1% mutation was not as good as with KAPA Manufacturing: Reagents for some primer-probe mixes were purchased separately for lot 2, others- same

The tubes used to aliquot the kits were from USA scientific, planned to be change to the stock of approved tubes from Fisher Scientific that are used for all the current product manufacturing.

The run time for the BioRad CFX 384 instrument exceeds 2h limit set as Product Requirement #5. The requirement was not an essential one and 2.5h run time was considered acceptable.

## 1. MATERIALS AND METHODS

Composition of the PCR reaction Mix

Table 50. PCR reaction mix

| Reagent | Volume in 10 $\mu$l reaction, $\mu$l | Final concentration |
|---|---|---|
| 2x Master Mix | 5 | 1x |
| 5X Primer and probe mix | 2 | 1X |
| 10X XNA | 1 | 1X |

(continued)

| Reagent | Volume in 10 μl reaction, μl | Final concentration |
|---|---|---|
| Template | 2 | |
| TOTAL | 10 | |

Reference templates:

CTNNB 1 CD 41: IDT gBlock, custom
CTNNB1 CD 45: ATCC CCL-247_D1
BRAF C600: BRAF C600 Reference standard (Horizon Cat#: HD238)
KRAS c13: KRAS G13D Reference Standard (Horizon Cat#: HD290)
KRAS c12: KRAS G12D Reference Standard (Horizon Cat#: HD272)
APC 1309: ATCC CRL-2158_D1
APC 1367: ATCC CRL-2102_D1
APC 1450: ATCC CCL-235_D1
Instruments
Roche LC480II DC2035, S/N 5536
BioRad CFX384 DC2044, S/N 786BR02318
Reagents/Kits
2 development lots (DL-1 and DL-2) of the Multiplexed Colorectal Cancer detection Kits including:

- 2X PCR master mix,
- 5x invention primer and probe mixes,
- 10× present invention XNA mixes and
- mixed positive controls as described in the reference templates and
- non-template control (NTC, nuclease free water.) The report on the development lots is in DDC.0041

VERIFICATION OF ASSAY PERFORMANCE PARAMETERS

Analytical sensitivity of the assay (LOD)

Analytical sensitivity of the assay was evaluated by testing 1%, 0.5% and 0.1% mutant DNA template at 2.5 ng, 5 ng and 10 ng input for all the present invention targets. For each target, 1%, 0.5% and 0.1% mutation at each of the three DNA input level were tested in triplicates and on 3 separate runs on LC 480. No template control (NTC), wild type DNA (clamping control) and mixed positive controls (APC 1309 and APC 1367 positive controls were prepared individually) were included in each run. Average Ct values, standard deviation (SD) and coefficient of variation (%CV) were calculated for both FAM (target) and HEX (internal control). The ΔCt values (ΔCt = Ct Fam - Ct Hex) were calculated from the averaged Ct values (Table 51 to Table ). The average ΔCt values over all 3 DNA input levels for all three runs were calculated. The cut-off ΔCt is set to be the average ΔCt values - 1.5 ΔCt SD (Table ). Correct call percentage were calculated for 1%, 0.5% and 0.1% mutation detection of all target at 2.5 ng, 5 ng and 10 ng DNA input (Table 53 and Table 54). Correct call percentage were also calculated for 1% mutation detection of all target at 5 ng DNA input with all runs during the V&V period and results were incorporated in Tables 53 and 54.

**Table 51.** Average FAM CT values for WT, 1%, 0.5% and 0.1 % mutant DNA template of at 2.5 ng, 5 ng and 10 ng DNA input.

| | 2.5 ng | | | 5 ng | | | 10 ng | | |
|---|---|---|---|---|---|---|---|---|---|
| | WT | | | | | | | | |
| Target | AVE | SD | CV | AVE | SD | CV | AVE | SD | CV |
| APC1309 | 50 | 0 | 0.00% | 50 | 0 | 0 | 48.82 | 3.34 | 6.84% |
| APC1367 | 50 | 0 | 0.00% | 48.74 | 3.58 | 0.07 | 48.66 | 3.8 | 7.82% |
| APC1450 | 44.56 | 4.87 | 10.93% | 39.65 | 0.9 | 0.02 | 40.51 | 3.53 | 8.72% |

(continued)

| Target | 2.5 ng | | | 5 ng | | | 10 ng | | |
|---|---|---|---|---|---|---|---|---|---|
| | AVE | SD | CV | AVE | SD | CV | AVE | SD | CV |
| | | | | WT | | | | | |
| Target | AVE | SD | CV | AVE | SD | CV | AVE | SD | CV |
| BTC CD41 | 42.17 | 2.07 | 4.90% | 39.22 | 1.43 | 0.04 | 38.19 | 1.08 | 2.83% |
| BTC CD45 | 41.97 | 1.31 | 3.13% | 40.45 | 1.19 | 0.03 | 39.21 | 0.67 | 1.72% |
| KRAS CD12 | 44.76 | 2.38 | 5.31% | 41.21 | 1.44 | 0.03 | 40.94 | 1.5 | 3.67% |
| KRAS CD13 | 43.71 | 1.86 | 4.25% | 41.32 | 2.09 | 0.05 | 41.67 | 1.21 | 1.89% |
| BRAF V600 | 39.91 | 1.1 | 2.76% | 39.48 | 0.74 | 0.02 | 38.18 | 0.88 | 2.30% |
| Control | 30.05 | 0.14 | 0.46% | 29.09 | 0.13 | 0.43% | 28.14 | 0.14 | 0.49% |
| | 1% mutation | | | | | | | | |
| Target | AVE | SD | CV | AVE | SD | CV | AVE | SD | CV |
| APC1309 | 42.56 | 8.33 | 19.57% | 32.41 | 1.8 | 0.06 | 30.9 | 0.55 | 1.76% |
| APC1367 | 33.87 | 0.8 | 2.36% | 34.11 | 0.54 | 0.02 | 31.34 | 0.17 | 0.53% |
| APC1450 | 36.17 | 0.69 | 1.90% | 34.9 | 0.58 | 0.02 | 33.74 | 0.26 | 0.77% |
| BTC CD41 | 32.27 | 0.39 | 1.20% | 30.51 | 0.14 | 0 | 29.31 | 0.11 | 0.37% |
| BTC CD45 | 33.99 | 0.34 | 0.99% | 32.98 | 0.42 | 0.01 | 31.79 | 0.29 | 0.92% |
| KRAS CD12 | 37.59 | 2.6 | 6.92% | 35.55 | 0.76 | 0.02 | 34.11 | 0.37 | 1.09% |
| KRAS CD13 | 38.42 | 1.02 | 2.65% | 36.67 | 0.54 | 0.01 | 35.6 | 0.52 | 1.45% |
| BRAF V600 | 36.66 | 1.38 | 3.77% | 34.65 | 0.6 | .0.02 | 33.57 | 0.39 | 1.18% |
| Control | 29.88 | 0.16 | 0.55% | 28.88 | 0.11 | 0.38% | 28.11 | 0.13 | 0.48% |
| | 0.5% mutation | | | | | | | | |
| Target | AVE | SD | CV | AVE | SD | CV | AVE | SD | CV |
| APC1309 | 44.71 | 7.52 | 16.83% | 34.08 | 2.34 | 0.07 | 48.11 | 5.33 | 11.08% |
| APC1367 | 35.36 | 0.65 | 1.83% | 35.71 | 0.86 | 0.02 | 32.53 | 0.17 | 0.53% |
| APC1450 | 38.5 | 4.09 | 10.63% | 36.06 | 0.38 | 0.01 | 34.9 | 0.33 | 0.94% |
| BTC CD41 | 33.15 | 0.45 | 1.36% | 31.5 | 0.18 | 0.01 | 30.82 | 0.25 | 0.80% |
| BTC CD45 | 35.35 | 0.64 | 1.80% | 33.74 | 0.45 | 0.01 | 32.8 | 0.53 | 1.61% |
| KRAS CD12 | 39.81 | 2.4 | 6.04% | 36.72 | 0.96 | 0.03 | 35.18 | 0.69 | 1.96% |
| KRAS CD13 | 39.86 | 2.19 | 5.48% | 38.24 | 0.68 | 0.02 | 36.85 | 0.94 | 2.55% |
| BRAF V600 | 38.54 | 2.66 | 6.91% | 35.52 | 0.68 | 0.02 | 34.59 | 0.84 | 2.43% |
| Control | 29.91 | 0.2 | 0.66% | 28.92 | 0.17 | 0.60% | 28.12 | 0.19 | 0.68% |

(continued)

|  | 0.1% mutation | | | | | | | | |
|---|---|---|---|---|---|---|---|---|---|
| Target | AVE | SD | CV | AVE | SD | CV | AVE | SD | CV |
| APC1309 | 50 | 0 | 0.00% | 50 | 0 | 0 | 50 | 0 | 0.00% |
| APC1367 | 43.26 | 6.42 | 14.84% | 39.13 | 4.88 | 0.12 | 35.05 | 0.75 | 2.13% |
| APC1450 | 40.13 | 3.63 | 9.05% | 37.76 | 0.56 | 0.01 | 36.64 | 0.44 | 1.21% |
| BTC CD41 | 38.31 | 5.39 | 14.06% | 33.24 | 1.12 | 0.03 | 32.92 | 0.23 | 0.70% |
| BTC CD45 | 41.09 | 3.03 | 7.38% | 37.34 | 2.14 | 0.06 | 35.37 | 0.99 | 2.79% |
| KRAS CD12 | 42.19 | 2.88 | 6.83% | 40.42 | 2.37 | 0.06 | 37.34 | 1.58 | 4.24% |
| KRAS CD13 | 41.33 | 1.72 | 4.17% | 39.79 | 2.02 | 0.05 | 39.28 | 1.57 | 3.99% |
| BRAF V600 | 39.01 | 2.21 | 5.66% | 37.61 | 1.44 | 0.04 | 36.71 | 1.46 | 3.98% |
| Control | 29.9 | 0.22 | 0.72% | 28.82 | 0.17 | 0.58% | 28.08 | 0.1 | 0.35% |

Table 52. **Average ∆CT values for WT, 1%, 0.5% and 0.1% mutant DNA template of at 2.5 ng, 5 ng and 10 ng DNA input.**

| WT | | | | |
|---|---|---|---|---|
| Target | 2.5 ng | 5 ng | 10 ng | aver ∆Ct, all conc. |
| APC1309 | 20.16 | 21.07 | 20.38 | 20.54 |
| APC1367 | 20.12 | 19.85 | 20.55 | 20.17 |
| APC1450 | 14.55 | 10.57 | 12.4 | 12.51 |
| BTC CD41 | 12.07 | 9.97 | 10.12 | 10.72 |
| BTC CD45 | 11.83 | 11.23 | 11.15 | 11.4 |
| KRAS CD12 | 14.6 | 12.11 | 12.75 | 13.15 |
| KRAS CD13 | 13.48 | 12.16 | 13.57 | 13.07 |
| BRAF V600 | 9.76 | 10.34 | 9.97 | 10.03 |
| 1% PC | | | | |
| Target | AVE | AVE | AVE | |
| APC1309 | 12.69 | 3.43 | 9.84 | 8.65 |
| APC1367 | 4.15 | 5.2 | 3.42 | 4.25 |
| APC 1450 | 6.32 | 5.94 | 5.55 | 5.94 |
| BTC CD41 | 2.41 | 1.65 | 1.35 | 1.81 |
| BTC CD45 | 4.02 | 4.04 | 3.69 | 3.92 |
| KRAS CD12 | 7.62 | 6.69 | 5.94 | 6.75 |
| KRAS CD13 | 8.53 | 7.79 | 7.37 | 7.9 |
| BRAF V600 | 6.74 | 5.95 | 5.47 | 6.05 |
| 0.5% PC | | | | |
| Target | AVE | AVE | AVE | |
| APC1309 | 14.99 | 10.31 | 20.08 | 15.13 |

(continued)

| 0.5% PC | | | | |
|---|---|---|---|---|
| Target | AVE | AVE | AVE | |
| APC1367 | 5.33 | 6.77 | 4.42 | 5.51 |
| APC1450 | 8.71 | 7.14 | 6.78 | 7.54 |
| BTC CD41 | 3.08 | 2.53 | 2.7 | 2.77 |
| BTC CD45 | 5.46 | 4.85 | 4.65 | 4.99 |
| KRAS CD12 | 9.86 | 7.91 | 7.12 | 8.3 |
| KRAS CD13 | 9.87 | 9.34 | 8.63 | 9.28 |
| BRAF V600 | 8.71 | 6.69 | 6.42 | 7.28 |
| 0.1% PC | | | | |
| Target | AVE | AVE | AVE | |
| APC1309 | 20.57 | 21.27 | 21.85 | 21.23 |
| APC1367 | 13.49 | 1.65 | 6.93 | 7.35 |
| APC1450 | 10.27 | 8.9 | 8.63 | 9.27 |
| BTC CD41 | 8.15 | 4.41 | 4.86 | 5.8 |
| BTC CD45 | 11.12 | 8.42 | 7.29 | 8.94 |
| KRAS CD12 | 12.07 | 11.55 | 9.3 | 10.97 |
| KRAS CD13 | 11.3 | 10.96 | 11.09 | 11.12 |
| BRAF V600 | 9.12 | 8.88 | 8.72 | 8.91 |

**Table 53.** ΔCt cut-off values for Roche LC480

| Assay | ΔCt | Pass/fail | Overall% correct |
|---|---|---|---|
| APC 1309 | 17.04 | Pass | 96% |
| APC 1367 | 16.37 | Pass | 96% |
| APC 1450 | 7.62 | Pass | 100% |
| BCT 41 | 8.4 | Pass | 96% |
| BCT 45 | 9.5 | Pass | 100% |
| KRAS 12 | 10.83 | Pass | 96% |
| KRAS 13 | 10.89 | Pass | 100% |
| BRAF | 8.5 | Pass | 96% |
| | | Overall % correct | 98% |

Table 54. Analytical sensitivity of the present invention assay based on the cut-off values from Table 3

| Reference DNA | DNA Input, ng/well | | | |
| --- | --- | --- | --- | --- |
| | | 10 | 5 | 2.5 |
| | | % Correct Call | % Correct Call | % Correct Call |
| APC 1309 | 1% mutation | 63% | 94% | 44% |
| | 0.5% mutation | 12% | 67% | 33% |
| | 0.10% mutation | 0% | 0% | 0% |
| APC 1367 | 1% mutation | 100% | 100% | 100% |
| | 0.5% mutation | 100% | 100% | 100% |
| | 0.10% mutation | 100% | 67% | 56% |
| APC 1450 | 1% mutation | 100% | 100% | 100% |
| | 0.5% mutation | 100% | 100% | 67% |
| | 0.10% mutation | 0% | 0% | 0% |
| BCT 41 | 1% mutation | 100% | 100% | 100% |
| | 0.5% mutation | 100% | 100% | 100% |
| | 0.10% mutation | 100% | 100% | 78% |
| BCT 45 | 1% mutation | 100% | 100% | 100% |
| | 0.5% mutation | 100% | 100% | 100% |
| | 0.10% mutation | 100% | 73% | 22% |
| KRAS 12 | 1% mutation | 100% | 100% | 100% |
| | 0.5% mutation | 100% | 100% | 67% |
| | 0.10% mutation | 87% | 33% | 24% |
| KRAS 13 | 1% mutation | 100% | 100% | 100% |
| | 0.5% mutation | 100% | 100% | 100% |
| | 0.10% mutation | 56% | 56% | 56% |
| BRAF V600 | 1% mutation | 100% | 100% | 78% |
| | 0.5% mutation | 100% | 100% | 67% |
| | 0.10% mutation | 44% | 56% | 156% |

Note: The data in table 54 were calculated based on the values from all the experiments that contained 1%, 5% and 0.1% mutant data for 5 ng input.

Table 55. Limit of detection summary for LC480

| Assay | Limit of DNA (ng per PCR) |
| --- | --- |
| APC1309 | 5 |
| APC1367 | 2.5 |
| APC1450 | 2.5 |
| BTC CD41 | 2.5 |
| BTC CD45 | 2.5 |
| KRAS CD12 | 2.5 |

(continued)

| Assay | Limit of DNA (ng per PCR) |
|---|---|
| KRAS CD13 | 2.5 |
| BRAF V600 | 5 |

CONCLUSION:

- 0.5% mutation frequency in APC (c1367, c1450), BCT (c41, c45), KRAS (c12, c13) can be reliably detected (100% correct call) at as low as 2.5 ng DNA input per PCR reaction.
- at 5 ng DNA input 1% mutation in APC 1309 and BRAF V600 can be detected with 94% and 100% correct calls respectively. For APC (c1367, c1450), BCT (c41, c45) and KRAS (c12, c13) analytic sensitivity is 0.5% at this input with 100% correct calls.

Limit of Blank of the assay

Non-template controls (nuclease free water) were run with each validation test to monitor for contamination in the PCR. The data for NTC from 50 replicates from multiple runs were compiled (Table 56) and analyzed to estimate level of background noise of the present invention assays.

Table 56. Limit of Blank test result

| | APC1309/1367 | APC 1450 | BCT 41 | BCT 45 | KRAS 12 | KRAS 13 | BRAF V600 |
|---|---|---|---|---|---|---|---|
| n | 50 | 50 | 50 | 50 | 50 | 50 | 50 |
| Mean | 48.05 | 50 | 50 | 50 | 50 | 49.87 | 50 |
| SD | 0.74 | 0 | 0 | 3 | 0 | 0.92 | 0 |
| CV | 1.54% | 0 | 0 | 0 | 0 | 1.84% | 0 |

**CONCLUSION:** For present invention targets including APC 1450, BCT 41, BCT 45, KRAS 12 and BRAF V600, there is no background amplification noise with no detected amplification for these targets when testing NTC. For APC 1309/1367 and KRAS 13, there is minimal background noise with average Ct over 48 and 49 respectively.

Matrix Interference

To determine whether residual common substance in DNA isolated from FFPE has interfering inhibitory effect on the performance of the present invention assay, Ethanol (ETOH) was spiked in the DNA samples at 2%, 5% and 10% concentration and tested in 5 replicates on Roche LC 480. The average Ct values were calculated for each sample. The average Ct difference between each sample spiked with alcohol and the unspiked sample was calculated and summarized in Table 57.

**Table 57.** Matrix Interference

| Target | WT | | | | | | |
| | unspiked | 2% | Ct test - Ct un | 5% ETOH | Ct test - Ct un | 10% | Ct test-Ct un |
|---|---|---|---|---|---|---|---|
| APC1309/1367 | 50.00 | 50.00 | 0.00 | 50.00 | 0.00 | 50.00 | 0.00 |
| APC1450 | 44.75 | 45.47 | 0.72 | 46.26 | 1.51 | 39.35 | 5.40 |
| BTC CD41 | 41.69 | 43.35 | 1.65 | 43.55 | 1.86 | 46.73 | 5.04 |
| BTC CD45 | 42.35 | 43.98 | 1.63 | 44.54 | 2.20 | 41.86 | 0.49 |
| KRAS CD12 | 43.59 | 43.41 | 0.18 | 47.00 | 3.41 | 50.00 | 6.41 |
| KRAS CD13 | 45.00 | 47.00 | 2.00 | 46.00 | 1.00 | 50.00 | 5.00 |

(continued)

| | WT | | | | | | |
|---|---|---|---|---|---|---|---|
| Target | unspiked | 2% | Ct test - Ct un | 5% ETOH | Ct test - Ct un | 10% | Ct test-Ct un |
| BRAF V600 | 41.86 | 42.15 | 0.30 | 41.46 | 0.40 | 43.68 | 1.82 |
| | PC | | | | | | |
| Target | unspiked | 2% | Ct test - Ct un | 5% ETOH | Ct test - Ct un | 10% | Ct test-Ct un |
| APC1309/1367 | 30.57 | 30.70 | 0.13 | 30.81 | 0.24 | 30.73 | 0.16 |
| APC1450 | 31.88 | 31.87 | 0.02 | 32.26 | 0.37 | 32.09 | 0.20 |
| BTC CD4] | 28.71 | 28.98 | 0.27 | 28.80 | 0.09 | 28.84 | 0.13 |
| BTC CD45 | 30.73 | 30.92 | 0.19 | 31.01 | 0.28 | 31.12 | 0.39 |
| KRAS CD12 | 32.35 | 33.47 | 1.12 | 33.79 | 1.44 | 37.85 | 5.50 |
| KRAS CD13 | 33.05 | 35.35 | 2.31 | 35.67 | 2.62 | 44.70 | 11.66 |
| BRAF V600 | 32.34 | 33.54 | 1.20 | 32.84 | 0.50 | 33.61 | 1.27 |

**CONCLUSION:**

The Ct difference between the unspiked and spiked test was used to determine if the tested ETOH amount caused inhibition of the present invention qPCR reactions. Data in Table showed that there is no EHOH interference on the present invention assays with up to 10% ETOH spiked in samples for most present invention targets including APC 1309/1367, APC 1450, BCT 41, BCT 45 and BRAF V600. KRAS 13amplification was inhibited by as little as 2% ETOH (dCT over 2).

Cross-reactivity.

There are 8 target mutation detection reactions in the present invention assay. Each target assay was tested against all positive reference material to evaluate the cross-reactivity. Each assay mix was tested with three replicates of the eight individual 1% mutation standards. Some of the reference materials carry more than one target mutations (e.g. the BRAF reference standard from Horizon carries BRAF V600E, BCT 45 and KRAS 13 mutations at 50% frequency, the BCT 45 standard from ATCC also carries KRAS 13 mutation at 50% frequency). $\Delta$Ct (Ct Fam- CtHex) was calculated for each standard with all the mutation reactions and summarized in Table 58. Mutational status (Positive or Negative) of each test sample was determined on the basis of the cut-off dCT values (see Table 53).

**Table 58.** Cross-reactivity of the invention assays

| Target | APC 1309 | APC 1367 | APC 1450 | BTC CD41 | BTC CD45 | KRAS CD12 | KRAS CD13 | BRAF V600 | |
|---|---|---|---|---|---|---|---|---|---|
| APC 1309/1367 | 9.79 | 4.45 | 21.48 | 21.11 | 21.17 | 21.36 | 21.41 | 21.36 | |
| APC 1450 | 10.87 | 14.09 | 1.34 | 12.92 | 15.56 | 15.48 | 15.51 | 15.12 | |
| BTC CD41 | 12.16 | 9.96 | 10.05 | -2.25 | 11.09 | 9.29 | 13.12 | 11.75 | |
| BTC CD45 | 11.46 | 10.63 | 12.63 | 7.31 | 0.6 | 11.9 | 11.87 | 0.46 | |
| KRAS | 13.84 | 16.36 | 9.33 | 14.68 | 10.12 | 3.13 | 8.93 | 8.87 | |
| KRAS | 14.52 | 14.01 | 15.11 | 15.64 | 3.86 | 14.15 | 3.08 | 3.25 | |
| BRAF V600 | 10.91 | 10.03 | 11.17 | 11.05 | 11.06 | 10.1 | 9.28 | 1.73 | |

**CONCLUSION:**

All target mutations including APC 1309, APC 1367, APC 1450, BCT 41, BCT 45, BRAF V600 were detected as expected by present invention assay, indicating there is no cross-reactivity of the different target detection. KRAS 12 is producing a signal in KRAS 13 positive samples, but there is 6 Ct difference between the true KRAS 13 signal and the crosstalk signal from KRAS 12. This pattern can be used to differentiate between true KRAS 12 and KRAS 13 positive samples. Since the kit is to detect KRAS 12 and KRAS 13 mutations but not to differentiate them, the cross-talk will not have impact on the performance of the kit. Therefore, only intended target mutations can be detected by the present invention kit.

DNA input limits

Based on the analytical sensitivity studies (section 5.1), 2.5 ng or 5 ng was found to be the minimum

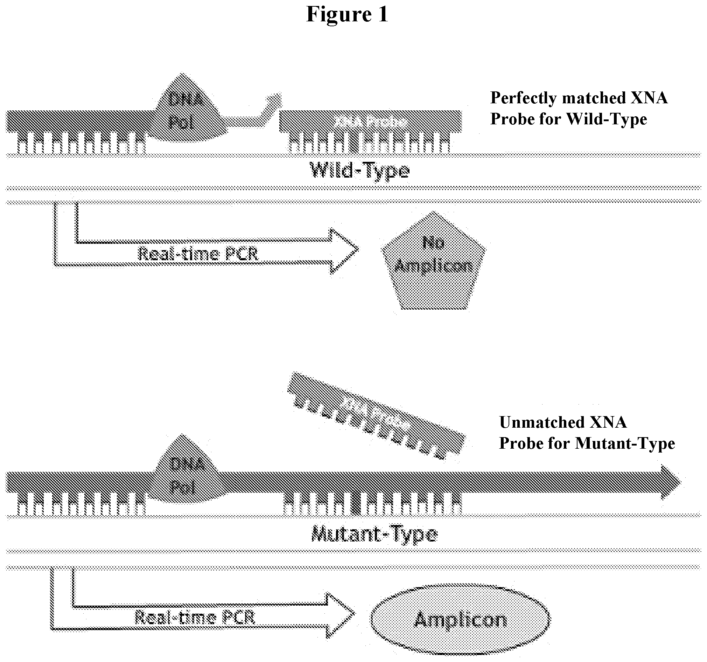

DNA input for the present invention kit to detect 1% mutations. To determine the maximum permissible DNA sample input for the present invention qPCR assays, high amounts of wild-type human genomic DNA were tested. Present invention qPCR assays (Fam and Hex) with different WT DNA inputs were performed for all targets in triplicates. The upper LOD was expected to be determined as the lowest DNA input levels producing false positive test results. qPCR with β-actin (Hex) was used to estimate the DNA amount and demonstrate PCR efficiency (Fig. 1). $\Delta$Ct ($\Delta$Ct = Ct Fam -Ct Hex) was calculated for each DNA input sample and compared to the $\Delta$Ct cut-off values to determine the mutation status of each DNA input sample (Table 53).

**Table 59.** Summary of the upper LOD test results ($\Delta$Ct = Ct Fam - Ct Hex).

| Assay | 2.5 ng | 5 ng | 10 ng | 20 ng | 40 ng | 80 ng | 160 ng | 320 ng |
|---|---|---|---|---|---|---|---|---|
| APC 1309/1367 | 20.30 | 21.01 | 18.14 | 23.61 | 24.77 | 25.58 | 26.47 | 27.33 |
| APC1450 | 16.75 | 17.76 | 13.92 | 9.83 | 10.62 | 10.07 | 10.74 | 10.52 |
| BCT41 | 14.07 | 11.25 | 9.81 | 10.29 | 9.63 | 9.63 | 9.41 | 9.47 |
| BCT45 | 12.80 | 10.20 | 10.08 | 11.52 | 9.86 | 10.20 | 10.00 | 10.10 |
| KRAS12 | 14.78 | 17.34 | 14.70 | 15.80 | 14.50 | 13.05 | 13.90 | 14.34 |
| KRAS13 | 16.33 | 13.35 | 13.53 | 13.99 | 13.64 | 12.82 | 12.33 | 13.50 |
| BRAFV600 | 10.62 | 10.71 | 12.93 | 10.15 | 9.68 | 10.30 | 9.82 | 9.73 |

**CONCLUSION:**

The DNA input amount (control Ct value) between $31 \leq Ct \leq 24$, was shown to be acceptable for the present invention assay corresponding to 2.5ng to 320 ng per gDNA per well. $\Delta$Ct analysis of different DNA input amounts showed 100% correct calls. No false positive results were observed with up to 320 ng DNA input. Since the recommended DNA input for present invention mutation detection assays is only 5 ng/per reaction, it is unlikely that there will be false positive result due to sample overloading at this level of input.

Assay Precision Study results

Two development lots of present invention Kit reagents were used in the reproducibility experiments - DL1 and DL2. Two operators (Qing Sun and Larry Pastor) were testing the kits on two different instruments. The main instrument was LC480 from Roche, the second test instrument was BioRad CFX384. These tests were performed to assess that the product meets requirements set in DDC.0006.
Experiments were performed to evaluate the reproducibility of the present invention assays including intra-assay, inter- assay, lot-to- lot, instrument comparison and operator reproducibility. For intra-assay reproducibility and instrument comparison, 9 replicates of each sample including NTC, WT and PC were tested in one run of each lot on one plate. To assess inter-assay, lot-to- lot and operator reproducibility, 3 replicates of each sample including NTC, WT and PC were tested in one run of each lot for all present invention targets on one plate. The intra-assay and inter-assay reproducibility experiments were repeated on DL2. The mean, SD, %CV value were calculated for each marker or each lot and test sample. The data are summarized in Tables 50 to 66 below.
All target Ct values are FAM signals, Control- from the internal control measured on HEX channel. Control values were calculated as averages for all replicates for each run.

**Table 60.** Intra-assay reproducibility test results (LC 480), (DL1)

| | WT | | | | | | | | | | | |
|---|---|---|---|---|---|---|---|---|---|---|---|---|
| Target | Repeat 1 | Repeat 2 | Repeat 3 | Repeat 4 | Repeat 5 | Repeat 6 | Repeat 7 | Repeat 8 | Repeat 9 | AVE | SD | CV |
| APC1309 | 50 | 50 | 50 | 50 | 50 | 50 | 50 | 50 | 50 | 50 | 0 | 0.00% |
| APC1367 | 50 | 50 | 50 | 50 | 50 | 50 | 50 | 50 | 50 | 50 | 0 | 0.00% |
| APC1450 | 40.93 | 39.38 | 42.23 | 50 | 38.44 | 40.37 | 40.18 | 38.96 | 50 | 42.28 | 4.26 | 10.10% |
| BTC CD41 | 41.26 | 44.33 | 38.59 | 37.63 | 37.44 | 37.64 | 41.72 | 38.99 | 40.33 | 39.77 | 2.2 | 5.50% |
| BTC CD45 | 40.47 | 40.49 | 38.61 | 38.83 | 37.17 | 40.94 | 19.02 | 41.64 | 39.02 | 39.58 | 1.32 | 3.30% |
| KRAS CD12 | 39.96 | 40.24 | 42.63 | 43.53 | 41.94 | 45 | 45 | 41.92 | 39.31 | 42.17 | 1.97 | 4.70% |
| KRAS CD13 | 42.9 | 45 | 42.44 | 42.4 | 41 | 41.67 | 41.7 | 41.63 | 40.55 | 42.14 | 1.22 | 2.90% |
| BRAF V600 | 38.98 | 38.49 | 39.02 | 37.35 | 38.95 | 40.2 | 39.1 | 38.9 | 40.77 | 39.08 | 0.91 | 2.30% |
| Control | 28.82 | 28.77 | 28.67 | 28.82 | 28.73 | 28.63 | 28.66 | 28.78 | 28.61 | 28.72 | 0.08 | 0.26% |
| | 5% PC | | | | | | | | | | | |
| Target | Repeat 1 | Repeat 2 | Repeat 3 | Repeat 4 | Repeat 5 | Repeat 6 | Repeat 7 | Repeat 8 | Repeat 9 | AVE | SD | CV |
| APC1309 | 30.1 | 30.01 | 30.22 | 30.21 | 30.12 | 29.82 | 30.12 | 30.4 | 30.36 | 30.15 | 0.17 | 0.60% |
| APC1367 | 30.27 | 29.97 | 29.93 | 30.14 | 30.05 | 29.89 | 30.13 | 30 | 30.05 | 30.05 | 0.12 | 0.40% |
| APC1450 | 31.85 | 32.15 | 32.19 | 31.77 | 26.85 | 32.04 | 32.02 | 31.82 | 32.14 | 31.43 | 1.62 | 5.20% |
| BTC CD41 | 28.04 | 27.64 | 27.93 | 27.85 | 27.92 | 27.93 | 27.98 | 27.84 | 27.79 | 27.88 | 0.12 | 0.40% |
| BTC CD45 | 30.24 | 30.62 | 30.22 | 30.2 | 30.24 | 30.43 | 30.83 | 30.36 | 30.11 | 30.36 | 0.22 | 0.70% |
| KRAS CD12 | 32.74 | 32.32 | 31.69 | 32.27 | 32.64 | 32.49 | 32.02 | 32.06 | 31.92 | 32.24 | 0.35 | 1.10% |
| KRAS CD13 | 32.88 | 33.11 | 33.48 | 32.89 | 33.17 | 33.5 | 33.58 | 33.78 | 33.19 | 33.29 | 0.3 | 0.90% |
| BRAF V600 | 32.15 | 31.58 | 31.72 | 31.82 | 32 | 31.93 | 31.82 | 32.26 | 31.87 | 31.91 | 0.21 | 0.70% |
| Control | 29.33 | 29.26 | 29.41 | 29.31 | 28.88 | 29.17 | 29.35 | 29.31 | 29.25 | 29.25 | 0.15 | 0.50% |
| | 1%PC | | | | | | | | | | | |
| Target | Repeat 1 | Repeat 2 | Repeat 3 | Repeat 4 | Repeat 5 | Repeat 6 | Repeat 7 | Repeat 8 | Repeat 9 | AVE | SD | CV |
| APC1309 | 32.16 | 32.47 | 32.41 | 32.61 | 32.5 | 32.9 | 32.81 | 32.57 | 12.71 | 32.57 | 0.21 | 0.55% |
| APC1367 | 32.34 | 32.79 | 32.95 | 32.53 | 32.83 | 33.05 | 32.39 | 32 | 32.79 | 32.63 | 0.34 | 0.53% |

(continued)

| Target | 1%PC | | | | | | | | | | | |
|--------|--------|----------|----------|----------|----------|----------|----------|----------|----------|-------|------|-------|
| | Repeat 1 | Repeat 2 | Repeat 3 | Repeat 4 | Repeat 5 | Repeat 6 | Repeat 7 | Repeat 8 | Repeat 9 | AVE | SD | CV |
| APC1450 | 34.95 | 34.88 | 34.74 | 34.41 | 34.35 | 34.73 | 34.16 | 34.64 | 34.12 | 34.55 | 0.29 | 0.63% |
| BTC CD41 | 30.55 | 30.63 | 30.24 | 30.25 | 30.35 | 30.74 | 30.53 | 29.93 | 30.31 | 30.39 | 0.25 | 0.32% |
| BTC CD45 | 32.67 | 32.73 | 32.48 | 33.07 | 32.95 | 33.76 | 33.92 | 32.12 | 32.73 | 32.94 | 0.55 | 0.45% |
| KRAS CD12 | 36.57 | 36.71 | 35.31 | 35.9 | 35.21 | 34.02 | 35.54 | 34.64 | 36.21 | 35.57 | 0.89 | 0.44% |
| KRAS CD13 | 36.71 | 36.02 | 36.61 | 35.96 | 36.52 | 36.78 | 36.72 | 36.02 | 38.19 | 36.61 | 0.64 | 1.44% |
| BRAF V600 | 34.08 | 34.54 | 34.53 | 35.65 | 35.14 | 34.64 | 35.72 | 34.91 | 35.32 | 34.95 | 0.55 | 0.45% |
| Control | 28.84 | 28.85 | 28.83 | 28.85 | 28.73 | 28.7 | 28.73 | 28.72 | 28.7 | 28.77 | 0.06 | 0.22% |

**Table 61.** Intra-assay reproducibility testing results (BioRad 384), DL1.

| | WT | | | | | | | | | | | |
|---|---|---|---|---|---|---|---|---|---|---|---|---|
| Target | Repeat 1 | Repeat 2 | Repeat | 3 Repeat 4 | Repeat 5 | 5 Repeat | 6 Repeat | 7 Repeat 8 | Repeat | 9 AVE | SD | CV |
| APC1309 | 36.74 | 50 | 50 | 50 | 50 | 50 | 50 | 50 | 50 | 48.53 | 4.17 | 8.60% |
| APC1367 | 50 | 37.72 | 50 | 50 | 50 | 38.2 | 50 | 50 | 50 | 47.32 | 5.01 | 10.60% |
| APC1450 | 38.01 | 41.34 | 41.32 | 38.7 | 40.38 | 37.1 | 37.55 | 43.82 | 37.71 | 39.55 | 2.15 | 5.40% |
| BTC CD41 | 38.28 | 41.26 | 36.22 | 41.14 | 40.19 | 44.41 | 38.19 | 45.57 | 36.01 | 40.14 | 3.16 | 7.90% |
| BTC CD45 | 42.82 | 38.5 | 39.48 | 38.73 | 40.38 | 41.54 | 39.78 | 42.3 | 42.66 | 40.69 | 1.59 | 3.90% |

| | WT | | | | | | | | | | | |
|---|---|---|---|---|---|---|---|---|---|---|---|---|
| Target | Repeat 1 | Repeat 2 | Repeat 3 | Repeat 4 | Repeat 5 | Repeat 6 | Repeat 7 | Repeat 8 | Repeat 9 | AVE | SD | CV |
| APC1309 | 36.74 | 50.00 | 50.00 | 50.00 | 50.00 | 50.00 | 50.00 | 50.00 | 50.00 | 48.53 | 4.17 | 8.6% |
| APC1367 | 50.00 | 37.72 | 50.00 | 50.00 | 50.00 | 38.20 | 50.00 | 50.00 | 50.00 | 47.32 | 5.01 | 10.6% |
| APC1450 | 38.01 | 41.34 | 41.32 | 38.70 | 40.38 | 37.10 | 37.55 | 43.82 | 37.71 | 39.55 | 2.15 | 5.4% |
| BTC CD41 | 38.28 | 41.26 | 36.22 | 41.14 | 40.19 | 44.41 | 38.19 | 45.57 | 36.01 | 40.14 | 3.16 | 7.9% |
| BTC CD45 | 42.82 | 38.50 | 39.48 | 38.73 | 40.38 | 41.54 | 39.78 | 42.30 | 42.66 | 40.69 | 1.59 | 3.9% |
| KRAS CD12 | 38.42 | 41.13 | 40.04 | 39.89 | 36.23 | 36.27 | 38.45 | 36.05 | 39.92 | 38.49 | 1.81 | 4.7% |
| KRAS CD13 | 38.66 | 40.01 | 40.68 | 45.61 | 39.31 | 37.83 | 40.06 | 38.99 | 38.11 | 39.92 | 2.20 | 5.5% |
| BRAF V600 | 37.31 | 37.32 | 38.61 | 39.22 | 37.50 | 39.27 | 37.67 | 37.20 | 37.36 | 37.94 | 0.80 | 2.1% |
| Control | 28.05 | 27.92 | 28.03 | 27.96 | 27.96 | 28.00 | 27.88 | 27.78 | 27.43 | 27.89 | 0.30 | 1.1% |

| | 5%PC | | | | | | | | | | | |
|---|---|---|---|---|---|---|---|---|---|---|---|---|
| Target | Repeat 1 | Repeat 2 | Repeat 3 | Repeat 4 | Repeat 5 | Repeat 6 | Repeat 7 | Repeat 8 | Repeat 9 | AVE | SD | CV |
| APC1309 | 28.30 | 27.81 | 28.29 | 28.10 | 28.27 | 28.21 | 28.05 | 28.24 27.72 | | 28.11 | 0.20 0.7% | |
| APC1367 | 28.13 | 27.36 | 28.05 | 27.76 | 28.30 | 27.58 | 28.41 | 28.00 | 27.31 | 27.88 | 0.40 | 1.4% |
| APC1450 | 29.68 | 29.86 | 30.16 | 29.49 | 29.66 | 29.62 | 29.61 | 29.46 | 29.32 | 29.65 | 10.23 | 0.8% |
| BTC CD41 | 26.57 | 26.72 | 26.53 | 26.53 | 26.49 | 26.46 | 26.60 | 26.39 | 26.22 | 26.50 | 10.14 | 0.5% |
| BTC CD45 | 30.14 | 30.53 | 30.52 | 30.09 | 30.20 | 29.97 | 30.06 | 30.16 | 30.08 | 30.19 | 0.19 | 0.6% |

(continued)

| 5%PC | Repeat 1 | Repeat 2 | Repeat 3 | Repeat 4 | Repeat 5 | Repeat 6 | Repeat 7 | Repeat 8 | Repeat 9 | AVE | SD | CV |
|---|---|---|---|---|---|---|---|---|---|---|---|---|
| Target | | | | | | | | | | | | |
| KRAS CD12 | 29.39 | 29.24 | 29.15 | 29.41 | 29.45 | 29.68 | 29.36 | 29.19 | 29.49 | 29.37 | 0.17 | 0.6% |
| KRAS CD13 | 31.63 | 31.64 | 31.84 | 31.78 | 31.55 | 31.34 | 31.57 | 31.42 | 31.81 | 31.62 | 0.16 | 0.5% |
| BRAF V600 | 30.16 | 31.64 | 31.42 | 31.29 | 31.64 | 31.04 | 31.69 | 31.12 | 31.26 | 31.25 | 0.47 | 1.5% |
| Control | 26.88 | 26.94 | 26.91 | 26.83 | 26.85 | 26.88 | 26.84 | 26.69 | 26.04 | 26.76 | 0.35 | 1.3% |

| 1%PC | Repeat 1 | Repeat 2 | Repeat 3 | Repeat 4 | Repeat 5 | Repeat 6 | Repeat 7 | Repeat 8 | Repeat 9 | AVE | SD | CV |
|---|---|---|---|---|---|---|---|---|---|---|---|---|
| Target | | | | | | | | | | | | |
| APC1309 | 30.56 | 30.77 | 30.22 | 30.89 | 30.67 | 29.83 | 30.58 | 30.32 | 31.04 | 30.54 | 0.35 | 1.1% |
| APC1367 | 32.63 | 30.80 | 31.02 | 31.27 | 30.04 | 30.63 | 31.05 | 30.74 | 30.70 | 30.99 | 0.70 | 2.3% |
| APC1450 | 32.38 | 32.08 | 32.09 | 32.40 | 31.52 | 32.11 | 32.18 | 31.81 | 31.89 | 32.05 | 0.26 | 0.8% |
| BTC CD41 | 29.12 | 28.82 | 29.08 | 28.92 | 29.21 | 28.87 | 29.13 | 28.88 | 28.71 | 28.97 | 0.17 | 0.6% |
| BTC CD45 | 32.52 | 32.59 | 33.01 | 32.90 | 32.83 | 33.76 | 32.04 | 32.04 | 32.67 | 32.71 | 0.49 | 1.5% |
| KRAS CD12 | 31.83 | 31.66 | 31.61 | 32.19 | 32.27 | 31.80 | 31.55 | 31.41 | 31.73 | 31.78 | 0.28 | 0.9% |
| KRAS CD13 | 33.50 | 35.74 | 34.69 | 34.68 | 34.40 | 33.70 | 33.57 | 34.18 | 33.52 | 34.22 | 0.70 | 2.1% |
| BRAF V600 | 33.77 | 33.59 | 32.86 | 32.83 | 33.09 | 33.26 | 34.40 | 33.11 | 32.78 | 33.30 | 0.53 | 1.6% |
| Control | 27.55 | 27.46 | 27.29 | 27.60 | 27.40 | 27.36 | 27.29 | 27.38 | 26.15 | 27.27 | 0.49 | 1.83% |

**Table 62.** Inter-assay reproducibility testing results. Average Ct values for each run are shown. Average (Ave) is a total average for three runs.

| | Run 1 | Run 2 | Run 3 | Ave | SD | CV% |
|---|---|---|---|---|---|---|
| Assay | WT | | | | | |
| APC1309/1367 | 50.00 | 50.00 | | 50.00 | 0.00 | 0.00% |
| APC1450 | 42.28 | 42.54 | 43.36 | 42.73 | 0.56 | 1.31% |
| BTC CD41 | 39.77 | 39.96 | 39.70 | 39.81 | 0.13 | 0.33% |
| BTC CD45 | 39.58 | 40.35 | 41.77 | 40.57 | 1.11 | 2.74% |
| KRAS CD12 | 42.17 | 43.39 | 45.32 | 43.63 | 1.59 | 3.64% |
| KRAS CD13 | 42.14 | 41.59 | 42.20 | 41.98 | 0.34 | 0.80% |
| BRAF V600 | 39.08 | 38.25 | 39.44 | 38.92 | 0.61 | 1.57% |
| Control | 28.72 | 29.09 | 29.23 | 29.01 | 0.26 | 0.91% |
| Assay | 5% PC | | | | | |
| APC1309/1367 | 30.05 | 30.29 | 30.21 | 30.18 0.10 | | 0.33% |
| APC1450 | 31.43 | 31.95 | 32.65 | 32.01 | 0.50 | 1.56% |
| BTC CD41 | 27.88 | 28.24 | 27.59 | 27.90 | 0.26 | 0.95% |
| BTC CD45 | 30.36 | 30.58 | 29.30 | 30.08 | 0.56 | 1.86% |
| KRAS CD12 | 32.24 | 33.02 | 31.72 | 32.33 | 0.53 | 1.65% |
| KRAS CD13 | 33.29 | 33.89 | 31.96 | 33.05 | 0.81 | 2.45% |
| BRAF V600 | 31.91 | 32.63 | 32.23 | 32.26 | 0.29 | 0.91% |
| Control | 29.25 | 29.67 | 28.39 | 29.10 | 0.53 | 1.83% |
| Assay | 1%PC | | | | | |
| APC1309/1367 | 32.63 | 32.92 | 32.73 | 32.76 | 0.12 | 0.37% |
| APC1450 | 34.55 | 34.47 | 34.78 | 34.60 | 0.13 | 0.38% |
| BTC CD41 | 30.39 | 30.60 | 30.32 | 30.44 | 0.12 | 0.39% |
| BTC CD45 | 32.94 | 33.02 | 31.81 | 32.59 | 0.55 | 1.69% |
| KRAS CD12 | 35.57 | 36.26 | 34.63 | 35.49 | 0.67 | 1.89% |
| KRAS CD13 | 36.61 | 37.30 | 33.84 | 35.92 | 1.50 | 4.16% |
| BRAF V600 | 34.95 | 34.90 | 34.27 | 34.71 | 0.31 | 0.89% |
| Control | 28.77 | 28.99 | 28.82 | 28.86 | 0.09 | 0.33% |

**Table 63.** Lot-to-lot variability (Roche LC 480), DL1 and DL2.

| | Lot 1 | Lot 2 | Ave | SD | CV% |
|---|---|---|---|---|---|
| Assay | WT | | | | |
| APC1309/1367 | 50.00 | 50.00 | 50.00 | 0.00 | 0.00% |
| APC1450 | 44.28 | 42.41 | 43.34 | 1.32 | 3.05% |
| BTC CD41 | 40.17 | 39.86 | 40.02 | 0.22 | 0.54% |
| BTC CD45 | 41.68 | 39.97 | 40.82 | 1.21 | 2.97% |
| KRAS CD12 | 44.52 | 42.78 | 43.65 | 1.23 | 2.82% |

(continued)

|  | Lot 1 | Lot 2 | Ave | SD | CV% |
|---|---|---|---|---|---|
| Assay | WT | | | | |
| KRAS CD13 | 42.81 | 41.87 | 42.34 | 0.67 | 1.58% |
| BRAF V600 | 39.94 | 38.67 | 39.30 | 0.90 | 2.29% |
| Control | 28.97 | 28.87 | 28.92 | 0.08 | 0.27% |
| Assay | 5% PC | | | | |
| APC 1309/1367 | 30.60 | 30.17 | 30.38 | 0.30 | 1.00% |
| APC1450 | 33.15 | 31.69 | 32.42 | 1.03 | 3.19% |
| BTC CD41 | 27.60 | 28.06 | 27.83 | 0.33 | 1.17% |
| BTC CD45 | 29.37 | 30.47 | 29.92 | 0.78 | 2.60% |
| KRAS CD12 | 32.31 | 32.63 | 32.47 | 0.23 | 0.70% |
| KRAS CD13 | 32.02 | 33.59 | 32.80 | 1.11 | 3.39% |
| BRAF V600 | 32.23 | 32.27 | 32.25 | 0.02 | 0.07% |
| Control | 28.82 | 29.46 | 29.14 | 0.45 | 1.56% |
|  | 1% PC | | | | |
| APC1309/1367 | 32.96 | 32.77 | 32.87 | 0.13 | 0.40% |
| APC1450 | 35.01 | 34.51 | 34.76 | 0.36 | 1.02% |
| BTC CD41 | 30.32 | 30.50 | 30.41 | 0.13 | 0.42% |
| BTC CD45 | 31.74 | 32.98 | 32.36 | 0.87 | 2.70% |
| KRAS CD12 | 35.38 | 35.91 | 35.64 | 0.38 | 1.07% |
| KRAS CD13 | 34.45 | 36.96 | 35.70 | 1.77 | 4.96% |
| BRAF V600 | 34.51 | 34.92 | 34.72 | 0.29 | 0.83% |
| Control | 28.80 | 28.88 | 28.84 | 0.06 | 0.21% |

**Table 64.** Lot-to-lot variability (BioRad CFX384), DL1 and DL2.

|  | WT | | | | |
|---|---|---|---|---|---|
| Target | Lot 1 | Lot 2 | AVE | SD | CV |
| APC 1309 | 48.52 | 48.53 | 48.52 | 0 | 0.00% |
| APC1367 | 47.23 | 47.32 | 47.28 | 0.05 | 0.10% |
| APC1450 | 43.5 | 39.55 | 41.52 | 1.97 | 4.75% |
| BTC CD41 | 40.73 | 40.14 | 40.44 | 0.3 | 0.73% |
| BTC CD45 | 44.31 | 40.69 | 42.5 | 1.81 | 4.25% |
| KRAS | 45.39 | 38.49 | 41.94 | 3.45 | 8.23% |
| KRAS | 40.71 | 39.92 | 40.32 | 0.4 | 0.99% |
| BRAF | 37.61 | 37.94 | 37.77 | 0.17 | 0.44% |
| Control | 27.89 | 27.89 | 27.89 | 0.3 | 1.10% |

(continued)

|  | 5% PC |  |  |  |  |
|---|---|---|---|---|---|
| Target | Lot 1 | Lot 2 | AVE | SD | CV |
| APC1309 | 27.53 | 28.11 | 27.82 | 0.29 | 1.04% |
| APC1367 | 27.63 | 27.88 | 27.75 | 0.18 | 0.64% |
| APC1450 | 29.77 | 29.65 | 29.71 | 0.06 | 0.21% |
| BTC CD41 | 26.25 | 26.5 | 26.38 | 0.18 | 0.66% |
| BTC CD45 | 29.34 | 30.19 | 29.77 | 0.43 | 1.43% |
| KRAS | 29.76 | 29.37 | 29.57 | 0.27 | 0.93% |
| KRAS | 30.06 | 31.62 | 30.84 | 0.78 | 2.53% |
| BRAF | 31.01 | 31.25 | 31.13 | 0.17 | 0.54% |
| Control | 26.76 | 26.76 | 26.76 | 0.35 | 1.31% |
|  | 1% PC |  |  |  |  |
| Target | Lot 1 | Lot 2 | AVE | SD | CV |
| APC1309 | 29.79 | 30.54 | 30.17 0.38 |  | 1.25% |
| APC1367 | 30.16 | 30.99 | 30.58 | 0.58 | 1.90% |
| APC1450 | 32.42 | 32.05 | 32.24 | 0.18 | 0.57% |
| BTC CD41 | 29.06 | 28.97 | 29.02 | 0.06 | 0.22% |
| BTC CD45 | 31.59 | 32.71 | 32.15 | 0.56 | 1.75% |
| KRAS | 32.33 | 31.78 | 32.06 | 0.39 | 1.21% |
| KRAS | 32.21 | 34.22 | 33.22 | 1 | 3.02% |
| BRAF | 32.1 | 33.3 | 32.7 | 0.84 | 2.58% |
| Control | [27.27 | 27.27 | 27.27 | 0.49 | 1.83% |

**Table 65.** Operator variability test (DL2, QS and LP).Average Ct values for each operator are shown.

|  | Operator1 | Operator2 |  | Ave | SD | CV% |
|---|---|---|---|---|---|---|
| Assay | WT |  |  |  |  |  |
| APC1309/1367 | 50.00 | 50.00 |  | 50.00 | 0.00 | 0.00% |
| APC1450 | 42.28 | 42.54 |  | 42.41 | 0.19 | 0.44% |
| BTC CD41 | 39.77 | 39.96 |  | 39.86 | 0.13 | 0.33% |
| BTC CD45 | 39.58 | 40.35 |  | 39.97 | 0.55 | 1.37% |
| KRAS CD12 | 42.17 | 43.39 |  | 42.78 | 0.87 | 1.02% |
| KRAS CD13 | 42.14 | 41.59 |  | 41.87 | 0.39 | 0.93% |
| BRAF V600 | 39.08 | 38.25 |  | 38.67 | 0.59 | 1.53% |
| Assay |  |  | 5%PC |  |  |  |
| APC1309/1367 | 30.05 | 30.29 |  | 30.17 | 0.17 | 0.57% |
| APC1450 | 31.43 | 31.95 |  | 31.69 | 0.37 | 1.16% |
| BTC CD41 | 27.88 | 28.24 |  | 28.06 | 0.26 | 0.92% |
| BTC CD45 | 30.36 | 30.58 |  | 30.47 | 0.16 | 0.52% |

(continued)

| Assay | | | 5%PC | | | |
|---|---|---|---|---|---|---|
| KRAS CD12 | 32.24 | 33.02 | | 32.63 | 0.55 | 1.69% |
| KRAS CD13 | 33.29 | 33.89 | | 33.59 | 0.43 | 1.28% |
| BRAF V600 | 31.91 | 32.63 | | 32.27 | 0.51 | 1.58% |
| Assay | | | 1%PC | | | |
| APC1309/1367 | 32.63 | 32.92 | | 32.77 | 0.20 | 0.62% |
| APC1450 | 34.55 | 34.47 | | 34.51 | 0.06 | 0.17% |
| BTC CD41 | 30.39 | 30.60 | | 30.50 | 0.15 | 0.48% |
| BTC CD45 | 32.94 | 33.02 | | 32.98 | 0.06 | 0.18% |
| KRAS CD12 | 35.57 | 36.26 | | 35.91 | 0.49 | 1.36% |
| KRAS CD13 | 36.61 | 37.30 | | 36.96 | 0.48 | 1.31% |
| BRAF V600 | 34.95 | 34.90 | | 34.92 | 0.04 | 0.10% |

**Table 66.** Instrument comparison on Roche LC 480 and BioRad 384.

| 5% mutant | | |
|---|---|---|
| Target | BioRad 384 % Correct Call | LC 480 % Correct Call |
| APC1309 | 100% | 100% |
| APC1367 | 100% | 100% |
| APC1450 | 100% | 100% |
| BTC CD41 | 100% | 100% |
| BTC CD45 | 100% | 100% |
| KRAS | 100% | 100% |
| KRAS | 100% | 100% |
| BRAF | 100% | 100% |
| 1% mutant | | |
| Target | BioRad 384 %Correct Call | LC 480 %Correct Call |
| APC1309 | 100% | 100% |
| APC1367 | 100% | 100% |
| APC1450 | 100% | 100% |
| BTC CD41 | 100% | 100% |
| BTC CD45 | 100% | 100% |
| KRAS | 100% | 100% |
| KRAS | 100% | 100% |
| BRAF | 100% | 100% |
| Note: Correct calls on LC 480 and BioRad 384 were made based on different cutoffs set on LC 480 and BioRad 384. | | |

**Assay Precision Summary:**

The data on precision testing of present invention kit reagents summarized in Tables 10 to 16 demonstrated that all the present invention assays have good intra-assay, inter-assay, lot-to-lot and operator reproducibility with %CV <10 **(Product requirement PR10 met).**

All planned tests of the sources of variation that could affect the reproducibility of the present invention assay were tested and results show that the assay is robust and meets product requirements as set in DDC.0007.

7.7 Assay sensitivity and specificity with FFPE samples (Matrix interference).

DNA from positive reference FFPE (KRAS G12D, Horizon Diagnostics) and negative (WT) FFPE was extracted with the QIAamp DSP DNA FFPE Tissue Kit (Catalog, Qiagen, REF 60604. QIAGEN GmbH, Hilden, Germany) following manufacturer's instructions. To determine the upper FFPE DNA input limit for the present invention assay (the maximum amount of WT DNA that can be tested without producing false positive results), different amounts of WT FFPE DNA (10 and 20 ng/well based on Qubit data) were used in the present invention reactions and tested on LC 480 instrument. Data are summarized in Table 67.

**Table 67.** Summary of the upper FFPE DNA input test results (ΔCt = Ct Fam - Ct Hex)

|  | 10 ng/well |  |  |  | 20 ng/well |  |  |  |
|---|---|---|---|---|---|---|---|---|
| Target | WT1 | WT2 | WT3 | AVE | WT1 | WT2 | WT3 | AVE |
| APC 1309 | 24.31 | 24.28 | 13.13 | 20.57 | 25.38 | 25.29 | 25.54 | 25.40 |
| APC 1367 | 24.44 | 24.37 | 24.59 | 24.47 | 25.42 | 25.48 | 13.96 | 21.62 |
| APC 1450 | 10.79 | 9.92 | 10.01 | 10.24 | 10.03 | 10.28 | 10.77 | 10.36 |
| BTC CD41 | 9.11 | 9.68 | 9.15 | 9.31 | 10.12 | 9.62 | 10.34 | 10.03 |
| BTC CD45 | 11.34 | 11.60 | 9.98 | 10.97 | 9.90 | 11.38 | 10.32 | 10.53 |
| KRAS CD12 | 13.27 | 15.18 | 15.07 | 14.51 | 14.30 | 12.16 | 13.50 | 13.32 |
| KRAS CD13 | 12.73 | 11.96 | 12.24 | 12.31 | 13.23 | 13.23 | 13.47 | 13.31 |
| BRAF V600 | 10.34 | 10.30 | 10.02 | 10.22 | 9.66 | 9.91 | 9.02 | 9.53 |
| Ct of BACT | 25.67 | 25.64 | 25.72 | 25.68 | 24.60 | 24.68 | 24.66 | 24.65 |

To estimate the assay sensitivity using DNA from FFPE, DNA input was set at 5 ng/well by Qubit data. DNA samples containing KRAS G12D mutation at 2% and 4% allelic frequency were tested and data were summarized in Table 68.

**Table 68.** Summary of FFPE DNA (2% and 4% KRAS G12D) test results, 5 ng/well (ΔCt = Ct Fam - Ct Hex). Positive calls are underlined, negative - not underlined

| Target | 4%G12D | 4%G12D | 4%G12D | AVE4% | 2%G12D | 2%G12D | 2%G12D | AVE2% |
|---|---|---|---|---|---|---|---|---|
| APC 1309 | 23.27 | 10.83 | 23.27 | 19.12 | 23.38 | 11.66 | 11.58 | 15.54 |
| APC 1367 | 23.31 | 23.44 | 23.6 | 23.45 | 23.31 | 10.51 | 23.48 | 19.1 |
| APC 1450 | 10.3 | 11.52 | 10.39 | 10.74 | 23.31 | 10.16 | 23.2 | 18.89 |
| BTC CD41 | 8.9 | 9.3 | 9.2 | 9.13 | 9.25 | 8.69 | 9.98 | 9.31 |
| BTC CD45 | 13.21 | 11.44 | 10.36 | 11.67 | 11.7 | 10.42 | 9.42 | 10.51 |
| KRAS CD12 | <u>8.46</u> | <u>8.34</u> | <u>8.38</u> | <u>8.39</u> | <u>9.87</u> | <u>9.89</u> | <u>10.11</u> | <u>9.96</u> |
| KRASCD13 | 11.18 | 13.51 | 11.86 | 12.18 | 18.2 | 11.65 | 14.79 | 14.88 |
| BRAF V600 | 10.74 | 9.94 | 10.31 | 10.33 | 8.65 | 8.63 | 9.3 | 8.86 |
| Ct of BACT | 26.77 | 26.74 | 26.64 | 26.72 | 26.78 | 26.77 | 26.73 | 26.76 |

CONCLUSIONS:

Specificity assessment for FFPE samples:

Test results of different FFPE DNA input indicated that FFPE DNA input up to 20 ng/per well produced no false positive results.

Sensitivity assessment for FFPE samples:

Initial testing on FFPE DNA with 2% and 4% KRAS G12D mutation suggested 2% of KRAS G12D can be detected with 100% accuracy at 5 ng input FFPE DNA level.

## Claims

1.  A method for detecting the presence or absence of a known mutated gene associated with colorectal cancer contained in a biological sample, said method comprising the steps of:

    (1) allowing a mixture of a clamp primer consisting of XNA which hybridizes with all or part of a target site having a sequence of a wild-type gene or a sequence complementary to the wild-type gene, a primer capable of amplifying a region comprising a target site having a sequence of the mutated gene, and the biological sample to coexist in a reaction solution for gene amplification, and selectively amplifying the region comprising a target site of the mutated gene by a gene amplification method, and
    (2) selectively detecting a detection region comprising the target site of the mutated gene by a gene detection method, using an amplified product obtained in step (1) or part thereof as a template, to detect the presence or absence of the mutated gene,

    wherein the mutations are selected from the group consisting of APC 1309, APC 1367, APC 1450, BCT 41, BCT 45, KRAS 12, KRAS 13 and BRAF V600, and
    wherein the XNA clamps and primers are selected from the group consisting of:

    SEQ ID NO:22 - ACGACACAGGAAGCAGATTCT,
    SEQ ID NO:23 - TCACAGGATCTTCAGCTGACCT,
    SEQ ID NO:24 - TTCCAATCTTTTATTTCTGCTATT,
    SEQ ID NO:25 - Lys-0-(CTGACCTAGTTCCAATCTTTTCTT)PNA,
    SEQ ID NO:26 - TTCAGGAGCGAAATCTCCC,
    SEQ ID NO:27 - TGAACATAGTGTTCAGGTG,
    SEQ ID NO:28 - 5'/56-FAM/CAAAAGTGG/ZEN/TGCTTAGACACCCAAAAG,
    SEQ ID NO:29 - Lys-O-(AGTGGTGCTCAGACA)PNA,
    SEQ ID NO:30 - CCAGATAGCCCTGGACAAACC,
    SEQ ID NO:31 - CTTTTCAGCAGTAGGTGCTTTATTTTTA,
    SEQ ID NO:32 - AGGTACTTCTCACTTGGTTT,
    SEQ ID NO:33 - TAGGTACTTCTCGCTTGGTTT,
    SEQ ID NO:34 - ACTCTGGAATCCATTCTGGTGC,
    SEQ ID NO:35 - AGAAAATCCCTGTTCCCACTCATA,
    SEQ ID NO:36 - AGGAAGAGGATGTGGATACCTCCCAAG,
    SEQ ID NO:37 - Lys-0-(TGCCACTACCACAGCTC)PNA,
    SEQ ID NO:38 - ACTCTGGAATCCATTCTGGTGC,
    SEQ ID NO:39 - AGAAAATCCCTGTTCCCACTCATA,
    SEQ ID NO:40 - AGGAAGAGGATGTGGATACCTCCCAAG,
    SEQ ID NO:41 - Ac-CTCCTTCTCTGAGTG-NH2,
    SEQ ID NO:42 - AAGGCCTGCTGAAAATGACTG,
    SEQ ID NO:43 - GTTGGATCATATTCGTCCAC,
    SEQ ID NO:44 - TCTGAATTAGCTGTATCGTCAAGGCACTC,
    SEQ ID NO:45 - CTACGCCACCAGCTCCAACTACCA-O-D-Lys,
    SEQ ID NO:46 - ACTTGTGGTAGTTGGAGCTGGT,
    SEQ ID NO:47 - GTTGGATCATATTCGTCCAC,
    SEQ ID NO:48 - TCTGAATTAGCTGTATCGTCAAGGCACTC,

SEQ ID NO:49 - D-LYS-PEG2-TCTTGCCTACGCCACCAGCTCCA-NH2,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG, and
SEQ ID NO:53 - ATCGAGATTTCACTGTAGCTAGAC.

**2.** The method according to claim 1, the method comprising: isolating DNA from a stool sample, fresh peripheral blood (PB), and formalin-fixed, paraffin-embedded (FFPE) tissues sample obtained from the patient suspected of having a condition associated with colorectal cancer mutations; performing PCR on the extracted DNA to produce amplified DNA while using a xenonucleic acid clamp for blocking amplification of wild-type DNA; sequencing the amplified DNA in an automated sequencer; analyzing an output of the automated sequencer to identify mutations in the sequence.

**3.** A kit for performing the method according to claim 1 or 2,
wherein the XNA clamps and primers are selected from the group consisting of:

SEQ ID NO:22 - ACGACACAGGAAGCAGATTCT,
SEQ ID NO:23 - TCACAGGATCTTCAGCTGACCT,
SEQ ID NO:24 - TTCCAATCTTTTATTTCTGCTATT,
SEQ ID NO:25 - Lys-O-(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,
SEQ ID NO:28 - 5'/56-FAM/CAAAAGTGG/ZEN/TGCTTAGACACCCAAAAG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAATCCATTCTGGTGC,
SEQ ID NO:35 - AGAAAATCCCTGTTCCCACTCATA,
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:42 - AAGGCCTGCTGAAAATGACTG,
SEQ ID NO:43 - GTTGGATCATATTCGTCCAC,
SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO:48 - TCTGAATTAGCTGTATCGTCAAGGCACTC,
SEQ ID NO:49 - D-LYS-PEG2-TCTTGCCTACGCCACCAGCTCCA-NH2,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG, and
SEQ ID NO:53 - ATCGAGATTTCACTGTAGCTAGAC.

**Patentansprüche**

**1.** Verfahren zum Nachweis des Vorhandenseins oder der Abwesenheit eines bekannten mutierten Gens, das mit kolorektalem Krebs assoziiert ist und in einer biologischen Probe enthalten ist, wobei das Verfahren die folgenden Schritte umfasst:

(1) Koexistierenlassen einer Mischung aus einem Clamp-Primer, bestehend aus XNA, die mit der gesamten oder einem Teil einer Zielstelle mit einer Sequenz eines Wildtyp-Gens oder einer zum Wildtyp-Gen komplementären Sequenz hybridisiert, einem Primer, der in der Lage ist, eine Region zu amplifizieren, die eine Zielstelle mit einer Sequenz des mutierten Gens umfasst, und der biologischen Probe in einer Reaktionslösung für die Genamplifikation und selektives Amplifizieren der Region, die eine Zielstelle des mutierten Gens umfasst, durch ein Genamplifikationsverfahren, und

(2) selektives Nachweisen einer Nachweisregion, die die Zielstelle des mutierten Gens umfasst, durch ein Gennachweisverfahren, wobei ein in Schritt (1) erhaltenes amplifiziertes Produkt oder ein Teil davon als Matrize verwendet wird, um die Anwesenheit oder Abwesenheit des mutierten Gens nachzuweisen,

wobei die Mutationen ausgewählt sind aus der Gruppe bestehend aus APC 1309, APC 1367, APC 1450, BCT 41, BCT 45, KRAS 12, KRAS 13 und BRAF V600, und wobei die XNA-Klammern und Primer ausgewählt sind aus der Gruppe, bestehend aus:

SEQ ID NO:22 - ACGACACAGGAAGCAGATTCT,
SEQ ID NO:23 - TCACAGGATCTTCAGCTGACCT,
SEQ ID NO:24 - TTCCAATCTTTTATTTCTGCTATT,
SEQ ID NO:25 - Lys-0-(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,
SEQ ID NO:28 - 5'/56-FAM/CAAAAGTGG/ZEN/TGCTTAGACACCCAAAAG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAA TCCATTCTGGTGC,
SEQ ID NO:35 - AGAAAA TCCCTGTTCCCACTCATA,
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:42 - AAGGCCTGCTGAAAATGACTG,
SEQ ID NO:43 - GTTGGATCATATTCGTCCAC,
SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO:48 - TCTGAATTAGCTGTATCGTCAAGGCACTC,
SEQ ID NO:49 - D-LYS-PEG2-TCTTGCCTACGCCACCAGCTCCA-NH2,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG, and
SEQ ID NO:53 - ATCGAGATTTCACTGTAGCTAGAC.

2. Verfahren nach Anspruch 1, wobei das Verfahren umfasst: Isolieren von DNA aus einer Stuhlprobe, frischem peripheren Blut (PB) und formalinfixierten, paraffin-eingebetteten (FFPE) Gewebeproben, die von dem Patienten erhalten wurden, bei dem der Verdacht besteht, dass er einen Zustand hat, der mit Darmkrebsmutationen assoziiert ist; Durchführen von PCR an der extrahierten DNA, um amplifizierte DNA zu erzeugen, während eine Xenonukleinsäure-Klammer zum Blockieren der Amplifikation von Wildtyp-DNA verwendet wird; Sequenzieren der amplifizierten DNA in einer automatisierten Sequenziervorrichtung; Analysieren eines Ergebnisses der automatisierten Sequenziervorrichtung, um Mutationen in der Sequenz zu identifizieren.

3. Kit zur Durchführung des Verfahrens nach Anspruch 1 oder 2, wobei die XNA-Klammern und Primer ausgewählt sind aus der Gruppe bestehend aus:

SEQ ID NO:22 - ACGACACAGGAAGCAGATTCT,

SEQ ID NO:23 - TCACAGGATCTTCAGCTGACCT,
SEQ ID NO:24 - TTCCAATCTTTTATTTCTGCTATT,
SEQ ID NO:25 - Lys-0-(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,
SEQ ID NO:28 - 5'/56-FAM/CAAAAGTGG/ZEN/TGCTTAGACACCCAAMG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAA TCCATTCTGGTGC,
SEQ ID NO:35 - AGAAAATCCCTGTTCCCACTCATA,
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:42 - AAGGCCTGCTGAAAATGACTG,
SEQ ID NO:43 - GTTGGATCATATTCGTCCAC,
SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO:48 - TCTGAATTAGCTGTATCGTCAAGGCACTC,
SEQ ID NO:49 - D-LYS-PEG2-TCTTGCCTACGCCACCAGCTCCA-NH2,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG, and
SEQ ID NO:53 - ATCGAGATTTCACTGTAGCTAGAC.

## Revendications

1. Procédé de détection de la présence ou de l'absence d'un gène muté connu associé au cancer colorectal contenu dans un échantillon biologique, ledit procédé comprenant les étapes suivantes :

(1) le fait de permettre un mélange d'une amorce de serrage constituée de XNA qui s'hybride avec tout ou une partie d'un site cible ayant une séquence d'un gène sauvage ou une séquence complémentaire du gène sauvage, une amorce capable d'amplifier une région comprenant un site cible site ayant une séquence du gène muté, et l'échantillon biologique pour coexister dans une solution de réaction pour l'amplification génique, et l'amplification sélectivement de la région comprenant un site cible du gène muté par un procédé d'amplification génique, et

(2) la détection sélectivement d'une région de détection comprenant le site cible du gène muté par un procédé de détection de gène, à l'aide d'un produit amplifié obtenu à l'étape (1) ou une partie de celui-ci comme matrice, pour détecter la présence ou l'absence du gène muté,

les mutations étant choisies dans le groupe constitué de APC 1309, APC 1367, APC 1450, BCT 41, BCT 45, KRAS 12, KRAS 13 et BRAF V600, et
les pinces XNA et les amorces étant choisies dans le groupe constitué de :

SEQ ID NO:22 - ACGACACAGGAAGCAGATTCT,
SEQ ID NO:23 - TCACAGGATCTTCAGCTGACCT,
SEQ ID NO:24 - TTCCAATCTTTTATTTCTGCTATT,
SEQ ID NO:25 - Lys-0(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,

SEQ ID NO:28 - 5'/56-FAM/CAAAAGTGG/ZEN/TGCTTAGACACCCAAAAG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAATCCATTCTGGTGC,
SEQ ID NO:35 - AGAAAATCCCTGTTGCCACTCATA,
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:42 - AAGGCCTGCTGAAAATGACTG,
SEQ ID NO:43 - GTTGGATCATATTCGTCCAC,
SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO:48 - TCTGAATTAGCTGTATCGTCAAGGCACTC,
SEQ ID NO:49 - D-LYS-PEG2-TCTTGCCTACGCCACCAGCTCCA NH2,
SEQ ID NQ:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG,
SEQ ID NO:53 - ATCGAGATTTCACTGTAGCTAGAC.

2. Procédé selon la revendication 1, le procédé comprenant : l'isolement d'ADN à partir d'un échantillon de selles, de sang périphérique (PB) frais et d'un échantillon de tissus fixés au formol et inclus en paraffine (FFPE) obtenus à partir du patient suspecté d'avoir un état associé à des mutations cancéreuses colorectales ; le fait d'effectuer une PCR sur l'ADN extrait pour produire de l'ADN amplifié tout en utilisant une pince à acide xénonucléique afin de bloquer l'amplification de l'ADN de type sauvage ; le séquençage de l'ADN amplifié dans un séquenceur automatisé ; l'analyse d'une sortie du séquenceur automatisé pour identifier des mutations dans la séquence.

3. Kit pour effectuer le procédé selon la revendication 1 ou 2,
les pinces XNA et les amorces étant choisies dans le groupe constitué de :

SEQ ID NO:22 - ACGACACAGGAAGCAGATTCT,
SEQ ID NO:23 - TCACAGGATCTTCAGCTGACCT,
SEQ ID NO:24 - TTCCAATCTTTTATTTCTGCTATT,
SEQ ID NO:25 - Lys-0-(CTGACCTAGTTCCAATCTTTTCTT)PNA,
SEQ ID NO:26 - TTCAGGAGCGAAATCTCCC,
SEQ ID NO:27 - TGAACATAGTGTTCAGGTG,
SEQ ID NO:28 - 5'/56-FAM/CAAAAGTGG/ZEN/TGCTTAGAGACCCAAAAG,
SEQ ID NO:29 - Lys-O-(AGTGGTGCTCAGACA)PNA,
SEQ ID NO:30 - CCAGATAGCCCTGGACAAACC,
SEQ ID NO:31 - CTTTTCAGCAGTAGGTGCTTTATTTTTA,
SEQ ID NO:32 - AGGTACTTCTCACTTGGTTT,
SEQ ID NO:33 - TAGGTACTTCTCGCTTGGTTT,
SEQ ID NO:34 - ACTCTGGAATCCATTCTGGTGC,
SEQ ID NO:35 - AGAAAATCCCTGTTCCCACTCATA
SEQ ID NO:36 - AGGAAGAGGATGTGGATACCTCCCAAG,
SEQ ID NO:37 - Lys-0-(TGCCACTACCACAGCTC)PNA,
SEQ ID NO:38 - ACTCTGGAATCCATTCTGGTGC,
SEQ ID NO:39 - AGAAAATCCCTGTTCCCACTCATA,
SEQ ID NO:40 - AGGAAGAGGATGTGGATACCTCCCAAG,
SEQ ID NO:41 - Ac-CTCCTTCTCTGAGTG-NH2,
SEQ ID NO:43 - GTTGGATCATATTCGTCCAC,

SEQ ID NO:44 - TCTGAATTAGCTGTATCGTCAAGGCACTC,
SEQ ID NO:45 - CTACGCCACCAGCTCCAACTACCA-O-D-Lys,
SEQ ID NO:46 - ACTTGTGGTAGTTGGAGCTGGT,
SEQ ID NO:47 - GTTGGATCATATTCGTCCAC,
SEQ ID NO-48 - TCTGAATTAGCTGTATCGTCAAGGCACTC,
SEQ ID NO:49 - D-LYS-PEG2-TCTTGGCTACGCCACCAGCTCCA-NH2,
SEQ ID NO:50 - ACAGTAAAAATAGGTGATTTTGGTCTAGCTA,
SEQ ID NO:51 - CATCCACAAAATGGATCCAGACAA,
SEQ ID NO:52 - CAAACTGATGGGACCCACTCCATCG,
SEQ ID NO:53 - ATCGAGATTTCACTGIAGCIAGAC.

Figure 1

## Figure 2

EP 3 494 236 B1

# Figure 3

## Amplification Curves

EP 3 494 236 B1

Figure 4

Figure 5

Amplification Curves

KRAS c12

# Figure 6

## Amplification Curves

EP 3 494 236 B1

# Figure 7

**Figure 8**

Figure 9

XNA Clamp Detects below 0.1%
Mutated DNA
$\Delta$Cq Mutant $\geq$ 1

EP 3 494 236 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160194691 A1
- WO 2011093606 A2
- US 78659117
- WO 0172823 A1

### Non-patent literature cited in the description

- Molecular diagnosis of response to neoadjuvant chemoradiation therapy in patients with locally advanced rectal cancer. **CHEN, Z et al.** J. Am. Coll. Surg. City of Hope, 06 January 2011, vol. 212, 1008-1017
- Frequency of KRAS, BRAF, and PIK3CA mutations in advanced colorectal cancers: comparison of peptide nucleic acid-mediated PCR clamping and direct sequencing in formalin-fixed, paraffin-embedded tissue. **KWON, M.J. et al.** Pathol. Res. Pract. Hallym University College of Medicine, 06 October 2011, vol. 207, 762-768
- Heterogenous distribution of K-ras mutations in primary colon carcinomas: implications for EGFR-directed therapy. **OLTEDAL, S et al.** Int. J. Colorectal Dis. Stavanger University Hospital, 15 May 2011, vol. 26, 1271-1277
- **MANIATIS et al.** Molecular Cloning: A Laboratory Manual, Cold Spring Harbor, N.Y. 1982, 280-281